# EUROPEAN PATENT APPLICATION

(11) **EP 4 851 194 A1**
(43) Date of publication of application: **09.12.2026**
(21) Application number: 25748016.0
(22) Date of filing: 27.01.2025
(51) Int. Cl.: C07K 16/28, C07K 19/00, C12N 15/62, A61K 39/395, A61P 35/00

(54) **BISPECIFIC ANTIBODY, AND CYTOKINE FUSION PROTEIN AND USE THEREOF**

(30) Priority: 02.02.2024 CN 202410148364; 02.02.2024 CN 202410153519
(71) Applicant: Changping National Laboratory, Beijing 102206 (CN)
(72) Inventor: YANG, Zaopeng, Beijing 102206 (CN); FU, Yangxin, Beijing 102206 (CN); REN, Zhenhua, Beijing 102206 (CN); LI, Shijie, Beijing 102206 (CN); WANG, Xinxin, Beijing 102206 (CN); LI, Nan, Beijing 102206 (CN); GUO, Jingya, Beijing 102206 (CN); CHEN, Yiwei, Beijing 102206 (CN)
(74) Representative: Ullrich & Naumann PartG mbB
(86) International application number: PCT/CN2025/075521
(87) International publication number: WO 2025/162454

(57) **Abstract**

Provided are a fusion protein and use thereof, and the fusion protein comprises a bispecific antibody and a cytokine.

## Description

### FIELD OF THE INVENTION

The present application relates to the field of molecular immunology in biomedicine, and in particular, to a fusion protein and use thereof.

### BACKGROUND OF THE INVENTION

Bifunctional antibody, also known as bispecific antibody (BsAb), is a specific drug that simultaneously targets two different antigens and can be produced by immune sorting and purification. BsAbs can be designed to simultaneously target the tumor cells and the effector cells, and to activate the effector cells to kill the tumor cells. The role of the immune system, particularly the T cell-mediated cytotoxicity, in the tumor control is well recognized, and more and more evidence indicates that T cells play the most prominent role in both controlling the tumor growth and survival in cancer patients. Over the past few decades, all of the therapeutic approaches for various types of tumors have aimed at reshaping and improving the function and activity of T cells to make them have stronger and more sustained anti-tumor characteristics. Among these, a T cell-bridging bispecific T-cell engager (BiTE) is a bispecific antibody composed of a T cell-binding domain (e.g., CD3) and a tumor-binding domain (primarily tumor cell surface-associated antigens, TAAs). It establishes a physical connection between T cells and the tumor cells, thereby promoting the killing of tumor cells by the cytotoxic T cells. In 2014, the U.S. Food and Drug Administration (FDA) approved the first BiTE, Blinatumomab (a bispecific antibody targeting both CD3 and CD19), for the treatment of relapsed/refractory B-cell acute lymphoblastic leukemia (ALL). This demonstrates that the killing of tumors by the endogenous T cells is achievable without the need for editing operations on immune cells in vitro, which is one aspect in which BiTEs are superior to cell therapy.

Although Blinatumomab has achieved successful application in B-cell acute lymphoblastic leukemia (ALL), BiTEs have shown limited efficacy in solid tumors, encountering difficulties similar to those encountered in cell therapy. This may be due, on the one hand, to the limited penetration of BiTEs into the tumor tissues, and on the other hand, to the presence of numerous immunosuppressive factors in the tumor microenvironment that restrict the restoration of T cell function within tumors by BiTEs. It has been reported that the addition of co-stimulatory factors, such as anti-CD28, to BiTEs can enhance the function of BiTEs. Cytokines, acting as the third signal in T cell activation, play a crucial role in improving the tumor microenvironment. However, the role of cytokines in BiTEs remains unknown. Based on this, we explored the role of cytokines in BiTE therapy.

### SUMMARY OF THE INVENTION

In view of the above-mentioned problems and therapeutic potential of BiTEs, further development is necessary in clinical applications to make them more effective and safer.

Specifically, the present invention provides the following aspects:
1. A fusion protein comprising structural unit A and structural unit B, wherein structural unit A is an IgG-type bispecific antibody comprising structural unit 1 and structural unit 2, wherein structural unit 1 is an anti-CD3 antibody, and structural unit 2 is an anti-tumor cell surface antigen antibody, and wherein the antibody represented by structural unit 1 and the antibody represented by structural unit 2 are in the form of Fab or scFv, respectively; and structural unit B is one or more (preferably two) cytokines or pro-cytokines, wherein structural unit B is linked to the N-terminus and/or C-terminus of the bispecific antibody via a cleavable or non-cleavable linker, and wherein the pro-cytokine is the cytokine linked to an antibody or a receptor of the cytokine via a cleavable linker; preferably, the fusion protein is in the form of a protein drug, mRNA, or CART.
2. The fusion protein according to item 1, wherein
   when structural unit 1 and structural unit 2 are both in the form of scFv, structural unit B is located at the N-terminus or C-terminus of structural unit 1;
   when structural unit 1 and/or structural unit 2 is in the form of scFv and/or Fab, the bispecific antibody further comprises structural unit 4 which is an Fc fragment of an antibody or a human serum albumin,
      I) when structural unit 4 is an Fc fragment of an antibody:
         structural unit 1 and structural unit 2 are both located at the N-terminus of the bispecific antibody; and structural unit 4 is linked to the C-terminus of structural unit 1 and the C-terminus of structural unit 2, respectively; and
         structural unit B is located at the N-terminus of structural unit 1 and/or the N-terminus of structural unit 2, or is located at the C-terminus of structural unit 4, and structural unit B is one or two cytokines or pro-cytokines;
      II) when structural unit 4 is a human serum albumin:
         the bispecific antibody comprises, in sequence from the N-terminus to the C-terminus:
         a) structural unit 2 -structural unit 4 -structural unit 1,
         b) structural unit 4 -structural unit 1 -structural unit 2,
         c) structural unit 2 -structural unit 1 -structural unit 4, or
         d) structural unit 1 -structural unit 4 -structural unit 2.
3. The fusion protein according to item 1, wherein structural unit A further comprises structural unit 4 which is an Fc fragment of an antibody; structural unit 1 and structural unit 2 are in the form of scFv, and structural unit 2 is located at the N-terminus of structural unit 1; the C-terminus of structural unit 1 is linked to one chain of the Fc fragment, and structural unit B is linked to the other chain of the Fc fragment; preferably, structural unit B is one or two cytokines or pro-cytokines.
4. The fusion protein according to item 1 or 2, wherein
   structural unit A further comprises structural unit 3 and/or structural unit 4,
   structural unit 3 is a tumor cell targeting peptide, linked to the N-terminus of structural unit 1 via a linker selected from the group consisting of:
      (2) linker 1, which is a pH-sensitive linker;
      (2) linker 2, which is a cleavable linker; and
      (3) linker 3, which is a combination of a pH-sensitive linker and a cleavable linker, wherein the cleavable linker and the pH-sensitive linker are sequentially linked (wherein the cleavable linker is located at the N-terminus or C-terminus), or the sequence of linker 3 is set forth in SEQ ID NO: 13 or 14;
   structural unit 4 is an Fc fragment of an antibody or a human serum albumin,
      I) when structural unit 4 is an Fc fragment of an antibody:
         structural unit 1, structural unit 2 and structural unit 3 are all located at the N-terminus or C-terminus of the bispecific antibody;
         structural unit 4 is linked to the C-terminus of structural unit 1 and the C-terminus of structural unit 2, respectively, while the N-terminus of structural unit 1 is linked to structural unit 3; or
         structural unit 4 is linked to the N-terminus of structural unit 1 and the N-terminus of structural unit 2, respectively, while the C-terminus of structural unit 1 is linked to structural unit 3;
      II) when structural unit 4 is a human serum albumin:
         the bispecific antibody comprises, in sequence from N-terminus to C-terminus:
         a) structural unit 2 -structural unit 4 -structural unit 1-structural unit 3,
         b) structural unit 3 -structural unit 4 -structural unit 1 - structural unit 2,
         c) structural unit 2 -structural unit 1 -structural unit 4-structural unit 3, or
         d) structural unit 3 -structural unit 1 -structural unit 4 -structural unit 2.
5. The fusion protein according to item 4, wherein
   (1) the bispecific antibody consists of structural units 1 to 4; structural units 1 to 3 are located at the N-terminus of the bispecific antibody, structural unit 4 is located at the C-terminus of the bispecific antibody, and structural unit 3 is linked to the N-terminus of structural unit 1 via a linker; structural units 1 and 3 are in the form of Fab, structural unit 2 is in the form of scFv, and structural unit 4 is an Fc fragment of an antibody;
      wherein the cytokine is linked to the C-terminus of the light chain variable region of structural unit 1 or to the C-terminus of either chain of structural unit 4 via a cleavable or non-cleavable linker;
   (2) the bispecific antibody consists of structural unit 1, structural unit 2 and structural unit 4; structural units 1 and 2 are located at the N-terminus of the bispecific antibody, structural unit 4 is located at the C-terminus of the bispecific antibody; structural unit 1 is in the form of Fab, structural unit 2 is in the form of scFv, and structural unit 4 is an Fc fragment of an antibody;
      wherein the cytokine is linked to the N-terminus of one chain of structural unit 1 via a cleavable or non-cleavable linker, and the receptor of the cytokine is linked to the N-terminus of the other chain of structural unit 1 via a cleavable or non-cleavable linker.
6. The fusion protein according to item 5, wherein
   when the cytokine is linked to the C-terminus of the light chain variable region of structural unit 1, another terminus of the cytokine is further linked to a receptor of the cytokine via a cleavable or non-cleavable linker; or
   when the cytokine is linked to the C-terminus of either chain of structural unit 4, structural unit B further comprises a receptor of the cytokine, wherein the receptor of the cytokine is linked to the C-terminus of another chain of structural unit 4 via a cleavable or non-cleavable linker, or the receptor of the cytokine is linked to another terminus of the cytokine via a cleavable or non-cleavable linker.
7. The fusion protein according to item 4, wherein structural unit 3 is selected from an anti-tumor cell surface antigen antibody, NGR, CNGRC, RGD, MT1-AF7p, YSA or TNYL; and when structural unit 3 is an anti-tumor cell surface antigen antibody which is Fab or scFv format, the tumor cell surface antigen targeted by structural unti 3 is same as or different from the tumor cell surface antigen targeted by structural unit 2.
8. The fusion protein according to item 4 or 7, wherein structural unit B is located at the C-terminus of structural unit A, and structural unit B is one or two cytokines or pro-cytokines.
9. The fusion protein according to item 1 or 2, wherein the cytokine is selected from a wild-of IL2 (such as ACCESSION P60568), IL15 (such as ACCESSION P40933), IL-21 (such as ACCESSION Q9HBE4-1 or Q9HBE4-2), IL-10 (such as ACCESSION P22301), or IL-4 (such as ACCESSION P05112), or a mutant thereof.
10. The fusion protein according to item 5 or 6, wherein the cytokine is a wild-type of IL2 or IL15, or a mutant thereof;
   preferably, the sequence of IL2 is set forth in ACCESSION P60568, the sequence of IL15 is set forth in SEQ ID NO: 135 or 137, the sequence of the IL2 mutant is set forth in any one of SEQ ID NOs: 110, and 171-177, and the sequence of the IL15 mutant is set forth in SEQ ID NOs: 147, 148, or 178;
   preferably, when structural unit B comprises a receptor of the cytokine, the sequence of the IL15 receptor is set forth in SEQ ID NO: 136 or 138, the IL15-IL15 receptor complex is selected from the sequence set forth in SEQ ID NO: 133 or 146, and the sequence of the IL2 receptor is set forth in SEQ ID NO: 167 or 168.
11. The fusion protein according to any one of items 1 to 10, wherein the anti-CD3 antibody is selected from the group consisting of:
   (1) OKT3, wherein the CDR1 sequence of the VL thereof is set forth in SEQ ID NO: 48, the CDR2 sequence of the VL thereof is DTS, the CDR3 sequence of the VL thereof is set forth in SEQ ID NO: 49, the CDR1 sequence of the VH thereof is set forth in SEQ ID NO: 50, the CDR2 sequence of the VH thereof is set forth in SEQ ID NO: 51, and the CDR3 sequence of the VH thereof is set forth in SEQ ID NO: 52; preferably, the amino acid sequence of the VL thereof is set forth in SEQ ID NO: 35, and the amino acid sequence of the VH thereof is set forth in SEQ ID NO: 36;
   (2) L2K, wherein the amino acid sequence of the VL thereof is set forth in SEQ ID NO: 37 and the amino acid sequence of the VH thereof is set forth in SEQ ID NO: 38;
   (3) UCHT1, wherein the CDR1 sequence of the VL thereof is set forth in SEQ ID NO: 53, the CDR2 sequence of the VL thereof is YTS, the CDR3 sequence of the VL thereof is set forth in SEQ ID NO: 54, the CDR1 sequence of the VH thereof is set forth in SEQ ID NO: 55, the CDR2 sequence of the VH thereof is set forth in SEQ ID NO: 56, and the CDR3 sequence of the VH thereof is set forth in SEQ ID NO: 57;
      preferably, the amino acid sequence of the VL thereof is set forth in SEQ ID NO: 39, and the amino acid sequence of the VH thereof is set forth in SEQ ID NO: 40;
   (4) SP34, wherein the CDR1 sequence of the VL thereof is set forth in SEQ ID NO: 58, the CDR2 sequence of the VL thereof is GTN, the CDR3 sequence of the VL thereof is set forth in SEQ ID NO: 59, the CDR1 sequence of the VH thereof is set forth in SEQ ID NO: 60, the CDR2 sequence of the VH thereof is set forth in SEQ ID NO: 61, and the CDR3 sequence of the VH thereof is set forth in SEQ ID NO: 62;
      preferably, the amino acid sequence of the VL thereof is set forth in SEQ ID NO: 41, and the amino acid sequence of the VH thereof is set forth in SEQ ID NO: 42;
   (5) 17A2, wherein the CDR1 sequence of the VL thereof is set forth in SEQ ID NO: 63, the CDR2 sequence of the VL thereof is YAS, the CDR3 sequence of the VL thereof is set forth in SEQ ID NO: 64, the CDR1 sequence of the VH thereof is set forth in SEQ ID NO: 65, the CDR2 sequence of the VH thereof is set forth in SEQ ID NO: 66, and the CDR3 sequence of the VH thereof is set forth in SEQ ID NO: 67;
      preferably, the amino acid sequence of the VL thereof is set forth in SEQ ID NO: 43, and the amino acid sequence of the VH thereof is set forth in SEQ ID NO: 44; and
   (6) 145-2C11, wherein the CDR1 sequence of the VL thereof is set forth in SEQ ID NO: 68, the CDR2 sequence of the VL thereof is YTN, the CDR3 sequence of the VL thereof is set forth in SEQ ID NO: 69, the CDR1 sequence of the VH thereof is set forth in SEQ ID NO: 70, the CDR2 sequence of the VH thereof is set forth in SEQ ID NO: 71, and the CDR3 sequence of the VH thereof is set forth in SEQ ID NO: 72; preferably, the amino acid sequence of the VL thereof is set forth in SEQ ID NO: 45, and the amino acid sequence of the VH thereof is set forth in SEQ ID NO: 46.
12. The fusion protein according to any one of items 4 to 10, wherein
   the pH-sensitive linker is selected from HA, or the cleavable linker is selected from the cleavage site of MMP, uPA, or CD13;
   preferably, the MMP is selected from the group consisting of:
      (1) any one of SEQ ID NOs: 6-8 and 83-94; or
      (2) each pairwise combination of any one sequence selected from SEQ ID NOs: 6-8 and any one sequence selected from SEQ ID NOs: 83-94; or
      (3) each pairwise combination of any one sequence selected from SEQ ID NOs: 1-3, 24, 28, 30, and 32 and any one sequence selected from SEQ ID NOs: 6-8 and 83-94.
13. The fusion protein according to any one of items 1 to 12, wherein the tumor cell surface antigen is selected from the group consisting of Claudin18.2 (such as ACCESSION: P56856), PDL1 (such as ACCESSION: Q9NZQ7), CD139 (such as ACCESSION: P15144), MSLN (such as ACCESSION: Q12321), GPC3 (such as ACCESSION: Q8CFZ4), Her29r (such as ACCESSION: P04626), EGFR (such as ACCESSION: P00533), GD2 (such as ACCESSION: Q9UI17), Trop2 (such as ACCESSION: P09758), CD19 (such as ACCESSION: P15391), CD20 (such as ACCESSION: P11836), Her3 ACCESSION: P21860, FolR1 (such as ACCESSION: P15328), Nectin4 (such as ACCESSION: Q96NY8), CD276 (such as ACCESSION: Q5ZPR3), CD30 (such as ACCESSION: P28908), CD70 (such as ACCESSION: P32970), BCMA (such as ACCESSION: Q02223), CD22 (such as ACCESSION: P20273), CD33 (such as ACCESSION: P20138), GPRC5D (such as ACCESSION: Q9NZD1), HLADR (such as ACCESSION: Q29744), VTCN1 (such as ACCESSION: Q7Z7D3), CD79B (such as ACCESSION: P40295), CDH3 ACCESSION: P22223, DR5 ACCESSION: P21918, MUC1 (such as ACCESSION: P15941), NT5E (such as ACCESSION: P21589), FAP (such as ACCESSION: Q12884), and PSMA (such as ACCESSION: Q04609).
14. The fusion protein according to any one of items 4 to 13, wherein the tumor cell surface antigen targeted by structural unit 2 is Claudin18.2, and the tumor cell surface antigen targeted by structural unit 3 is Claudin18.2;
   wherein the tumor cell surface antigen targeted by structural unit 2 is Claudin18.2, and the tumor cell surface antigen targeted by structural unit 3 is PDL1;
   wherein the tumor cell surface antigen targeted by structural unit 2 is PDL1, and the tumor cell surface antigen targeted by structural unit 3 is Claudin18.2;
   wherein the tumor cell surface antigen targeted by structural unit 2 is PDL1, and the tumor cell surface antigen targeted by structural unit 3 is PDL1;
   wherein the tumor cell surface antigen targeted by structural unit 2 is MSLN, and the tumor cell surface antigen targeted by structural unit 3 is MSLN; or
   wherein the tumor cell surface antigen targeted by structural unit 2 is FAP, and the tumor cell surface antigen targeted by structural unit 3 is FAP;
   preferably, for structural unit 2 or 3 targeting the tumor cell surface antigen PDL1, the LCDR1 sequence thereof is set forth in SEQ ID NO: 73, the LCDR2 sequence thereof is SAS, the LCDR3 sequence thereof is set forth in SEQ ID NO: 74, the HCDR1 sequence thereof is set forth in SEQ ID NO: 75, the HCDR2 sequence thereof is set forth in SEQ ID NO: 76, and the HCDR3 sequence thereof is set forth in SEQ ID NO: 77;
   for structural unit 2 or 3 targeting the tumor cell surface antigen Claudin18.2, the LCDR1 sequence thereof is set forth in SEQ ID NO: 78, the LCDR2 sequence thereof is WAS, the LCDR3 sequence thereof is set forth in SEQ ID NO: 79, the HCDR1 sequence thereof is set forth in SEQ ID NO: 80, the VCDR2 sequence is set forth in SEQ ID NO: 81, and the HCDR3 sequence thereof is set forth in SEQ ID NO: 82; or
   for structural unit 2, the LCDR1 sequence thereof is set forth in SEQ ID NO: 78, the LCDR2 sequence thereof is WAS, the LCDR3 sequence thereof is set forth in SEQ ID NO: 79, the HCDR1 sequence thereof is set forth in SEQ ID NO: 80, the HCDR2 sequence thereof is set forth in SEQ ID NO: 81, and the HCDR3 sequence thereof is set forth in SEQ ID NO: 82; and for structural unit 3, the LCDR1 sequence there thereof of is set forth in SEQ ID NO: 73, the LCDR2 sequence thereof is SAS, the LCDR3 sequence is set forth in SEQ ID NO: 74, the HCDR1 sequence thereof is set forth in SEQ ID NO: 75, the HCDR2 sequence thereof is set forth in SEQ ID NO: 76, and the HCDR3 sequence thereof is set forth in SEQ ID NO: 77.
15. The fusion protein according to any one of items 4 to13, wherein
   the tumor cell surface antigen targeted by structural unit 2 is Claudin18.2 as set forth in ACCESSION: P56856, and structural unit 3 is CNGRC;
   the tumor cell surface antigen targeted by structural unit 2 is Claudin18.2, and structural unit 3 is RGD;
   the tumor cell surface antigen targeted by structural unit 2 is Claudin18.2 as set forth in ACCESSION: P56856, and structural unit 3 is TNYL; or
   the tumor cell surface antigen targeted by structural unit 2 is MSLN, and structural unit 3 is CNGRC.
16. The fusion protein according to any one of items 4 to 15, wherein the Fc fragment is an Fc fragment of IgG1, IgG2, IgG3, or IgG4; preferably, the Fc fragment is a humanized Fc fragment.
17. The fusion protein according to any one of items 4 to 16, wherein the heavy chain constant region of the Fc fragment is an Ig gamma-1 chain C region of ACCESSION: P01857; and the light chain constant region of the Fc fragment is an Ig kappa chain C region of ACCESSION: P01834;
   preferably, according to the EU numbering system, based on the Ig gamma-1 chain C region of ACCESSION: P01857, the heavy chain constant region of the Fc fragment has the following mutations at positions 234, 235, and/or 237:
   L234A and L235A,
   L234A and G237A,
   L235A and G237A, or
   L234A, L235A, and G237A;
   preferably, according to the EU numbering system, the heavy chain constant region of the Fc fragment further has one or more mutations selected from the group consisting of: N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, H268A, E318A, and K320A.
18. The fusion protein according to any one of items 4 to 17, wherein the sequence of the Fc fragment is selected from SEQ ID NO: 19 and SEQ ID NO: 20.
19. The fusion protein according to any one of items 4 to 18, wherein when structural unit 4 is a human serum albumin, it is the human serum albumin of ACCESSION: P02768.
20. The fusion protein according to any one of items 4 to 19, wherein the sequence of structural unit 2 is selected from SEQ ID NO: 17, SEQ ID NO: 18, or SEQ ID NO: 156; and/or the sequence of structural unit 3 is selected from SEQ ID NO: 17, SEQ ID NO: 18, SEQ ID NO: 156, SEQ ID NO: 33, or SEQ ID NO: 34;
   preferably, the bispecific antibody is selected from the group consisting of:
   (1) wherein the sequence of structural unit 2 is selected from the sequence as set forth in SEQ ID NO: 17, and the sequence of structural unit 3 is selected from the sequence as set forth in SEQ ID NO: 18;
   (2) wherein the sequence of structural unit 2 is selected from the sequence as set forth in SEQ ID NO: 18, and the sequence of structural unit 3 is selected from the sequence as set forth in SEQ ID NO: 17;
   (3) wherein the sequence of structural unit 2 is selected from the sequence as set forth in SEQ ID NO: 17, and the sequence of structural unit 3 is selected from the sequence as set forth in SEQ ID NO: 33;
   (4) wherein the sequence of structural unit 2 is selected from the sequence as set forth in SEQ ID NO: 18, and the sequence of structural unit 3 is selected from the sequence as set forthin SEQ ID NO: 33;
   (5) wherein the sequence of structural unit 2 is selected from the sequence as set forth inSEQ ID NO: 18, and the sequence of structural unit 3 is selected from the sequence as set forth in SEQ ID NO: 34; and
   (6) wherein the sequence of structural unit 2 is selected from the sequence as set forth in SEQ ID NO: 17, and the sequence of structural unit 3 is selected from the sequence as set forth in SEQ ID NO: 34.
21. The fusion protein according to any one of items 4 to 20, wherein the bispecific antibody is selected from the group consisting of:
   (1) a bispecific antibody comprising or consisting of the sequences as set forth in SEQ ID NO: 22 and SEQ ID NO: 23;
   (2) a bispecific antibody comprising or consisting of the sequences as set forth in SEQ ID NO: 22 and SEQ ID NO: 25;
   (3) a bispecific antibody comprising or consisting of the sequences as set forth in SEQ ID NO: 26 and SEQ ID NO: 27;
   (4) a bispecific antibody comprising or consisting of the sequences as set forth in SEQ ID NO: 26 and SEQ ID NO: 29;
   (5) a bispecific antibody comprising or consisting of the sequences as set forth in SEQ ID NO: 26 and SEQ ID NO: 31;
   (6) a bispecific antibody comprising or consisting of the sequence as set forth in SEQ ID NO: 22 and a sequence selected from any one of SEQ ID NOs: 95-109;
   (7) a bispecific antibody comprising or consisting of the sequences as set forth in SEQ ID NO: 22, SEQ ID NO: 107, and SEQ ID NO: 108;
   (8) a bispecific antibody comprising or consisting of the sequences as set forth in SEQ ID NO: 22, SEQ ID NO: 107, and SEQ ID NO: 109;
   (9) a bispecific antibody prodrug comprising or consisting of the sequences represented by SEQ ID NO: 22, sequence A, SEQ ID NO: 158, SEQ ID NO: 159, SEQ ID NO: 162, and SEQ ID NO: 163, wherein sequence A is selected from any one of SEQ ID NOs: 4-14, 83-94, or 149-155;
   (10) a bispecific antibody prodrug comprising or consisting of the sequences represented by SEQ ID NO: 22, sequence A, SEQ ID NO: 160, SEQ ID NO: 161, SEQ ID NO: 162, and SEQ ID NO: 163, wherein sequence A is selected from any one of SEQ ID NOs: 4-14, 83-94, or 149-155;
   (11) a bispecific antibody prodrug comprising the sequences represented by SEQ ID NO: 156, sequence A, SEQ ID NO: 158, SEQ ID NO: 159, SEQ ID NO: 164, and SEQ ID NO: 165, or consisting of the sequences represented by SEQ ID NO: 22, sequence A, SEQ ID NO: 158, SEQ ID NO: 159, SEQ ID NO: 164, and SEQ ID NO: 165, wherein sequence A is selected from any one of SEQ ID NOs: 4-14, 83-94, or 149-155; and
   (12) a bispecific antibody prodrug comprising or consisting of the sequences represented by SEQ ID NO: 156, sequence A, SEQ ID NO: 160, SEQ ID NO: 161, SEQ ID NO: 164, and SEQ ID NO: 165, wherein sequence A is selected from any one of SEQ ID NOs: 4-14, or 83-94, and 149-155;
   preferably, the fusion protein is selected from the group consisting of:
   (1) a fusion protein comprising or consisting of the sequence as set forth in SEQ ID NO: 111 and the sequence selected from any one of SEQ ID NOs: 22, 26, or 156;
   (2) a fusion protein comprising or consisting of the sequences as set forth in SEQ ID NO: 112 and SEQ ID NO: 113;
   (3) a fusion protein comprising or consisting the sequences as set forth in SEQ ID NO: 112 and SEQ ID NO: 114;
   (4) a fusion protein comprising or consisting of the sequences as set forth in SEQ ID NO: 112 and SEQ ID NO: 115;
   (5) a fusion protein comprising or consisting of the sequence as set forth in SEQ ID NO: 116 and the sequence selected from any one of SEQ ID NOs: 22, 26, or 156;
   (6) a fusion protein comprising or consisting of the sequence selected from any one of SEQ ID NO: 22, 26, or 156, and the sequences as set forth in SEQ ID NO: 117 and SEQ ID NO: 108;
   (7) a fusion protein comprising or consisting of the sequences as set forth in SEQ ID NO: 112, SEQ ID NO: 118, and SEQ ID NO: 107;
   (8) a fusion protein comprising or consisting of the sequences as set forth in SEQ ID NO: 112, SEQ ID NO: 119, and SEQ ID NO: 107;
   (9) a fusion protein comprising or consisting of the sequence selected from any one of SEQ ID NOs: 120-125 and the sequence selected from any one of SEQ ID NOs: 25, 29, or 95-109;
   (10) a fusion protein comprising or consisting of the sequence selected from any one of SEQ ID NOs: 22, 26, or 156 and the sequence selected from any one of SEQ ID NOs: 126-128;
   (11) a fusion protein comprising or consisting of the sequence as set forth in SEQ ID NO: 129 and the sequence selected from any one of SEQ ID NOs: 23, 25, 27, 29, 95-109, or 157;
   (12) a fusion protein comprising or consisting of the sequences as set forth in SEQ ID NO: 130, and SEQ ID NO: 109, and the sequence selected from any one of SEQ ID NOs: 22, 26, or 156;
   (13) a fusion protein comprising or consisting of the sequence as set forth in SEQ ID NO: 131 and the sequence selected from any one of SEQ ID NOs: 22, 26, or 156;
   (14) a fusion protein comprising or consisting of the sequence as set forth in SEQ ID NO: 132 and the sequence selected from any one of SEQ ID NOs: 22, 26, or 156;
   (15) a fusion protein comprising or consisting of the sequence selected from any one of SEQ ID NO: 22, 26, or 156 and a sequence selected from any one of SEQ ID NOs: 134, 139, 140, or 143;
   (16) a fusion protein comprising or consisting of the sequence as set forth in SEQ ID NO: 141 and a sequence selected from any one of SEQ ID NOs: 25, 29, 95-109, or 157;
   (17) a fusion protein comprising or consisting of the sequence as set forth in SEQ ID NO: 142 and a sequence selected from any one of SEQ ID NOs: 25, 29, 95-109, or 157;
   (18) a fusion protein comprising or consisting of the sequences as set forth in SEQ ID NO: 139 and SEQ ID NO: 141;
   (19) a fusion protein comprising or consisting of the sequences as set forth in SEQ ID NO: 140 and SEQ ID NO: 142;
   (20) a fusion protein comprising or consisting of the sequence selected from any one of SEQ ID NOs: 22, 26, or 156, and the sequences as set forth in SEQ ID NO: 144 and SEQ ID NO: 108;
   (21) a fusion protein comprising or consisting of the sequence selected from any one of SEQ ID NOs: 22, 26, or 156, and the sequences as set forth in SEQ ID NO: 144 and SEQ ID NO: 109;
   (22) a fusion protein comprising or consisting of the sequence selected from any one of SEQ ID NOs: 22, 26, or 156, and the sequences as set forth in SEQ ID NO: 145 and SEQ ID NO: 108;
   (23) a fusion protein comprising or consisting of the sequence selected from any one of SEQ ID NO: 22, 26, or 156, and the sequences as set forth in SEQ ID NO: 145 and SEQ ID NO: 109;
   (24) a fusion protein comprising or consisting of the sequences as set forth in SEQ ID NO: 185, SEQ ID NO: 186, SEQ ID NO: 162, SEQ ID NO: 149, and SEQ ID NO: 161;
   (25) a fusion protein comprising or consisting of an anti-CD3 antibody, an anti-tumor cell surface antigen antibody, an Fc fragment, a cytokine, and a linker, wherein the sequence of the anti-CD3 antibody is selected from the sequence as set forth in any one of SEQ ID NOs: 23, 25, 27, 29, 95-109, or 157, the sequence of the anti-tumor cell surface antigen antibody is selected from the sequence as set forth in any one of SEQ ID NO: 22, 26, or 156, the sequence of the cytokine is selected from the sequence as set forth in any one of SEQ ID NOs: 110, 133, 135, 137, 146-148, 166, or 169-184, and the sequence of the linker is selected from the sequence as set forth in any one of SEQ ID NOs: 1-14, 24, 28, 30, 32, 83-94, or 149-155; preferably, the anti-CD3 antibody is in the form of scFv, and the cytokine is located at the C-terminus of the Fc fragment;
   (26) a fusion protein comprising or consisting of an anti-CD3 antibody, an anti-tumor cell surface antigen antibody, an Fc fragment, a cytokine, a receptor of the cytokine, and a linker, wherein the anti-CD3 antibody comprises the sequences as set forth in SEQ ID NO: 158 and SEQ ID NO: 159, the sequence of the anti-tumor cell surface antigen antibody is selected from the sequence as set forth in any one of SEQ ID NOs: 22, 26, or 156, the sequence of the cytokine is selected from the sequence as set forth in any one of SEQ ID NOs: 110, 133, 135, 137, 147-148, 166, or 171-178, the sequence of the receptor of the cytokine is selected from the sequence as set forth in SEQ ID NOs: 136, 138, 167, or 168, and the sequence of the linker is selected from the sequence as set forth in any one of SEQ ID NOs: 1-14, 24, 28, 30, 32, 83-94, or 149-155; wherein the anti-CD3 antibody is in the form of Fab, and the cytokine and the receptor of the cytokine are located at the N-terminus of the anti-CD3 antibody;
   (27) a fusion protein comprising or consisting of an anti-CD3 antibody, an anti-tumor cell surface antigen antibody, an Fc fragment, a cytokine, a receptor of the cytokine, and a linker, wherein the anti-CD3 antibody comprises the sequences as set forth in SEQ ID NO: 160 and SEQ ID NO: 161, the sequence of the anti-tumor cell surface antigen antibody is selected from the the sequence as set forth in any one of SEQ ID NOs: 22, 26, or 156, the sequence of the cytokine is selected from the sequence as set forth in any one of SEQ ID NOs: 110, 133, 135, 137, 147-148, 166, or 171-178, the sequence of the receptor of the cytokine is selected from the sequence as set forth in SEQ ID NOs: 136, 138, 167, or 168, and the sequence of the linker is selected from the sequence as set forth in any one of SEQ ID NOs: 1-14, 24, 28, 30, 32, 83-94, or 149-155; wherein the anti-CD3 antibody is in the form of Fab, and the cytokine and the receptor of the cytokine are located at the N-terminus of the anti-CD3 antibody;
   (28) a fusion protein comprising or consisting of an anti-CD3 antibody, two anti-tumor cell surface antigen antibodies, an Fc fragment, a cytokine, and a linker, wherein the anti-CD3 antibody comprises the sequences as set forth in SEQ ID NO: 158, SEQ ID NO: 159, SEQ ID NO: 162, and SEQ ID NO: 163, the anti-tumor cell surface antigen antibody comprises the sequence as set forth in SEQ ID NO: 22, the sequence of the cytokine is selected from the sequence as set forth in any one of SEQ ID NOs: 110, 133, 135, 137, 146-148, 166, or 169-184, and the sequence of the linker is selected from the sequence as set forth in any one of SEQ ID NOs: 1-14, 24, 28, 30, 32, 83-94, or 149-155; wherein the anti-CD3 antibody is in the form of Fab, one of the anti-tumor cell surface antigen antibodies is in the form of Fab and is located at the N-terminus of the anti-CD3 antibody, and the cytokine is located at the C-terminus of the anti-CD3 antibody or at the C-terminus of the Fc fragment;
   (29) a fusion protein comprising or consisting of an anti-CD3 antibody, two anti-tumor cell surface antigen antibodies, an Fc fragment, a cytokine, and a linker, wherein the anti-CD3 antibody comprises the sequences as set forth in SEQ ID NO: 160, SEQ ID NO: 161, SEQ ID NO: 162, and SEQ ID NO: 163, the anti-tumor cell surface antigen antibody comprises the sequence as set forth in SEQ ID NO: 22, the sequence of the cytokine is selected from the sequence as set forth in any one of SEQ ID NOs: 110, 133, 135, 137, 146-148, 166, or 169-184, and the sequence of the linker is selected from the sequence as set forth in any one of SEQ ID NOs: 1-14, 24, 28, 30, 32, 83-94, or 149-155; wherein the anti-CD3 antibody is in the form of Fab, one of the anti-tumor cell surface antigen antibodies is in the form of Fab and is located at the N-terminus of the anti-CD3 antibody, and the cytokine is located at the C-terminus of the anti-CD3 antibody;
   (30) a fusion protein comprising or consisting of an anti-CD3 antibody, two anti-tumor cell surface antigen antibodies, an Fc fragment, a cytokine, and a linker, wherein the anti-CD3 antibody comprises the sequences as set forth in SEQ ID NO: 160, SEQ ID NO: 161, SEQ ID NO: 164, and SEQ ID NO: 165, the anti-tumor cell surface antigen antibody comprises the sequence as set forth in SEQ ID NO: 156, the sequence of the cytokine is selected from the sequence as set forth in any one of SEQ ID NOs: 110, 133, 135, 137, 147-148, 166, or 169-184, and the sequence of the linker is selected from the sequence as set forth in any one of SEQ ID NOs: 1-14, 24, 28, 30, 32, 83-94, or 149-155; wherein the anti-CD3 antibody is in the form of Fab, one of the anti-tumor cell surface antigen antibodies is in the form of Fab and is located at the N-terminus of the anti-CD3 antibody, and the cytokine is located at the C-terminus of the anti-CD3 antibody;
   (31) a fusion protein comprising or consisting of an anti-CD3 antibody, two anti-tumor cell surface antigen antibodies, an Fc fragment, a cytokine, and a linker, wherein the anti-CD3 antibody comprises the sequences as set forth in SEQ ID NO: 158, SEQ ID NO: 159, SEQ ID NO: 164, and SEQ ID NO: 165, the anti-tumor cell surface antigen antibody comprises the sequence as set forth in SEQ ID NO: 156, the sequence of the cytokine is selected from the sequence as set forth in any one of SEQ ID NOs: 110, 133, 135, 137, 147-148, 166, or 169-184, and the sequence of the linker is selected from the sequence as set forth in any one of SEQ ID NOs: 1-14, 24, 28, 30, 32, 83-94, or 149-155; wherein the anti-CD3 antibody is in the form of Fab, one of the anti-tumor cell surface antigen antibodies is in the form of Fab and is located at the N-terminus of the anti-CD3 antibody, and the cytokine is located at the C-terminus of the anti-CD3 antibody;
   (32) a fusion protein comprising or consisting of an anti-CD3 antibody, two anti-tumor cell surface antigen antibodies, an Fc fragment, a cytokine, a receptor of the cytokine, and a linker, wherein the anti-CD3 antibody comprises the sequences as set forth in SEQ ID NO: 158, SEQ ID NO: 159, SEQ ID NO: 162, and SEQ ID NO: 163, the anti-tumor cell surface antigen antibody comprises the sequence as set forth in SEQ ID NO: 22, the sequence of the cytokine is selected from the sequence as set forth in any one of SEQ ID NOs: 110, 133, 135, 137, 147-148, 166, or 171-178, the sequence of the receptor of the cytokine is selected from the sequence as set forth in SEQ ID NOs: 136, 138, 167, or 168, and the sequence of the linker is selected from the sequence as set forth in any one of SEQ ID NOs: 1-14, 24, 28, 30, 32, 83-94, or 149-155; wherein the anti-CD3 antibody is in the form of Fab, one of the anti-tumor cell surface antigen antibodies is in the form of Fab and is located at the N-terminus of the anti-CD3 antibody;
      wherein the cytokine and the receptor of the cytokine are located at the C-terminus of the Fc fragment, respectively, or located at the C-terminus of the anti-CD3 antibody and the C-terminus of the anti-tumor cell surface antigen antibody in the form of scFv, respectively; or
      wherein the cytokine and the receptor of the cytokine are linked together and the receptor of the cytokine is linked to the C-terminus of the Fc fragment or the C-terminus of the anti-CD3 antibody;
   (33) a fusion protein comprising or consisting of an anti-CD3 antibody, two anti-tumor cell surface antigen antibodies, an Fc fragment, a cytokine, a receptor of the cytokine, and a linker, wherein the anti-CD3 antibody comprises the sequences as set forth in SEQ ID NO: 160, SEQ ID NO: 161, SEQ ID NO: 162, and SEQ ID NO: 163, the anti-tumor cell surface antigen antibody comprises the sequence as set forth in SEQ ID NO: 22, the sequence of the cytokine is selected from the sequence as set forth in any one of SEQ ID NOs: 110, 133, 135, 137, 147-148, 166, or 171-178, the sequence of the receptor of the cytokine is selected from the sequence as set forth in SEQ ID NO: 136, 138, 167, or 168, and the sequence of the linker is selected from the sequence as set forth in any one of SEQ ID NOs: 1-14, 24, 28, 30, 32, 83-94, or 149-155; wherein the anti-CD3 antibody is in the form of Fab, one of the two anti-tumor cell surface antigen antibodies is in the form of Fab and is located at the N-terminus of the anti-CD3 antibody;
      wherein the cytokine and the receptor of the cytokine are located at the C-terminus of the Fc fragment, respectively, or located at the C-terminus of the anti-CD3 antibody and the C-terminus of the anti-tumor cell surface antigen antibody in the form of scFv, respectively; or
      wherein the cytokine and the receptor of the cytokine are linked together and the receptor of the cytokine is linked to the C-terminus of the Fc fragment or the C-terminus of the anti-CD3 antibody;
   (34) a fusion protein comprising or consisting of an anti-CD3 antibody, two anti-tumor cell surface antigen antibodies, an Fc fragment, a cytokine, a receptor of the cytokine, and a linker, wherein the anti-CD3 antibody comprises the sequences as set forth in SEQ ID NO: 160, SEQ ID NO: 161, SEQ ID NO: 164, and SEQ ID NO: 165, the anti-tumor cell surface antigen antibody comprises the sequence as set forth in SEQ ID NO: 156, the sequence of the cytokine is selected from the sequence as set forth in any one of SEQ ID NOs: 110, 133, 135, 137, 147-148, 166, or 171-178, the sequence of the receptor of the cytokine is selected from the sequence as set forth in SEQ ID NO: 136, 138, 167, or 168, and the sequence of the linker is selected from the sequence as set forth in any one of SEQ ID NOs: 1-14, 24, 28, 30, 32, 83-94, or 149-155; wherein the anti-CD3 antibody is in the form of Fab, one of the anti-tumor cell surface antigen antibodies is in the form of Fab and is located at the N-terminus of the anti-CD3 antibody;
      wherein the cytokine and the receptor of the cytokine are located at the C-terminus of the Fc fragment, respectively, or located at the C-terminus of the anti-CD3 antibody and the C-terminus of the anti-tumor cell surface antigen antibody in the form of scFv, respectively; or
      wherein the cytokine and the receptor of the cytokine are linked together and the receptor of the cytokine is linked to the C-terminus of the Fc fragment or the C-terminus of the anti-CD3 antibody;
   (35) a fusion protein comprising or consisting of an anti-CD3 antibody, two anti-tumor cell surface antigen antibodies, an Fc fragment, a cytokine, a receptor of the cytokine, and a linker, wherein the anti-CD3 antibody comprises the sequences as set forth in SEQ ID NO: 158, SEQ ID NO: 159, SEQ ID NO: 164, and SEQ ID NO: 165, the anti-tumor cell surface antigen antibody comprises the sequence as set forth in SEQ ID NO: 156, the sequence of the cytokine is selected from the sequence as set forth in any one of SEQ ID NOs: 110, 133, 135, 137, 147-148, 166, or 171-178, the sequence of the receptor of the cytokine is selected from the sequence as set forth in SEQ ID NO: 136, 138, 167, or 168, and the sequence of the linker is selected from the sequence as set forth inany one of SEQ ID NOs: 1-14, 24, 28, 30, 32, 83-94, or 149-155; wherein the anti-CD3 antibody is in the form of Fab, one of the anti-tumor cell surface antigen antibodies is in the form of Fab and is located at the N-terminus of the anti-CD3 antibody;
      wherein the cytokine and the receptor of the cytokine are located at the C-terminus of the Fc fragment, respectively, or located at the C-terminus of the anti-CD3 antibody and the C-terminus of the anti-tumor cell surface antigen antibody in the form of scFv, respectively; or
      wherein the cytokine and the receptor of the cytokine are linked together and the receptor of the cytokine is linked to the C-terminus of the Fc fragment or the C-terminus of the anti-CD3 antibody;
   (36) a fusion protein comprising the sequences as set forth in SEQ ID NO: 22, SEQ ID NO: 160, and SEQ ID NO: 161;
   (37) a fusion protein comprising the sequence as set forth in SEQ ID NO: 22, sequence a, and sequence b, wherein sequence a comprises, from the N-terminus to the C-terminus, the sequences as set forth in SEQ ID NO: 163, SEQ ID NO: 149, and SEQ ID NO: 160; and sequence b comprises, from the N-terminus to the C-terminus, the sequences as set forth in SEQ ID NO: 162, SEQ ID NO: 149, and SEQ ID NO: 161;
   (38) a fusion protein comprising sequence a, sequence b, and sequence c, wherein sequence a comprises, from the N-terminus to the C-terminus, the sequences as set forth in SEQ ID NO: 22, SEQ ID NO: 3, and SEQ ID NO: 110; sequence b comprises, from the N-terminus to the C-terminus, the sequences as set forth in SEQ ID NO: 163, SEQ ID NO: 149, and SEQ ID NO: 160; and sequence c comprises, from the N-terminus to the C-terminus, the sequences as set forth in SEQ ID NO: 162, SEQ ID NO: 149, and SEQ ID NO: 161;
   (39) a fusion protein comprising sequence a, sequence b, and sequence c, wherein sequence a comprises, from the N-terminus to the C-terminus, the sequences as set forth in SEQ ID NO: 22, SEQ ID NO: 3, and SEQ ID NO: 176; sequence b comprises, from the N-terminus to the C-terminus, the sequences as set forth in SEQ ID NO: 163, SEQ ID NO: 149, and SEQ ID NO: 160; and sequence c comprises, from the N-terminus to the C-terminus, the sequences as set forth in SEQ ID NO: 162, SEQ ID NO: 149, and SEQ ID NO: 161;
   (40) a fusion protein comprising sequence a, and the sequences as set forth in SEQ ID NO: 160 and SEQ ID NO: 161, wherein sequence a comprises, from the N-terminus to the C-terminus, the sequences as set forth in SEQ ID NO: 22, SEQ ID NO: 3, and SEQ ID NO: 176;
   (41) a fusion protein comprising the sequence as set forth in SEQ ID NO: 22, sequence a, and sequence b, wherein sequence a comprises, from the N-terminus to the C-terminus, the sequences as set forth in SEQ ID NO: 167, SEQ ID NO: 149, and SEQ ID NO: 160; and sequence b comprises, from the N-terminus to the C-terminus, the sequences as set forth in SEQ ID NO: 110, SEQ ID NO: 32, and SEQ ID NO: 161;
   (42) a fusion protein comprising the sequence as set forth in SEQ ID NO: 22, sequence a, and sequence b, wherein sequence a comprises, from the N-terminus to the C-terminus, the sequences as set forth in SEQ ID NO: 163, SEQ ID NO: 149, and SEQ ID NO: 160; and sequence b comprises, from the N-terminus to the C-terminus, the sequences as set forth in SEQ ID NO: 162, SEQ ID NO: 149, and SEQ ID NO: 161;
   (43) a fusion protein comprising the sequences as set forth in SEQ ID NO: 185 and SEQ ID NO: 186, and sequence a, wherein sequence a comprises, from the N-terminus to the C-terminus, the sequences as set forth in SEQ ID NO: 162, SEQ ID NO: 149, and SEQ ID NO: 161;
   (44) a fusion protein comprising sequence a, sequence b, and sequence c, wherein sequence a comprises, from the N-terminus to the C-terminus, the sequences as set forth in SEQ ID NO: 22, SEQ ID NO: 3, and SEQ ID NO: 137; sequence b comprises, from the N-terminus to the C-terminus, the sequences as set forth in SEQ ID NO: 163, SEQ ID NO: 149, and SEQ ID NO: 160; and sequence c comprises, from the N-terminus to the C-terminus, the sequences as set forth in SEQ ID NO: 162, SEQ ID NO: 149, and SEQ ID NO: 161;
   (45) a fusion protein comprising the sequence as set forth in SEQ ID NO: 22 and sequence a, wherein sequence a comprises, from the N-terminus to the C-terminus, the sequences as set forth in SEQ ID NO: 23, SEQ ID NO: 3, and SEQ ID NO: 137;
   (46) a fusion protein comprising the sequences as set forth in SEQ ID NO: 22 and SEQ ID NO: 23;
   (47) a fusion protein comprising the sequences as set forth in SEQ ID NO: 22 and SEQ ID NO: 157;
   (48) a fusion protein comprising the sequences as set forth in SEQ ID NO: 129 and SEQ ID NO: 157;
   (49) a fusion protein comprising the sequence as set forth in SEQ ID NO: 22 and sequence a, wherein sequence a comprises, from the N-terminus to the C-terminus, the sequences as set forth in SEQ ID NO: 181, SEQ ID NO: 32, SEQ ID NO: 149, and SEQ ID NO: 157;
   (50) a fusion protein comprising the sequences as set forth in SEQ ID NO: 22 and SEQ ID NO: 116;
   (51) a fusion protein comprising the sequence as set forth in SEQ ID NO: 22 and sequence a, wherein sequence a comprises, from the N-terminus to the C-terminus, the sequences as set forth in SEQ ID NO: 184, SEQ ID NO: 3, and SEQ ID NO: 23; or
   (52) a fusion protein comprising the sequence as set forth in SEQ ID NO: 22 and sequence a, wherein sequence a comprises, from the N-terminus to the C-terminus, the sequences as set forth in SEQ ID NO: 184, SEQ ID NO: 32, SEQ ID NO: 149, and SEQ ID NO: 23.
22. A biological material selected from the group consisting of:
   (1) a polynucleotide encoding the fusion protein according to any one of items 1 to 21;
   (2) a vector comprising the fusion protein according to any one of items 1 to 21; and
   (3) a host cell comprising the fusion protein according to any one of items 1-21.
23. A conjugate comprising the fusion protein according to any one of items 1 to 21 and a conjugated moiety, wherein the conjugated moiety is selected from a purification tag (such as a His tag, a streptavidin tag), a cytotoxic agent, a detectable label, a radioisotope, a luminescent substance, a colored substance, an enzyme, or polyethylene glycol.
24. A pharmaceutical composition comprising the fusion protein according to any one of items 1 to 21 and a pharmaceutical carrier, preferably, the pharmaceutical composition is in a dosage form suitable for oral administration to the gastrointestinal (GI) tract; preferably, the dosage form is selected from a tablet, a capsule, a pill, a powder, a granule, an emulsion, a microemulsion, a solution, a suspension, a syrup, and an elixir; or the pharmaceutical composition is in a dosage form suitable for administration by subcutaneous injection, intradermal injection, intravenous injection, intramuscular injection, or intralesional injection.
25. A kit comprising the fusion protein according to any one of items 1 to 21 and a therapeutic agent for treating a tumor, preferably, the therapeutic agent for treating a tumor is selected from a chemotherapeutic agent, a targeted therapeutic agent, T cells expressing a chimeric antigen receptor, or an angiogenesis inhibitor.
26. Use of the fusion protein according to any one of items 1 to 21 in the treatment of a tumor, or in the manufacture of a medicament for the treatment of a tumor.
27. Use according to item 26, wherein the tumor is selected from gastric cancer, pancreatic cancer, lung cancer, colon cancer, melanoma, microglioma.

### DEFINITIONS

The antibodies described herein may be derived from any animal source, including birds and mammals, including primates. Preferably, the antibodies are human, baboon, rhesus monkey, cynomolgus monkey, murine, donkey, rabbit, goat, guinea pig, camel, llama, horse, or chicken antibodies.

The antibodies involved in the present disclosure are IgG antibodies, wherein the CDRs (based on the heavy chain variable region and light chain variable region, a person of ordinary skill in the art can define the CDR sequences according to known numbering systems in the art, such as the Kabat numbering system, the IMGT numbering system, or the Chothia numbering system), the heavy chain variable regions, the light chain variable regions, the light chain constant regions, the heavy chain constant regions, the hinge regions, and the disulfide bonds are all terms well known in the art.

As used herein, the term "detectable lable" refers to a compound or composition that can be detected directly or indirectly, wherein the compound or composition binds directly or indirectly to a composition to be detected (e.g., a polynucleotide or a protein, such as an antibody), so as to obtain a "labelled" composition. The term also includes a sequence that binds to the polynucleotide and provide a signal through the expression of the inserted sequence, such as green fluorescent protein (GFP). The label itself may be detectable (e.g., a radioisotope label or a fluorescent label), or, in the case of an enzyme label, it may catalyze a chemical change of a substrate compound or composition, and the change is detectable. The label may be used for small-scale detection or may be more suitable for high-throughput screening. Likewise, suitable labels include, but are not limited to, radioisotopes, fluorescent dyes, chemiluminescent compounds, dyes, and proteins (including enzymes). The label may be detected only or may be quantified. Reactions that are only detected generally include those where the presence thereof can only be confirmed, whereas reactions that can be quantified generally include those that have quantifiable (e.g., numerically reportable) values such as intensity, polarization, and/or other properties. In luminescence or fluorescence assays, the luminophores or fluorophores that are associated with the analyzed components and are actually involved in binding can be directly used in the detectable reaction, or the luminophores or fluorophores linked to another component (e.g., a reporter molecule or indicator) can be indirectly used in the detectable reaction.

The term "single-chain variable fragment" or "scFv" refers to a fusion protein of variable regions of the heavy chain (VH) and light chain (VL) of an immunoglobulin. In some respects, these regions are linked by a short linker peptide of 10 to about 25 amino acids. This linker may be glycine-rich for flexibility, or contain serine or threonine for solubility, and can link the N-terminus of VH to the C-terminus of VL, and vice versa. The protein retains the characteristics of the original immunoglobulin, with only the constant regions removed and the linker introduced. ScFv molecules are known in the art, such as those described in U.S. Patent 5,892,019.

As used herein, the term "antibody fragment" or "antigen-binding fragment" refers to a portion of an antibody, such as F(ab')₂, F(ab)₂, Fab', Fab, Fv, scFv, and the like.

A person of ordinary skill in the art understands that the term "cleavable linker" refers to some amino acid sequences that can be cleaved in the middle after being recognized by certain enzymes in vivo or in vitro, such as selected from MMP enzymes, uPA enzymes, serine hydrolases, and the like, thereby allowing the sequences linked at both ends to be separated.

As used herein, the term "host cell" refers to a cell into which a vector can be introduced, including, but not limited to, prokaryotic cells such as *Escherichia coli* or *Bacillus subtilis*; fungal cells such as yeast cells or Aspergillus cells; insect cells such as S2 Drosophila cells or Sf9 cells; or animal cells such as fibroblasts, CHO cells, COS cells, NSO cells, HeLa cells, GS cells, BHK cells, HEK 293 cells, or human cells.

As used herein, the term "specific binding" refers to a non-random binding reaction between two molecules, such as the reaction between an antibody and its target antigen. In certain embodiments, an antibody that specifically binds to an antigen (or an antibody having specificity for a certain antigen) refers to an antibody that binds to the antigen with an affinity (KD) of less than about 10⁻⁵ M, for example, less than about 10⁻⁶ M, 10⁻⁷ M, 10⁻⁸ M, 10⁻⁹ M, or 10⁻¹⁰ M or even lower.

As used herein, the term "K_{D}" refers to the equilibrium dissociation constant of a particular antibody-antigen interaction, which is used to describe the binding affinity between an antibody and an antigen. The lower the equilibrium dissociation constant, the tighter the antibody-antigen binding, and the higher the affinity between the antibody and the antigen. Generally, an antibody binds to an antigen (e.g., the PD-1 protein) with an equilibrium dissociation constant (K_{D}) of less than about 10⁻⁵ M, for example, less than about 10⁻⁶ M, 10⁻⁷ M, 10⁻⁸ M, 10⁻⁹ M, or 10⁻¹⁰ M or even lower. The K_{D} can be determined by methods known to those skilled in the art, for example, using a Fortebio molecular interaction Analyzer.

In the present invention, structural unit A is a bispecific antibody. In some embodiments, the bispecific antibody is in the form of a bispecific antibody prodrug, wherein the anti-CD3 antibody is linked to a cytokine, a pro-cytokine, or a tumor cell targeting peptide via a cleavable linker, and the activity of the anti-CD3 antibody is restored upon cleavage of the cytokine, the pro-cytokine, or the tumor cell targeting peptide.

### DESCRIPTION OF THE DRAWINGS

Figure 1. Schematic diagrams of several structures of bispecific antibodies that bind to the same tumor surface-specific antigen.
Figure 2. Schematic diagrams of several structures of bispecific antibodies that bind to different tumor surface-specific antigens.
Figure 3. Schematic diagrams of several structures of bispecific antibodies that comprise a tumor-targeting peptide and an antibody that binds to a tumor surface-specific antigen.
Figure 4. Schematic diagrams of several structures of bispecific antibodies that comprise tandem forms of a tumor-targeting peptide and an antibody that binds to a tumor surface-specific antigen.
Figure 5. SEC-HPLC analysis of the purity of bispecific antibodies.
Figure 6. Flow cytometric analysis of the binding ability of bispecific antibodies to CLDN18.2 antigen overexpressed on the colorectal cancer cell line MC38. The bispecific antibodies used are PDL1×CD3 (UCHT1) having the structure as shown in Figure 2A, PDL1×CLDN-CD3 (UCHT1) having the structure as shown in Figure 2D, CLDN×CD3 (UCHT1) having the structure as shown in Figure 1A, and CLDN×CLDN-CD3 (UCHT1) having the structure as shown in Figure 1A.
Figure 7. SDS-PAGE analysis of the cleavage efficiency and result of the fusion protein by the MMP14 enzyme. The bispecific antibody used is PDL1×CLDN-MMP(double)-CD3 (UCHT1) having the structure as shown in Figure 2D. Lane 1 shows the electrophoresis result of PDL1×CLDN-MMP(double)-CD3 (UCHT1) alone; Lane 2 shows the electrophoresis result of PDL1×CLDN-MMP(double)-CD3 (UCHT1) after overnight cleavage with the MMP14 enzyme; and Lane 3 shows the electrophoresis result of PDL1×CD3 (UCHT1) having the structure as shown in Figure 1A alone.
Figure 8. Assessment of the recovery of biological activity of the fusion protein with or without enzymatic cleavage using spleen cells from Human CD3 KI mice. The bispecific antibodies used are PDL1×CLDN-MMP(double)-CD3 (UCHT1) N having the structure as shown in Figure 2D, PDL1×CLDN-MMP(double)-CD3 (UCHT1) C having the structure as shown in Figure 2D, and PDL1×CD3 (UCHT1) having the structure as shown in Figure 2A, wherein N represents the absence of MMP cleavage and C represents the presence of MMP cleavage.
Figure 9. Compared with the bispecific antibody CLDN×CD3, the bispecific antibody of the present invention exhibits superior in vivo anti-tumor efficacy. The bispecific antibodies used are CLDN×CD3 (UCHT1) having the structure as shown in Figure 1A, and CLDN×CLDN-CD3 (UCHT1) having the structure as shown in Figure 1D.

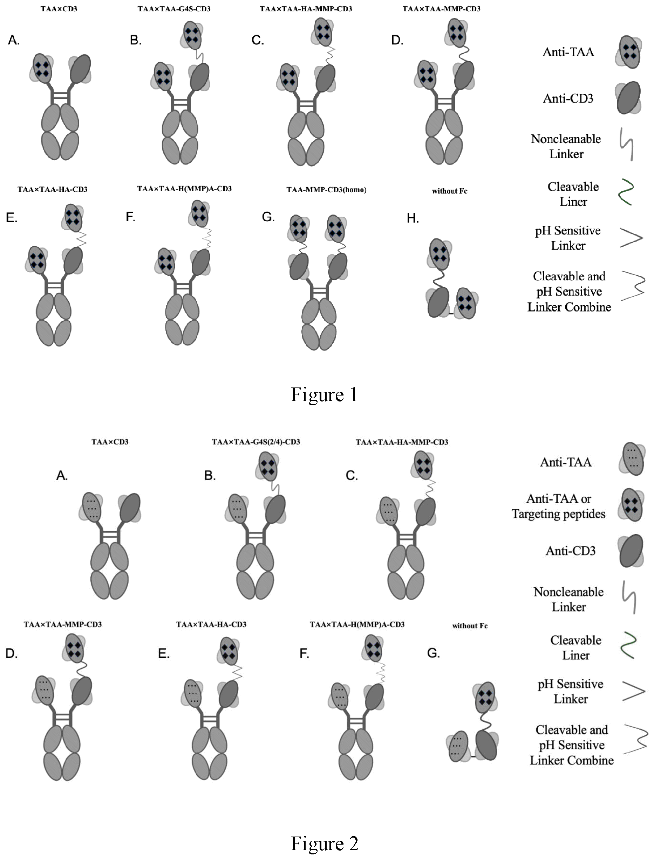

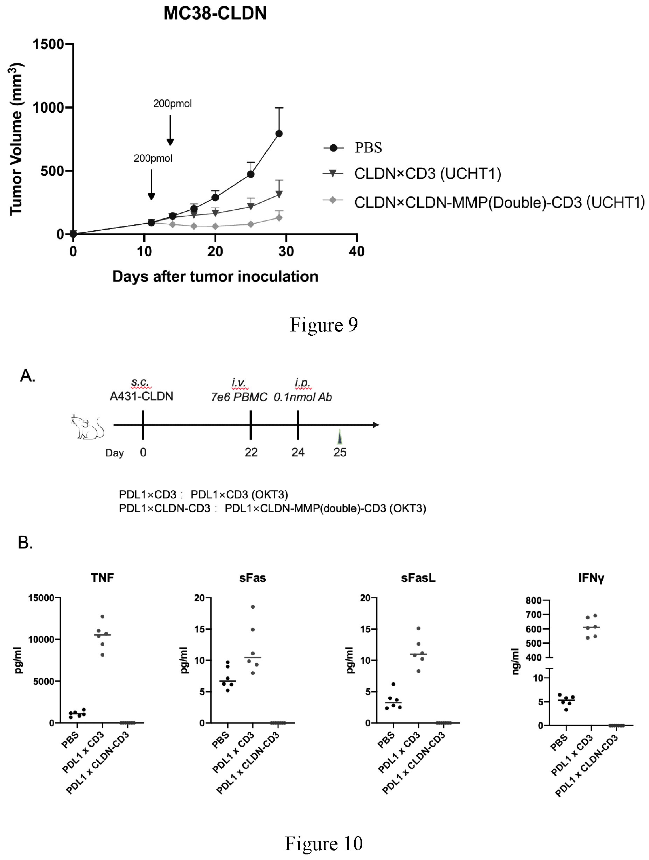

Figure 10. The experimental procedure is shown in Figure 10A. Peripheral blood was collected 24 hours post-administration, and serum was isolated for cytokine detection. Figure 10B shows the cytokine levels in the peripheral blood serum. The results demonstrated that, the secretion levels of inflammatory cytokines (including IL-6, IFN, TNF, etc.) were lower in the serum of the bispecific antibody PDL1×CLDN-MMP(double)-CD3 (OKT3) having the structure as shown in Figure 2D compared with that of the bispecific antibody PDL1×CD3, indicating improved better safety. The bispecific antibody used is PDL1×CD3 (OKT3) having the structure as shown in Figure 1A.
Figure 11. SDS-PAGE profiles of the bispecific antibodies with or without MMP14 enzymatic cleavage. Wherein, Figure 11A shows the SDS-PAGE profile of PDL1×CLDN-MMP(double)-CD3 (OKT3) having the structure as shown in Figure 2D, with (+) or without (-) MMP14 enzymatic cleavage, wherein Lanes 3, 4, 6, and 7 from the left are irrelevant samples; and Figure 11B shows the SDS-PAGE profile of PDL1×targeting peptide-HA-MMP(double)-CD3 (OKT3) having the structure as shown in Figure 3C, with (+) or without (-) MMP14 enzymatic cleavage, wherein Lanes 3 and 4 from the left are irrelevant samples.
Figure 12. Activation of PBMCs by bispecific antibodies with or without MMP14 enzymatic cleavage. Wherein, Figure 12A shows the activation of PBMCs by PDL1×CLDN-MMP(double)-CD3 (OKT3) having the structure as shown in Figure 2D with or without MMP14 enzymatic cleavage; and Figure 12B shows the activation of PBMCs by PDL1×NGR-HA-MMP(double)-CD3 (OKT3) having the structure as shown in Figure 3C with or without MMP14 enzymatic cleavage, wherein N represents the absence of MMP cleavage and C represents the presence of MMP cleavage.

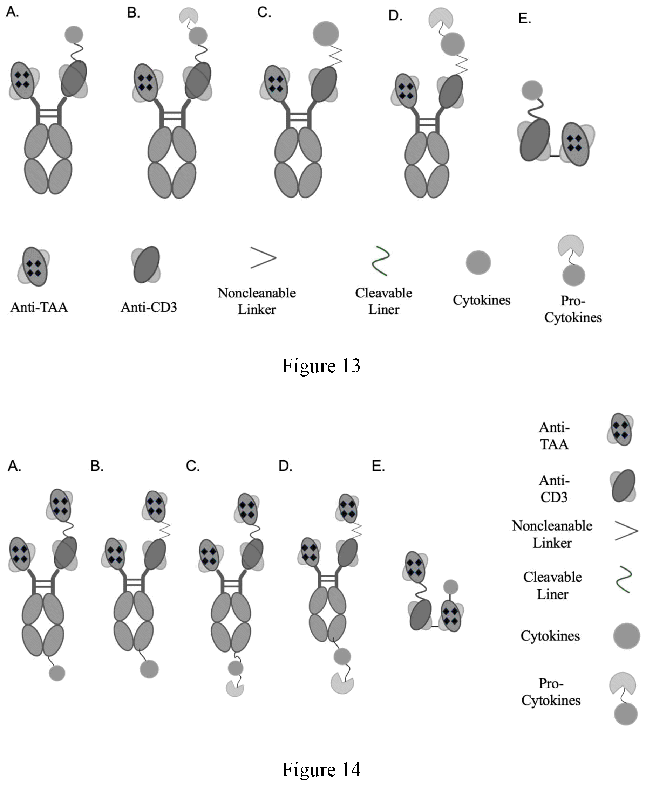

Figure 13. Exemplary structural diagrams of the fusion proteins of the present invention. Wherein, Figure 13A shows that a cytokine is linked to an anti-CD3 antibody via a cleavable linker; Figure 13B shows that a pro-cytokine is linked to an anti-CD3 antibody via a cleavable linker; Figure 13C shows that a cytokine is linked to an anti-CD3 antibody via a non-cleavable linker; Figure 13D shows that a pro-cytokine is linked to an anti-CD3 antibody via a non-cleavable linker; and Figure 13E shows that a cytokine is linked to an anti-CD3 antibody via a cleavable linker, wherein the anti-CD3 antibody and the anti-tumor cell surface antigen antibody are in the form of scFv and are linked in tandem.
Figure 14. Exemplary structural diagrams of the fusion proteins of the present invention. Wherein, Figure 14A shows that an anti-CD3 antibody is linked to an anti-TAA (or a tumor cell targeting peptide) via a cleavable linker, and a cytokine is linked to the C-terminus of an Fc fragment; Figure 14B shows that an anti-CD3 antibody is linked to an anti-TAA (or a tumor cell targeting peptide) via a non-cleavable linker, and a cytokine is linked to the C-terminus of an Fc fragment; Figure 14C shows that an anti-CD3 antibody is linked to an anti-TAA (or a tumor cell targeting peptide) via a cleavable linker, and a pro-cytokine is linked to the C-terminus of an Fc fragment; Figure 14D shows that an anti-CD3 antibody is linked to an anti-TAA (or a tumor cell targeting peptide) via a non-cleavable linker, and a pro-cytokine is linked to the C-terminus of an Fc fragment; and Figure 14E shows that an anti-CD3 antibody is linked to an anti-TAA (or a tumor cell targeting peptide) via a cleavable linker, and in the absence of an Fc fragment, a cytokine is linked in tandem to the anti-TAA (or the tumor cell-targeting peptide).

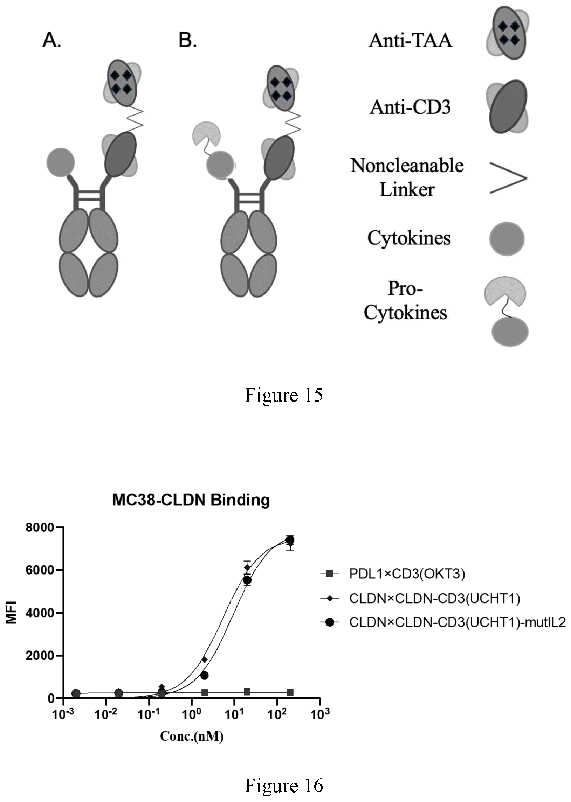

Figure 15. Exemplary structural diagrams of the fusion proteins of the present invention. Wherein, Figure 15A shows that an anti-CD3 antibody is linked to one chain of an Fc fragment and is linked to an anti-TAA (or a tumor cell targeting peptide) via a non-cleavable linker, and a cytokine is linked to the other chain of the Fc fragment, forming a heterodimer; and Figure 15B shows that an anti-CD3 antibody is linked to one chain of an Fc fragment and is linked to an anti-TAA (or a tumor cell targeting peptide) via a non-cleavable linker, and a pro-cytokine is linked to the other chain of the Fc fragment, forming a heterodimer.
Figure 16. Flow cytometric analysis of the binding ability of the exemplary fusion protein of the present invention to CLDN18.2 antigen overexpressed on the colorectal cancer cell line MC38.
Figure 17. In vivo anti-tumor efficacy of exemplary fusion proteins of the present invention. Figure 17A shows changes in tumor volume, and Figure 17B shows changes in body weight.

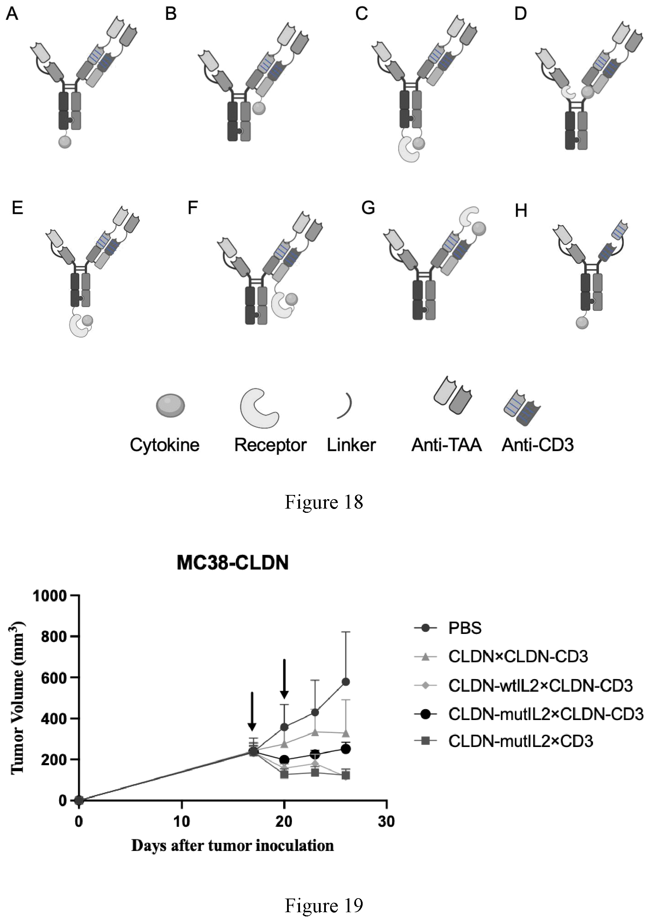

Figure 18. Exemplary structural diagrams of the fusion proteins of the present invention. Wherein, Figure 18A shows that a cytokine is fused to the C-terminus of the heavy chain of an anti-CD3 antibody (and may also be fused to the C-terminus of the other chain); Figure 18B shows that a cytokine is fused to the C-terminus of the light chain of an anti-CD3 antibody; Figure 18C shows that a cytokine and a receptor are separately fused to the C-termini of the heavy chains of an anti-TAA antibody (wherein the positions of the cytokine and the receptor can be interchanged); Figure 18D shows that a cytokine and a receptor of the cytokine are separately fused between the hinge region of an Fc fragment and the heavy and light chains of an anti-CD3 antobody and an anti-TAA antibody (wherein the positions of the cytokine and the receptor can be interchanged); Figure 18E shows that a cytokine and a receptor are fused to the C-terminus of the heavy chain of an anti-TAA antibody (wherein the positions of the cytokine and the receptor can be interchanged); Figure 18F shows that a cytokine and a receptor of the cytokine are fused to the C-terminus of the light chain of an anti-CD3 antibody (wherein the positions of the cytokine and the receptor can be interchanged); Figure 18G shows that a cytokine and a receptor are separately fused to the N-termini of the heavy and light chains of an anti-CD3 antibody (wherein the positions of the cytokine and the receptor can be interchanged); and Figure 18H shows that a cytokine is fused to the C-terminus of the light and heavy chains of an anti-TAA (and may also be fused to the C-terminus of the other chain).
Figure 19. In vivo anti-tumor efficacy of the exemplary IL2 fusion proteins of the present invention.
Figure 20. In vivo anti-tumor efficacy of an exemplary IL2 and IL2 receptor Ra fusion protein of the present invention in a PBMC-humanized model.
Figure 21. In vivo anti-tumor efficacy of an exemplary IL15 fusion protein of the present invention.
Figure 22. In vivo anti-tumor efficacy of an exemplary IL15 fusion protein without structural unit 3 of the present invention.
Figure 23. In vivo anti-tumor efficacy (Figure 23A) and the cytokine levels in peripheral blood serum (Figure 23B) of an exemplary IL21 fusion protein of the present invention.
Figure 24. In vivo anti-tumor efficacy (Figure 24A) and the cytokine levels in peripheral blood serum (Figure 24B) of an exemplary IL4 fusion protein of the present invention.
Figure 25. Binding ability of the exemplary IL10 fusion proteins having the structures as shown in Figures 13A and 13C of the present invention to PBMCs.
Figure 26. In vivo anti-tumor efficacy (Figure 26A) and the cytokine levels in peripheral blood serum (Figure 26B) of the exemplary IL10 fusion proteins of the present invention having the structures as shown in Figures 13A and 13C.

### DETAILED DESCRIPTION OF THE INVENTION

The present invention is further described in detail by the following examples. Those skilled in the art will appreciate that the present invention is not limited to the following content. The scope of the present invention is defined by the claims. Where the specific techniques or conditions are not specified in the examples, the procedures are carried out according to techniques or conditions described in the literature in the art (e.g., with reference to Molecular Cloning: A Laboratory Manual, 3rd edition, by J. Sambrook et al., translated by Huang Peitang et al., Science Press) or according to the product manual. Where the manufacturers of reagents or instruments used are not specified, they are the conventional products commercially available.

### Experimental Animals

Human CD3 KI (i.e., hCD3EDG) mice were purchased from GemPharmatech Co., Ltd. NSG-SGM3 mice were imported from JAX in the United States and bred in-house. Wild-type C57BL/6 mice were purchased from Beijing Vital River Laboratory Animal Technology Co., Ltd. Unless otherwise specified, all mice used in the experiments were 8 to 10 weeks of age. All mice were housed in a specific pathogen-free (SPF) barrier environment. Animal housing and experimental procedures were performed in accordance with the regulations of the Institutional Animal Care and Use Committee of Beijing Changping Laboratory.

### Cell Lines

The FreeStyle^{™} 293F cell line (Invitrogen) is a suspension cell line derived from the HEK293 cell line and was cultured in SMM293-TII medium for transient transfection and expression of the fusion proteins.

A549 is a human non-small cell lung cancer cell line, and MC38 is a mouse colorectal cancer cell line with a C57 background; both of which were purchased from ATCC. A549-CLDN and MC38-CLDN cell lines (i.e., the cell lines overexpressing CLDN) were established by the lentiviral transduction to overexpress the mouse CLDN18.2 protein (P56856), and cultured in DMEM complete medium containing 10% inactivated fetal bovine serum, 2 mmol/L L-glutamine, 0.1 mmol/L non-essential amino acids, 100 U/mL penicillin, and 100 µg/mL streptomycin.

### Biostatistical Analysis

All data were analyzed using GraphPad Prism statistical software. Mouse survival curves were analyzed by one-way ANOVA, and other data were analyzed by two-tailed t-test.

### Example 1. Construction, Expression and Purification of Fusion Proteins

### 1. Construction of BiTEs:

To design a tumor-targeting bispecific antibody fusion protein with high efficacy and low toxicity, on the basis of a T cell-bridging bispecific T-cell engager (BiTE) (Figure 1A), the sequences of an antibody that binds to a tumor surface-specific antigen (anti-TAA) (or a tumor cell targeting peptide) and an anti-CD3 antibody were linked together via various linkers to mask the activity of the anti-CD3 antibody by the anti-TAA or the tumor cell targeting peptide, wherein the linkers were selected from the group consisting of:
i. the non-cleavable linkers (e.g., the GS linkers with various lengths (SEQ ID NOs: 1-3, 24, 28, 30, and 32), Figure 1B);
ii. the linkers formed by the tandem linkage of a cleavable linker and a pH-sensitive linker (e.g., a polypeptide substrate cleavable by MMP and an HA linker, SEQ ID NOs: 4-5, Figure 1C);
iii. the cleavable linkers (e.g., the polypeptide substrates cleavable by MMP (SEQ ID NOs: 6-9), Figure 1D);
iv. the linkers formed by tandem linkage of the pH-sensitive linkers (e.g., multiple HA linkers (SEQ ID NOs: 10-12), Figure 1E);
v. the linkers formed by inserting a cleavable linker into the middle of a pH-sensitive linker (e.g., an alternating arrangement of a polypeptide substrate sequence cleavable by MMP and an HA linker (SEQ ID NOs: 13-14), Figure 1F);
vi. a homo form in which the anti-TAA and anti-CD3 antibody sequences are linked together via a cleavable linker at one terminus of the Fc fragment (SEQ ID NOs: 19-20), as shown in Figure 1G; and
vii. a cleavable linker (e.g., a polypeptide substrate cleavable by MMP (SEQ ID NOs: 6-9)) that linking structural unit 1 and structural unit 3 in the absence of Fc fragment (SEQ ID NOs: 19-20), as shown in Figure 1H.

Figure 2 shows the BiTE structures, wherein Figure 2A shows a general BiTE structure comprising a CD3-binding domain and a TAA-binding domain; Figure 2B shows that, based on the structure of Figure 2A, the CD3-binding domain is linked to an anti-TAA (or a tumor cell targeting peptide) via a non-cleavable linker; Figure 2C shows that, based on the structure of Figure 2A, the CD3-binding domain is linked to an anti-TAA (or a tumor cell targeting peptide) via a cleavable linker and a pH-sensitive linker; Figure 2D shows that, based on the structure of Figure 2A, the CD3-binding domain is linked to an anti-TAA (or a tumor cell targeting peptide) via a cleavable linker; Figure 2E shows that, based on the structure of Figure 2A, the CD3-binding domain is linked to an anti-TAA (or a tumor cell targeting peptide) via a pH-sensitive linker; Figure 2F shows that, based on the structure of Figure 2A, the CD3-binding domain is linked to an anti-TAA (or a tumor cell targeting peptide) via a cleavable linker and a pH-sensitive linker arranged alternately; and Figure 2G shows that, based on the structure of Figure 2D, the CD3-binding domain is linked to an anti-TAA (or a tumor cell targeting peptide) via a cleavable linker and a pH-sensitive linker in the absence of an Fc fragment.

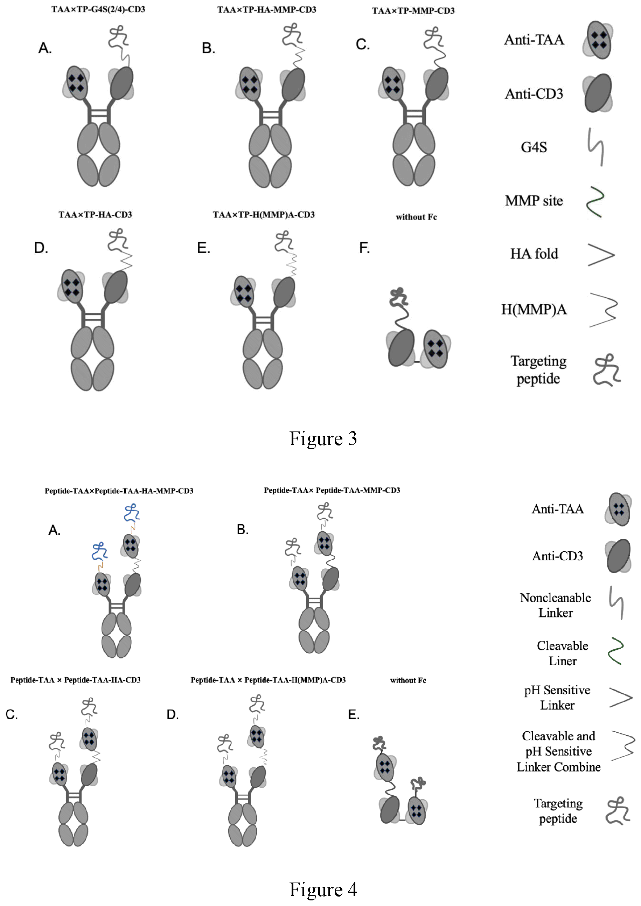

Figure 3 shows that the BiTE comprises two different tumor antigen-binding moieties, wherein one is a tumor cell targeting peptide (e.g., NGR, EphA2, EphA4) used for masking the anti-CD3 activity, and the other is an anti-TAA having a non-masking function. Figure 3A shows that, based on the basic BiTE structure, the CD3-binding domain is linked to structural unit 3 via a non-cleavable linker; Figure 3B shows that, based on the basic BiTE structure, the CD3-binding domain is linked to structural unit 3 via a cleavable linker and a pH-sensitive linker in tandem; Figure 3C shows that, based on the basic BiTE structure, the CD3-binding domain is linked to structural unit 3 via a cleavable linker; Figure 3D shows that, based on the basic BiTE structure, the CD3-binding domain is linked to structural unit 3 via a pH-sensitive linker; Figure 3E shows that, based on the basic BiTE structure, the CD3-binding domain is linked to structural unit 3 via a cleavable linker and a pH-sensitive linker arranged alternately; and Figure 3F shows that, based on the structure as shown in Figure 3A, the CD3-binding domain is linked to structural unit 3 via a cleavable linker and a pH-sensitive linker in the absence of an Fc fragment.

Figure 4 shows that the BiTE binds the same tumor antigen, and the tumor-targeting peptide and the anti-TAA are arranged in tandem for masking the anti-CD3 activity. Figure 4A shows that, based on Figure 2C, the anti-TAA is linked to the tumor cell targeting peptide; Figure 4B shows that, based on Figure 2D, the anti-TAA is linked to the tumor cell targeting peptide; Figure 4C shows that, based on Figure 2E, the anti-TAA is linked to the tumor cell targeting peptide; Figure 4D shows that, based on Figure 2F, the anti-TAA is linked to the tumor cell targeting peptide; and Figure 4E shows that, based on Figure 2G, the anti-TAA is linked to the tumor cell targeting peptide.

The sequences used in the examples are as follows:
The anti-CD3 antibody sequence was selected from the UCHT1 clone (SEQ ID NO: 15) or the OKT3 clone (SEQ ID NO: 16), both of which can activate the CD3 signaling.

The anti-PDL1 antibody used was the commercially available Atezolizumab (SEQ ID NO: 17).

The anti-CLDN18.2 sequence is the one as set forth in SEQ ID NO: 18.

The human IgG1 sequences with abolished ADCC function and comprising "Knob" and "Hole" mutations are those as set forth in SEQ ID NO: 19 and SEQ ID NO: 20.

After linking the various antibodies via different linkers, the constructs were cloned into the expression vector-pEE6.4 plasmid (purchased from lifescience-market), using a signal peptide (SEQ ID NO: 21; SEQ ID NO: 47 may also be used). Host cells comprising the vectors were generated by transient transfection of 293F cells. The host cells were cultured, and the cell supernatant was collected. The proteins were then purified by Protein A/G affinity chromatography, aliquoted, and stored at -80°C.

For the expression of heterodimeric proteins, two expression vector plasmids at an equimolar ratio were co-transfected. The monomers expressed in the cells were able to spontaneously form heterodimers.

Specifically, the expression plasmids for various fusion proteins were constructed by gene synthesis in the pEE6.4 vector. The expression supernatant for each protein was then obtained via transient transfection of 293F cells. Finally, the various proteins were purified by Protein A affinity chromatography.

The following expression vectors were constructed:
(1) PEE6.4-HindIII-IgK(signal)-anti-Claudin 18.2-Fck-EcoRI
(2) PEE6.4-HindIII-IgK(signal)-anti-CD3 (UCHT1)-Fch-EcoRI
(3) PEE6.4-HindIII-IgK(signal)-anti-Claudin 18.2-linker 1-anti-CD3-Fch-EcoRI
(4) PEE6.4-HindIII-IgK(signal)-anti-Claudin 18.2-linker 2-anti-CD3-Fch-EcoRI
(5) PEE6.4-HindIII-IgK(signal)-anti-Claudin 18.2-linker 2×2-anti-CD3-Fch-EcoRI
(6) PEE6.4-HindIII-IgK(signal)-anti-Claudin 18.2-linker 3+2-anti-CD3-Fch-EcoRI
(7) PEE6.4-HindIII-IgK(signal)-anti-Claudin 18.2-linker 3×2-anti-CD3-Fch-EcoRI
(8) PEE6.4-HindIII-IgK(signal)-anti-Claudin 18.2-linker 4×2-anti-CD3-Fch-EcoRI
(9) PEE6.4-HindIII-IgK(signal)-anti-PDL1-Fck-EcoRI
(10) PEE6.4-HindIII-IgK(signal)-anti-CD3 (OKT3)-Fch-EcoRI
(11) PEE6.4-HindIII-IgK(signal)-NGR-HA-linker 2×2-anti-CD3 (OKT3)-Fch-EcoRI
(12) PEE6.4-HindIII-IgK(signal)-anti-Claudin 18.2-linker 2×2-anti-CD3 (UCHT1)-Fch-mutIL2-EcoRI
wherein HindIII and EcoRI are restriction enzyme sites; Fck represents the inactivated Fc region (Knob, SEQ ID NO: 19), Fch represents the inactivated Fc region (Hole, SEQ ID NO: 20), and mutIL2 represents the mutated IL2 (SEQ ID NO: 110).

The sequences linking the individual fragments are as follows:
(1) Linker 1: G4S: GGGGS (SEQ ID NO: 32);
(2) Linker 2: MMP: any one of SEQ ID NOs: 6-8; Linker 2×2 represents any pairwise combination of SEQ ID NOs: 6-8;
(3) Linker 3: HA: NQHTIDLADS (SEQ ID NO: 10); and
(4) Linker 4: H(MMP14)A: NQHIANPVDS (SEQ ID NO: 13).

The obtained fusion proteins are as follows:
anti-CLDN18.2 × anti-CD3 (UCHT1) as shown in Figure 1A, i.e., CLDN×CD3 [anti-CLDN single-chain (VL-VH) and CD3 single-chain (VL-VH)], with CLDN-Fc (Knob) as set forth in SEQ ID NO: 22, and CD3-Fc (Hole) as set forth in SEQ ID NO: 23;
anti-CLDN18.2 × anti-CLDN18.2-MMP(double)-anti-CD3 (UCHT1) as shown in Figure 1D, i.e., CLDN×CLDN-MMP(double)-CD3 (UCHT1) [anti-CLDN single-chain (VL-VH) and CD3 single-chain (VL-VH)], with CLDN-Fc (Knob) as set forth in SEQ ID NO: 22, and CLDN-MMP(double)-CD3-Fc (Hole) as set forth in SEQ ID NO: 25;
anti-PDL1 × anti-CD3 (OKT3) as shown in Figure 2A, i.e., PDL1×CD3 (OKT3) [anti-PDL1 single-chain (VL-VH) and CD3 single-chain (VL-VH)], with PDL1-Fc (Knob) as set forth in SEQ ID NO: 26, and CD3-Fc (Hole) as set forth in SEQ ID NO: 27;
anti-PDL1 × anti-CLDN18.2-MMP(double)-anti-CD3 (OKT3) as shown in Figure 2D, i.e., PDL1×CLDN-MMP(double)-CD3 (OKT3) [anti-PDL1 single-chain (VL-VH) and CD3 single-chain (VL-VH)], with PDL1-Fc (Knob) as set forth in SEQ ID NO: 26, and CLDN-MMP(double)-CD3-Fc (Hole) as set forth in SEQ ID NO: 29; and
PDL1×NGR-HA-MMP(double)-CD3 (OKT3) as shown in Figure 3B, i.e., PDL1×NGR-HA-MMP(double)-CD3 (OKT3) [anti-PDL1 single-chain (VL-VH) and CD3 single-chain (VL-VH)], with PDL1-Fc (Knob) as set forth in SEQ ID NO: 26, and NGR-HA-MMP(double)-CD3-Fc (Hole) as set forth in SEQ ID NO: 31.

In the examples, MMP(double) represents the linker combination of SEQ ID NO: 8 and SEQ ID NO: 6, and the sequence of which is set forth in SEQ ID NO: 9.

### 1.2 Transient Transfection for Rapid Expression of Target Proteins

Freestyle 293F cells were revived according to standard protocols. The plasmid/PEI mixture was added to the cell suspension, and following standard transfection procedures, the supernatant was collected. The target proteins were then purified using Protein A affinity chromatography.

### 1.3 SEC-HPLC Identification

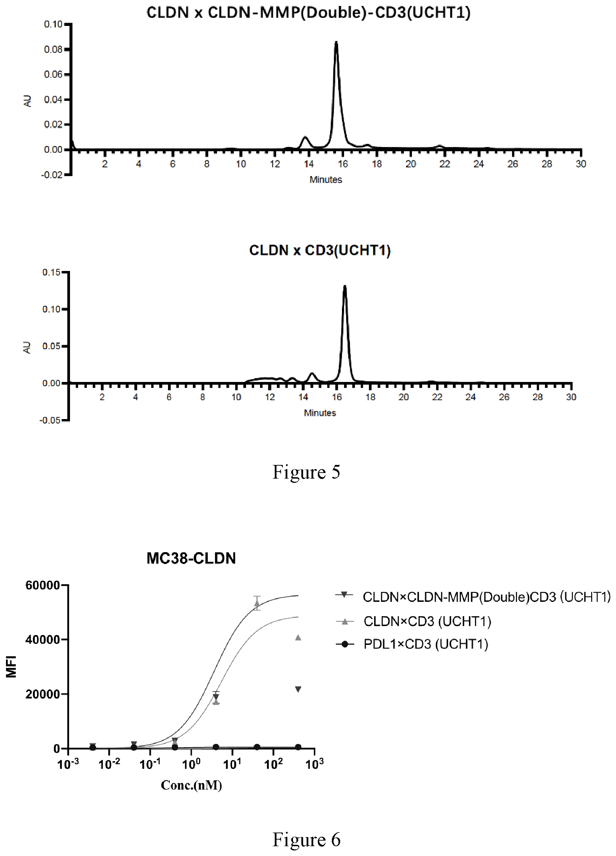

Taking the anti-CLDN18.2 × anti-CLDN18.2-MMP(double)-anti-CD3 (UCHT1) as shown in Figure 1D and the anti-CLDN18.2 × anti-CD3 (UCHT1) as shown in Figure 1A as examples, the mobile phase was firstly prepared: 500 mL of 1 M Tris-HCl (pH 6.8). The analytical column (Thermo MAbPac SEC-1, 5 µm, 300 Å, 4 × 300 mm) was installed on the instrument (Waters ACQUITY Premier UPLC). Detection was performed with a PDA detector at UV 280 nm, with a flow rate of 0.15 mL/min and a separation time of 30 minutes. Data were acquired, processed, and exported using Empower software. The SEC-HPLC identification chromatograms are shown in Figure 5. The HPLC peak profiles showed that the proteins of anti-CLDN18.2 × anti-CLDN18.2-MMP(double)-anti-CD3 (UCHT1) and anti-CLDN18.2 × anti-CD3 (UCHT1) were essentially free of impurity peaks (Figure 5).

### Example 2. In Vitro Biological Activity Studies of Fusion Proteins

### 2.1 Detection of Antigen Binding Ability of Fusion Proteins

The binding properties of the purified bispecific antibodies were analyzed by flow cytometry. The results of the analysis are shown in Figure 6. The bispecific antibodies used were PDL1×CD3 (UCHT1) having the structure as shown in Figure 1A, PDL1×CLDN-MMP(double)-CD3 (UCHT1) having the structure as shown in Figure 2A, CLDN×CD3 (UCHT1) having the structure as shown in Figure 1A, CLDN×CLDN-MMP(double)-CD3 (UCHT1) having the structure as shown in Figure 1D, and PDL1×CLDN-MMP(double)-CD3 (UCHT1) having the structure as shown in Figure 2D.
(1) Target cell preparation: The MC38 cell line overexpressing Claudin 18.2 (ACCESSION: P56856) was selected as the target cells for the binding assay. The cells were cultured in DMEM containing 10% FBS (fetal bovine serum) and 1% P/S (penicillin and streptomycin) for more than three passages. Prior to use, the cells were resuspended and adjusted to a concentration of 5×10⁶/mL.
(2) Protein sample preparation: Each protein sample was prepared at a highest concentration of 400 nmol/L (equimolar), and then serially diluted 10-fold. Tubes were labeled and arranged in the following order: 1:10, 1:100, 1:1000, 1:10000, and 1:100000. 450 µL of FACS buffer was added to each tube. 50 µL of the highest concentration protein sample was transferred to the 1:10 dilution tube and mixed thoroughly by pipetting. Serial dilution was continued by transferring 50 µL from the 1:10 tube to the 1:100 tube, and sequentially to the 1:100000 tube.
(3) Incubation and binding of cells with samples: 100 µL of the prepared cell suspension was added to each well of a round-bottom 96-well plate, i.e., 5×10⁵ cells per well. After centrifugation at 1000 rpm for 5 minutes, the supernatant was discarded. The cells were then resuspended in the prepared protein samples, in duplicate, and incubated at 4°C in the dark for 30 minutes.
(4) Labeling samples by binding with a commercial fluorescent antibody (Biolegend): 150 µL of FACS buffer was added to each well of the 96-well plate, mixed, and centrifuged at 1000 rpm for 5 minutes, and the supernatant was discarded. This process was repeated twice. Then, the commercial anti-human Fc-APC/PE antibody (Biolegend) was added at 0.5 µL per well (0.5 µL of antibody was mixed with 50 µL of FACS buffer), and the plate was incubated at 4°C in the dark for 30 minutes.
(5) Flow cytometry analysis: 150 µL of FACS buffer was added to each well, mixed, and centrifuged at 1000 rpm for 5 minutes, and the supernatant was discarded. This process was repeated twice. The cells were then resuspended in 150-200 µL of FACS buffer and analyzed by flow cytometry.

The results in Figure 6 showed that the bispecific antibody CLDN×CLDN-MMP(double)-CD3 (UCHT1) having the structure as shown in Figure 1D exhibited the highest binding affinity to the MC38-CLDN cell line, followed by PDL1×CLDN-MMP(double)-CD3 (UCHT1) having the structure as shown in Figure 2D, whereas PDL1×CD3 (UCHT1) having the structure as shown in Figure 1A exhibited no binding to this cell line.

### 2.2 In Vitro MMP14 Enzymatic Cleavage Assay

The cleavage efficiency of protein samples by MMP14 enzyme was evaluated in vitro.

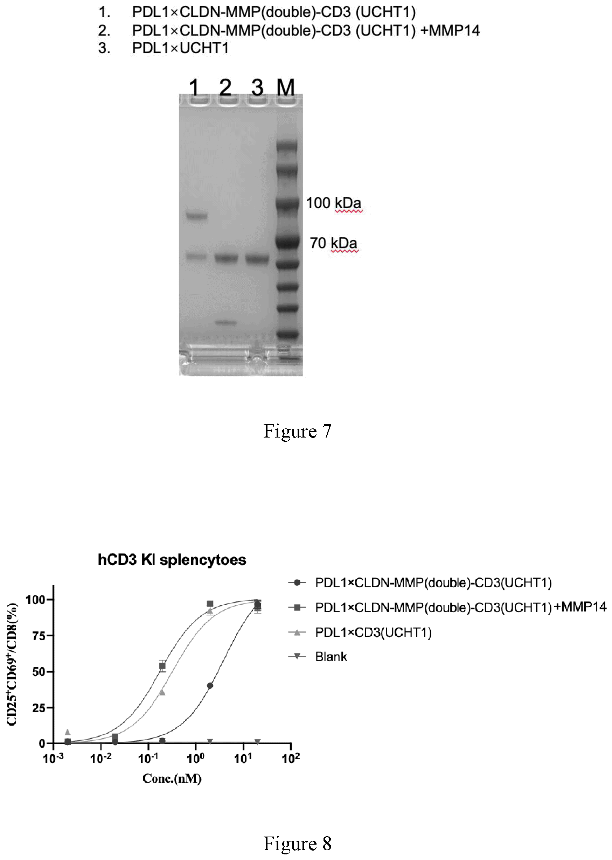

Firstly, the recombinant human MMP14 enzyme (R&D Sciences) at a concentration of 80 µg/mL was incubated with 1.72 µg/mL of rhFurin (Roche) in activation buffer (composed of 50 mM Tris, 1 mM CaCl₂, 0.5% (w/v) Brij 35, pH 9.0, which was filtered through a 0.22 µm filter) for 2 hours. The protein sample and the activated MMP14 enzyme at a molar ratio of 5:1 were added into the assay buffer (composed of 50 mM Tris, 3 mM CaCl₂, 1 µM ZnCl₂, pH 8.5, which was filtered through a 0.22 µm filter) and incubated for 24 hours, with the protein concentration in the enzyme cleavage system being 100 µg/mL. The proteins were then analyzed by SDS-PAGE, and the relative gel band intensities were analyzed to quantify the extent of cleavage in each protein/MMP14 mixture. The SDS-PAGE identification profile is shown in Figure 7. The results in Figure 7 showed that PDL1×CLDN-MMP(double)-CD3 (UCHT1) having the structure as shown in Figure 2D was completely cleaved by MMP14 enzyme.

### 2.3 Activity Recovery Assay of Fusion Protein After In Vitro MMP14 Enzymatic Cleavage

Following the method described in Section 2.2 for in vitro MMP14 enzymatic cleavage detection, in vitro cleavage experiment on PDL1×CLDN-MMP(double)-CD3 (UCHT1) having the structure as shown in Figure 2D was performed. A control was set up under the same incubation conditions but without MMP14 enzyme. The SDS-PAGE profile is shown in Figure 7, and the results of binding ability recovery after enzymatic cleavage are shown in Figure 8.

Screening of CD3⁺ cells: CD3⁺ cells were sorted using the MojoSort^{™} Human CD3 T Cell Isolation Kit (Biolegend) according to the instructions provided. The kit provides all necessary materials. The cell concentration was adjusted to 1 × 10⁸/mL using the MojoSort^{™} buffer. 100 µL of the biotin-antibody cocktail was added, mixed thoroughly, and incubated on ice for 15 minutes. The beads were resuspended by vortexing at maximum speed with 5 taps. 10 µL of streptavidin nanobeads was added, mixed thoroughly, and incubated on ice for 15 minutes. 2.5 mL of the MojoSort^{™} buffer was added, and the tube was placed in a magnet for 5 minutes.

The liquid was decanted and collected, and the cell concentration was adjusted to 3 × 10⁶/mL.
(1) Protein sample preparation: Each protein sample was prepared at a highest concentration of 200 nmol/L (equimolar). For protein sample that had been cleaved, the concentration was still calculated based on the original molar mass. Serial 10-fold dilutions were prepared and mixed thoroughly by pipetting.
(2) Incubation and binding of cells with protein samples: 100 µL of the prepared cell suspension was added to each well of a round-bottom 96-well plate, i.e., 3×10⁵ cells per well. After centrifugation at 1600 rpm for 5 minutes, the supernatant was discarded. The cells were then resuspended in the prepared protein samples, in duplicate, and incubated at 4°C f in the dark or 30 minutes.
(3) Labeling protein samples by binding with a commercial fluorescent antibody (Biolegend): 150 µL of FACS buffer was added to each well of the 96-well plate, mixed, and centrifuged at 1600 rpm for 5 minutes, and the supernatant was discarded. This process was repeated twice. Then, the commercial anti-human Fc-APC/PE antibody (Biolegend) was added at 0.5 µL per well (0.5 µL of antibody was mixed with 50 µL of FACS buffer), and the plate was incubated at 4°C in the dark for 30 minutes.
(4) Flow cytometry analysis: 150 µL of FACS buffer was added to each well, mixed, and centrifuged at 1000 rpm for 5 minutes, and the supernatant was discarded. This process was repeated twice. The cells were then resuspended in 150-200 µL of FACS buffer and analyzed by flow cytometry.
(5) To assess T cell activation in vitro, the purified human PBMCs were resuspended in culture medium (RPMI 1640 supplemented with 10% FBS, 1% P/S, and 1% glutamine) (Thermo Fisher Scientific) to a concentration of 5×10⁶ cells/mL, and 100 µL of the cell suspension was added to each well of a round-bottom 96-well plate.
(6) As described in Section 2.2-2, the cleaved or uncleaved protein samples were prepared at various concentrations in RPMI 1640 medium. The prepared protein samples were then added sequentially to each well in duplicate and incubated in a tissue culture incubator for 48 hours.
(7) After incubation, the cells were centrifuged at 1600 rpm for 5 minutes. The supernatant was collected for a cytokine release measurement, or discarded as appropriate. The cells were stained with fluorescently labeled antibodies against T cell markers (CD3, CD4, CD8, etc.) and activation markers (CD69, CD25, etc.), and analyzed by flow cytometry. Data were analyzed using the FlowJo software (FlowJo v10) to generate the plot as shown in Figure 8. The results in Figure 8 showed that after in vitro enzymatic cleavage, the biological activity of the CD3 antibody in the fusion protein PDL1×CLDN-MMP(double)-CD3 (UCHT1) having the structure as shown in Figure 2D was efficiently restored, reaching a level comparable to that of PDL1×CD3 (UCHT1) having the structure as shown in Figure 1A (Figure 8). These results indicated that the activity of CD3 in the antibody was efficiently blocked by the anti-CLDN18.2 moiety, and that its activity against T cells could be effectively restored upon cleavage by MMP14.

### Example 3. Novel BiTEs Demonstrate Superior Anti-Tumor Efficacy Compared to Conventional BiTEs

To determine whether the designed and produced TAA×TAA/targeting peptide-CD3 BiTEs are effective against tumors following systemic administration, and to compare their therapeutic efficacy with that of the conventional bispecific antibodies, the hCD3 humanized mice (hCD3EDG) were inoculated with MC38-CLDN cells (MC38 cells overexpressing Claudin 18.2) and treated with a low dose of the BiTEs via systemic administration.

The hCD3EDG mice (n=5 per group) were subcutaneously inoculated with 1×10⁶ MC38-CLDN cells on day 0. On day 11, when the tumors reached a volume of 80 to 120 mm³, the mice were randomly assigned to different groups. On days 11 and 14, the mice were treated via intraperitoneal injection of PBS, PDL1×CD3 (UCHT1) having the structure as shown in Figure 2A (non-targeting), PDL1×CLDN-MMP(double)-CD3 (UCHT1) having the structure as shown in Figure 2D (targetable to the tumor microenvironment, and non-targeting after MMP enzymatic cleavage), CLDN×CD3 (UCHT1) having the structure as shown in Figure 1A, or CLDN×CLDN-MMP(double)-CD3 (UCHT1) having the structure shown in Figure 1D, at an equimolar dose of 200 pmol per mouse. Tumor volumes of the tumor-bearing mice were recorded.

The experimental results are shown in Figure 9. The CLDN×CD3 group was able to suppress the tumor growth, but only 40% of the mice showed tumor volume reduction and a rapid rebound. The CLDN×CLDN-MMP(double)-CD3 group exhibited significant anti-tumor activity, with 80% of the mice showing tumor volume reduction and 10% achieving complete tumor regression, although a rebound was also observed at later stages. Under the low-dose treatment conditions, the anti-tumor activity of the CLDN×CLDN-MMP(double)-CD3 group was markedly superior to that of the CLDN×CD3 group. No significant body weight loss was observed in any of the treatment groups. These results indicated that CLDN×CLDN-MMP(double)-CD3 could effectively eliminate tumors and exhibited superior tumor clearance compared to the conventional bispecific antibody.

### Example 4. Novel BiTEs Exhibit Lower Peripheral Blood Toxicity Compared to Conventional BiTEs

Since the human CD3 KI mice were not particularly sensitiveto the peripheral blood toxicity, the hPBMC-humanized (hu-PBMC) NSG mice were used as an in vivo model. These mice were generated by injecting the purified human PBMCs from healthy donors. The use of adult peripheral blood mononuclear cells provides the fastest engraftment rate and enables the short-term studies with robust effector and memory T cell functions.

A549-CLDN cells, generated by lentiviral transduction to overexpress human CLDN18.2 protein (P56856), were used as the tumor model in this study. 1 × 10⁶ A549-CLDN cells were subcutaneously inoculated into the right dorsal flank of mice, which was designated as day 0. On day 22 after tumor inoculation, PBMCs were transplanted via tail vein injection. Two days later, the mice were randomly assigned to different groups and intraperitoneally injected with 100 pmol of PDL1×CD3 (PDL1×CD3 (OKT3) [anti-PDL1 single-chain (VL-VH) and CD3 single-chain (VL-VH)]) or PDL1×CLDN-CD3 (PDL1×CLDN-MMP(double)-CD3 (OKT3) [anti-PDL1 single-chain (VL-VH) and CD3 single-chain (VL-VH)]). Twenty-four hours later, blood was collected from the tail vein of the mice and allowed to stand for serum separation. The samples were then centrifuged at 10,000 rpm for 10 minutes, and the clear serum was transferred to new Eppendorf tubes. The levels of inflammatory cytokines in the serum were measured using the BD^{™} Cytometric Bead Array (CBA) Mouse Inflammation Kit (BD Biosciences), and the data were analyzed by flow cytometry using the BD CBA analysis software. The results are shown in Figure 10. Compared with the PBS control group, administration of PDL1×CD3 (OKT3) having the structure as shown in Figure 2A resulted in significantly elevated expression levels of cytokines in peripheral blood serum, whereas administration of PDL1×CLDN-MMP(double)-CD3 (OKT3) having the structure as shown in Figure 2D led to markedly decreased expression levels of cytokines in peripheral blood serum.

### Example 5. The Activity of Novel BiTEs Can Be Restored After Enzymatic Cleavage.

OKT3 (SEQ ID NO: 16) and UCHT1 (SEQ ID NO: 15) are the widely used monoclonal antibodies targeting CD3. In the structures of bispecific antibodies, OKT3 was used as the core component. Following the method described in Example 1, the genes encoding the proteins PDL1×CLDN-MMP(double)-CD3(PDL1×CLDN-MMP(double)-CD3 (OKT3) [anti-PDL1 single-chain (VL-VH) and CD3 single-chain (VL-VH)] and PDL1×NGR-HA-MMP(double)-CD3(PDL1×NGR-HA-MMP(double)-CD3 (OKT3) [anti-PDL1 single-chain (VL-VH) and CD3 single-chain (VL-VH)] were synthesized, and the proteins were produced in 293F cells.

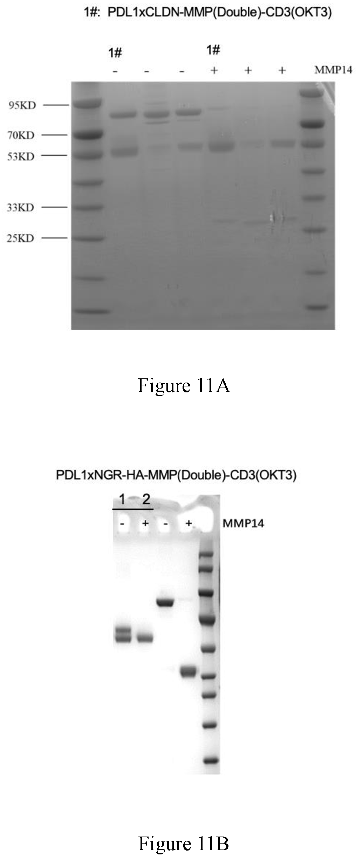

Firstly, the cleavage activity of the two proteins was assessed in vitro by repeating the MMP enzymatic cleavage method for bispecific antibody as described in Section 2.2 of Example 2. The results are shown in Figure 11. The SDS-PAGE results demonstrated that both forms of antibodies could be completely cleaved, as shown in Figure 11A (PDL1×CLDN-MMP(double)-CD3) and Figure 11B (PDL1×NGR-HA-MMP(double)-CD3).

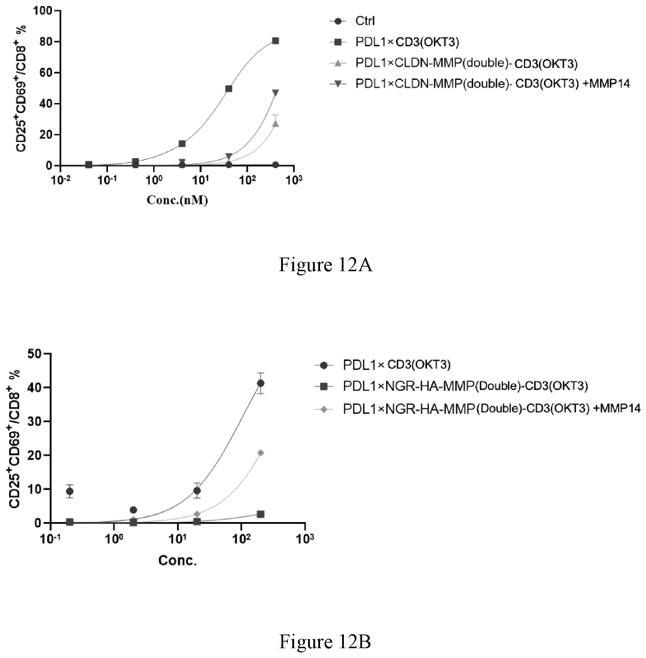

The in vitro PBMC activation assay after enzymatic cleavage described in Section 2.3 of Example 2 was repeated. It could be observed that both of the bispecific antibodies generated using an antibody against a tumor surface-specific antigen (Figure 12A, PDL1×CLDN-MMP(double)-CD3) and a targeting peptide (Figure 12B, PDL1×NGR-HA-MMP(double)-CD3) could effectively reduce the CD3 activity, and the CD3 activity was partially restored after enzymatic cleavage.

### Example 6. Development of Bispecific Antibody Fused with Cytokine

The construction, expression, and purification of a bispecific antibody fused with an IL-2 mutant cytokine (SEQ ID NO: 110) were described in detail below as a representative example. The same methods were applied to the expression and purification of other proteins of the present invention. Firstly, the fusion protein was constructed in the pEE6.4 expression vector. Host cells comprising the vector were generated by transient transfection of 293F cells. For the expression of heterodimeric proteins, two expression vector plasmids at an equimolar ratio were co-transfected, and the monomers expressed in the cells were able to spontaneously form heterodimers. The host cells were cultured, and the cell supernatant were collected. The proteins were then purified by Protein A/G affinity chromatography. The specific steps were as follows:

### Construction of Expression Vectors

(1) Anti-CLDN18.2 single-chain (VL-VH)
(2) Anti-CD3 single-chain (VL-VH, UCHT1) and mutated IL2 (F42A, R38L, i.e., mut IL2)
(3) Anti-PDL1 × anti-CD3 (OKT3)

Fusion protein was expressed by transient transfection of 293F cells:
Anti-CLDN18.2 × anti-CLDN18.2-MMP(double)-anti-CD3 (UCHT1)-MutIL2 as shown in Figure 14A, i.e., CLDN×CLDN-MMP(double)-CD3 (UCHT1) [anti-CLDN single-chain (VL-VH) and CD3 single-chain (VL-VH)-mutIL2], with CLDN-Fc (Knob) as set forth in SEQ ID NO: 22, CLDN-MMP(double)-CD3-Fc (Hole) as set forth in SEQ ID NO: 25, and mutIL2 as set forth in SEQ ID NO: 110.

### Example 7. In Vitro Biological Activity Studies of Fusion Proteins

### Detection of Antigen Binding Ability of Fusion Proteins

As described in Example 2.1, the bispecific antibodies used were PDL1×CD3 (OKT3) having the structure as shown in Figure 2A, CLDN×CLDN-MMP(double)-CD3 (UCHT1) having the structure as shown in Figure 1D, and PDL1×CLDN-MMP(double)-CD3 (UCHT1)-mutIL2 having the structure as shown in Figure 14A. The binding ability of the anti-CLDN18.2 antibody within the fusion proteins to its antigen was assessed by flow cytometry, and the results are shown in Figure 16.

The results showed that anti-CLDN18.2 × anti-CLDN18.2-MMP(double)-anti-CD3 (UCHT1)-mutIL2 exhibited slightly higher binding ability to CLDN18.2 antigen on the surface of MC38 cells compared to anti-CLDN18.2 × anti-CD3 (UCHT1), whereas anti-PDL1 × anti-CD3 (OKT3) exhibited no binding to this cell line (Figure 16).

### Example 8. Anti-Tumor Efficacy of Fusion Proteins

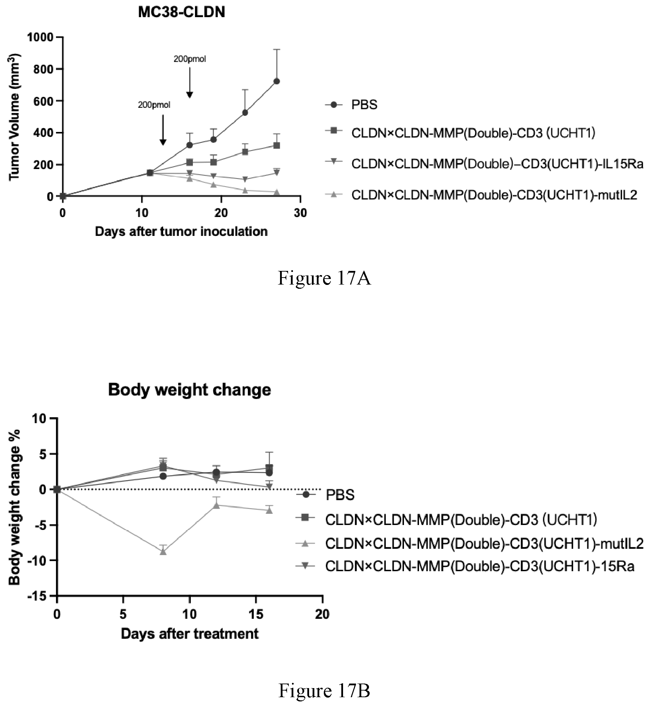

Taking the fusion proteins anti-CLDN18.2 × anti-CLDN18.2-MMP(double)-anti-CD3 (UCHT1)-mutIL2 and anti-CLDN18.2 × anti-CLDN18.2-MMP(double)-anti-CD3 (UCHT1)-IL15Ra having the structures as shown in Figure 14A as examples (wherein the sequence of CLDN18.2 is set forth in SEQ ID NO: 22, the sequence of mutIL2 is set forth in SEQ ID NO: 111, and the sequence of IL15Ra is set forth in SEQ ID NO: 134), whether these fusion proteins could be effectively against tumors and whether they exhibited superior efficacy compared to the bispecific antibody alone upon systemic administration were evaluated. The hCD3 humanized mice (hCD3EDG) were inoculated with MC38-CLDN cells (MC38 cells overexpressing Claudin 18.2) and treated with a low dose of the fusion proteins via systemic administration. The experimental results are shown in Figure 17.

The hCD3EDG mice (n=5 per group) were subcutaneously inoculated with 1×10⁶ MC38-CLDN cells on day 0. On day 13, when the tumors reached a volume of 220 mm³, the mice were randomly assigned to different groups. On days 13 and 16, the calculated amount of protein was dissolved in 200 µl of PBS and administered at an equimolar dose of 200 pmol per mouse. The mice were treated via intraperitoneal injection of PBS (as a control group), CLDN×CLDN-MMP(double)-CD3 (UCHT1), CLDN×CLDN-MMP(double)-CD3 (UCHT1)-mutIL2, and CLDN×CLDN-MMP(double)-CD3 (UCHT1)-IL15Ra. Tumor length (a), width (b) and height (c) were measured with a vernier caliper, and the mouse tumor volume was calculated as a × b × c / 2. Tumor sizes were measured twice weekly, and the tumor growth curves were recorded. The tumor growth curves demonstrated that the bispecific antibody fused with cytokines exhibited superior anti-tumor efficacy (Figure 17).

### Example 9. Anti-Tumor Efficacy of IL2 Fusion Proteins

Taking CLDN×CLDN-CD3, CLDN-wtIL2×CLDN-CD3, CLDN-mutIL2×CLDN-CD3, and CLDN-mutIL2×CD3 having the structures as shown in Figure 18A as examples, whether these fusion proteins could be effectively against tumors and whether they exhibited superior efficacy compared to the bispecific antibody alone upon systemic administration were evaluated. The anti-tumor efficacy of the novel fusion proteins was assessed using the method described in Example 8. The results demonstrated that the bispecific antibodies fused with either wild-type or mutated IL2 exhibited superior anti-tumor efficacy (Figure 19).

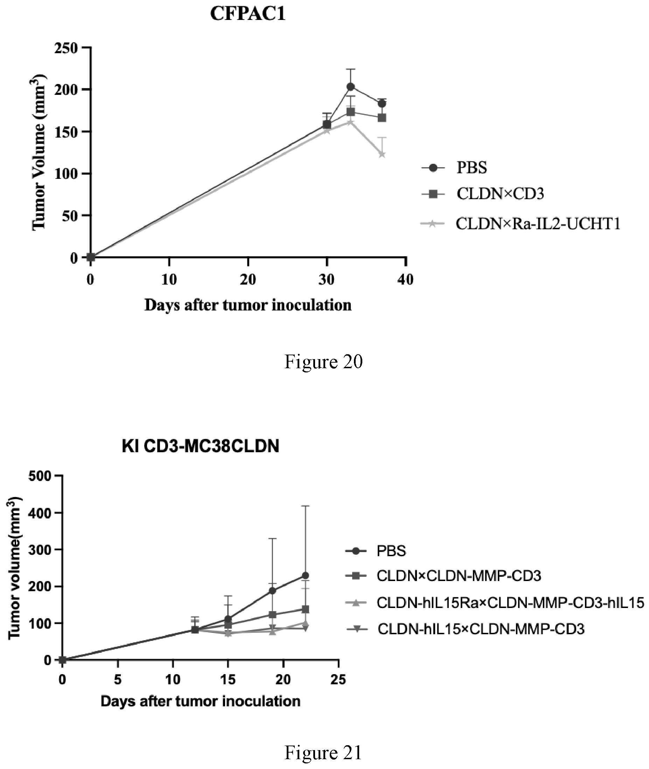

Following the experimental procedure described in Example 4, the NCG mice with systemic immunodeficiency were inoculated with CFPAC1 tumor cells (purchased from ATCC). When the tumors reached 100 mm³, the mice were intravenously transplanted with 3 × 10⁶ human PBMCs via tail vein injection. Five days later, the mice were administered with 200 pmol of the fusion protein drugs (CLDN×CD3 and CLDN×Ra-IL2-CD3 (see Figure 18G for structure)) via intraperitoneal injection. Tumor growth was monitored (Figure 20). The results demonstrated that the bispecific antibody fused with wild-type IL2 and IL2 receptor Ra exhibited superior anti-tumor efficacy (Figure 20).

The sequences used in Example 9 are as follows:

| | | | |
|---|---|---|---|
| CLDN(scFv) × CD3(Fab) | SEQ ID NO:22 | SEQ ID NO:160 | SEQ ID NO:161 |
| CLDN × CDLN-CD3 (Fig. 18A, without a cytokine) | SEQ ID NO:22 | from the N-terminus to the C-terminus: SEQ ID NO:163, SEQ ID NO:149, and SEQ ID NO:160 | from the N-terminus to the C-terminus: SEQ ID NO:162, SEQ ID NO:149, and SEQ ID NO:161 |
| CLDN-wtIL2 × CDLN-CD3 (Fig. 18A, wtL2) | from the N-terminus to the C-terminus: SEQ ID NO:22, SEQ ID NO:3, and SEQ ID NO:110 | from the N-terminus to the C-terminus: SEQ ID NO:163, SEQ ID NO:149, and SEQ ID NO:160 | from the N-terminus to the C-terminus: SEQ ID NO:162, SEQ ID NO:149, and SEQ ID NO:161 |
| CLDN-mutIL2 × CDLN-CD3 (Fig. 18A, mutIL2) | from the N-terminus to the C-terminus: SEQ ID NO:22, SEQ ID NO:3, and SEQ ID NO:176) | from the N-terminus to the C-terminus: SEQ ID NO:163, SEQ ID NO:149, and SEQ ID NO:160 | from the N-terminus to the C-terminus: SEQ ID NO:162, SEQ ID NO:149, and SEQ ID NO:161 |
| CLDN-mutIL2 × CD3 (Fig. 18H, wherein the CD3 antibody is in the form of Fab) | from the N-terminus to the C-terminus: SEQ ID NO:22, SEQ ID NO:3, and SEQ ID NO:176) | SEQ ID NO:160 | SEQ ID NO:161 |
| CLDN × Ra-IL2-UCHT1 (Fig. 18G) | SEQ ID NO:22 | from the N-terminus to the C-terminus: SEQ ID NO:167, SEQ ID NO:149, and SEQ ID NO:160 | from the N-terminus to the C-terminus: SEQ ID NO:110, SEQ ID NO:32, and SEQ ID NO:161 |

### Example 10. Anti-Tumor Efficacy of IL15 Fusion Proteins

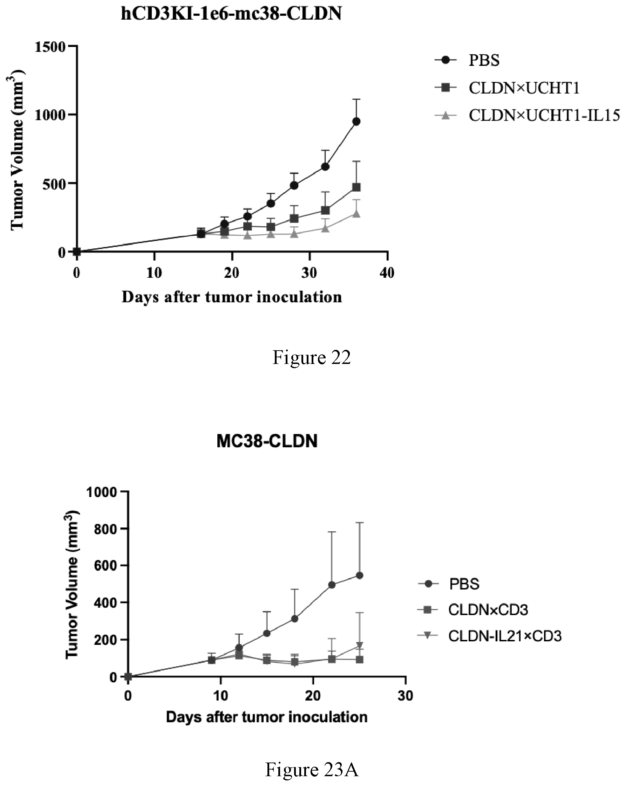

Taking CLDN-hIL15×CLDN-MMP-CD3 having the structure as shown in Figure 18A and CLDN-hIL15Ra×CLDN-MMP-CD3-hIL15 having the structure as shown in Figure 18D as examples, whether these fusion proteins could be effectively against tumors upon systemic administration and whether they exhibited superior efficacy compared to the bispecific antibody alone upon systemic administration were evaluated. The anti-tumor efficacy of the novel fusion proteins was assessed using the method described in Example 8. The results demonstrated that the bispecific antibody prodrugs fused with wild-type IL15, or with both IL15 and IL15Ra, exhibited superior anti-tumor efficacy (Figure 21). In addition, fusing IL15 directly to the bispecific antibody (CLDN-hIL15×CD3), as shown in Figure 18H, also resulted in improved anti-tumor efficacy (Figure 22).

The sequences used in Example 10 are as follows:

| | | | |
|---|---|---|---|
| CLDN × CDLN-MMP-CD3 (Fig. 1D) | SEQ ID NO:22 | from the N-terminus to the C-terminus: SEQ ID NO:163, SEQ ID NO:149, and SEQ ID NO:160 | from the N-terminus to the C-terminus: SEQ ID NO:162, SEQ ID NO:149, and SEQ ID NO:161 |
| CLDN-hIL15Ra × CLDN-MMP-CD3-hIL15 (Fig. 18D) | SEQ ID NO:185 | SEQ ID NO:186 | from the N-terminus to the C-terminus: SEQ ID NO:162, SEQ ID NO:149, and SEQ ID NO:161 |
| CLDN-hIL15 × CLDN-MMP-CD3 (Fig. 18A) | from the N-terminus to the C-terminus: SEQ ID NO:22, SEQ ID NO:3, and SEQ ID NO:137 | from the N-terminus to the C-terminus: SEQ ID NO:163, SEQ ID NO:149, and SEQ ID NO:160 | from the N-terminus to the C-terminus: SEQ ID NO:162, SEQ ID NO:149, and SEQ ID NO:161 |
| CLDN × UCHT1-IL15 (Fig. 18H) | SEQ ID NO:22 | from the N-terminus to the C-terminus: SEQ ID NO:23, SEQ ID NO:3, and SEQ ID NO:137 | |
| CLDN × UCHT1(Fig. 18H, without a cytokine) | SEQ ID NO:22 | SEQ ID NO:23 | |

### Example 11. Anti-Tumor Efficacy and Peripheral Blood Toxicity of IL21 Fusion Protein

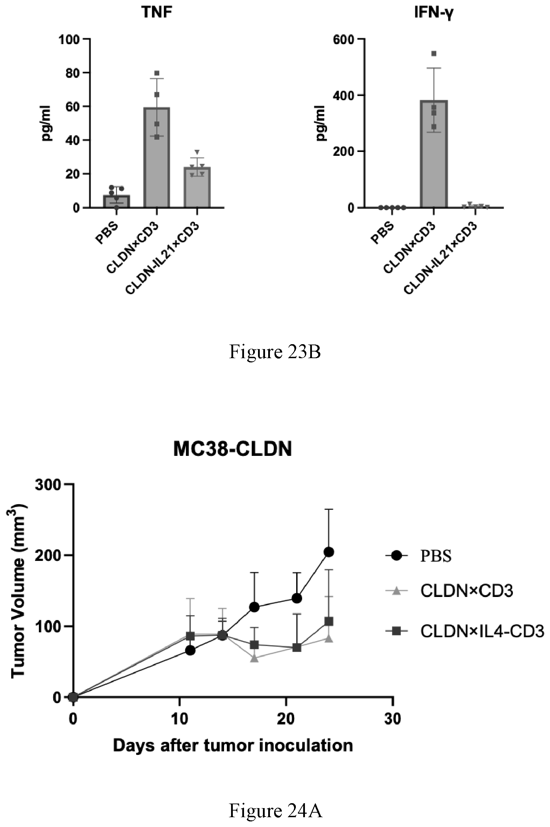

Using CLDN-IL21×CD3 having the structure as shown in Figure 18H, in which IL21 was directly fused to the bispecific antibody, the anti-tumor efficacy of the novel fusion protein was assessed using the method described in Example 8. It was found that although the anti-tumor efficacy was not improved after the fusion of IL21 (Figure 23A), the levels of cytokines IFN and TNF in the peripheral blood were significantly reduced (Figure 23B), indicating that the IL21 fusion protein could reduce the peripheral blood toxicity induced by the BiTE.

The sequences used in Example 11 are as follows:

| | | |
|---|---|---|
| CLDN × CD3 (Fig. 18H, without a cytokine) | SEQ ID NO:22 | SEQ ID NO:157 |
| CLDN-IL21 × CD3 (Fig. 18H) | SEQ ID NO:129 | SEQ ID NO:157 |

### Example 12. Anti-Tumor Efficacy and Peripheral Blood Toxicity of IL4 Fusion Protein

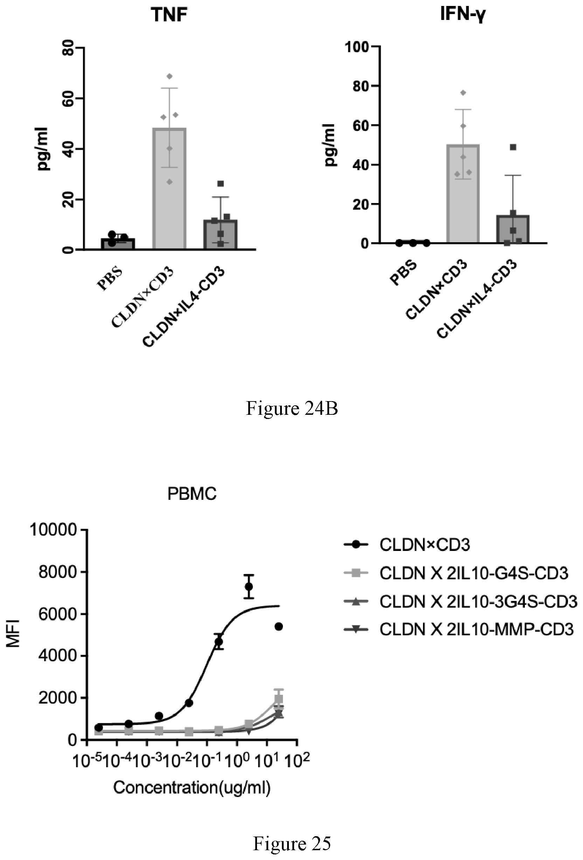

Using CLDN×IL4-CD3 having the structure as shown in Figure 13A, in which IL4 was fused to the N-terminus of CD3 via a cleavable linker based on the bispecific antibody, the anti-tumor efficacy of the novel fusion protein was assessed using the method described in Example 8. It was found that although the anti-tumor efficacy was not improved after the fusion of IL4 (Figure 24A), the levels of cytokines IFN and TNF in the peripheral blood were significantly reduced (Figure 24B), indicating that the IL4 fusion protein could reduce the peripheral blood toxicity induced by the BiTE.

The sequences used in Example 12 are as follows:

| | | |
|---|---|---|
| CLDN × CD3 (Fig. 18H, without a cytokine) | SEQ ID NO:22 | SEQ ID NO:157 |
| CLDN × IL4 - CD3 (Fig.13A) | SEQ ID NO:22 | from the N-terminus to the C-terminus: SEQ ID NO:181, SEQ ID NO:32, SEQ ID NO:149, and SEQ ID NO:157 |

### Example 13. Anti-Tumor Efficacy and Peripheral Blood Toxicity of IL10 Fusion Proteins

Using the structures as shown in Figures 13A and 13C, two tandem IL10 molecules were fused to the N-terminus of the anti-CD3 antibody sequence, linked in between via cleavable linkers (MMP) or non-cleavable linkers (G4S or (G4S)₃) with varying lengths. The different forms of fusion proteins were incubated with PBMCs in vitro at various concentrations for 30 minutes, followed by staining with anti-hFc-APC secondary antibody (as described in Example 2.1). The mean fluorescence intensity of each group was then analyzed by flow cytometry. The results as shown in Figure 25, demonstrated that the fusion of two IL10 molecules significantly affected the affinity for CD3.

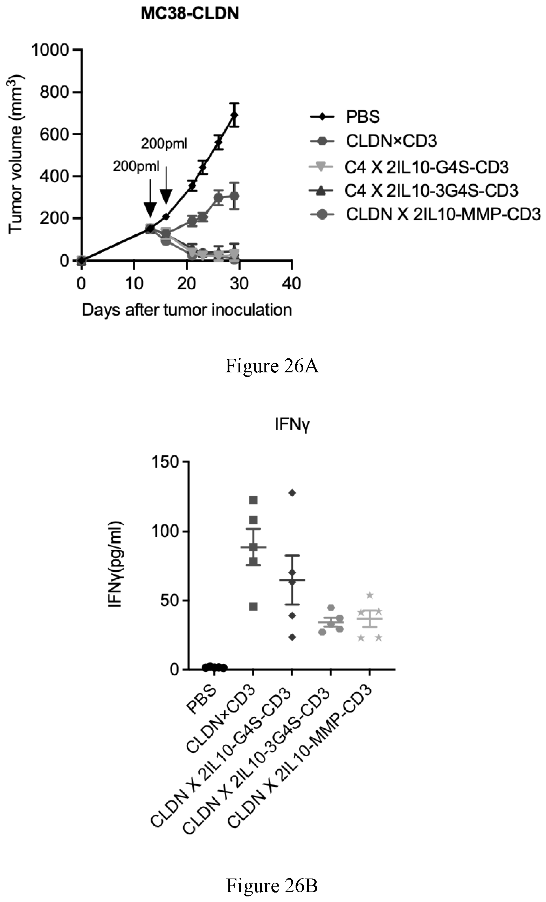

Subsequently, the anti-tumor efficacy of the IL10 fusion proteins was assessed using the method as described in Example 8. The results demonstrated that the fusion of IL10 to the bispecific antibody significantly enhanced anti-tumor efficacy (Figure 26A), while also reduced the secretion of cytokines in the peripheral blood (Figure 26B), thereby achieving both increased efficacy and reduced toxicity.

The sequences used in Example 13 are as follows:

| | | |
|---|---|---|
| CLDN × CD3 (Fig. 18H, without cytokines) | SEQ ID NO:22 | SEQ ID NO:23 |
| CLDN × 2IL10-G4S-CD3 (Fig. 13C, the linker is G4S) | SEQ ID NO:22 | SEQ ID NO:116 |
| CLDN × 2IL10-(G4S)₃-CD3 (Fig. 13C, the linker is (G4S)₃) | SEQ ID NO:22 | from the N-terminus to the C-terminus: SEQ ID NO:184, SEQ ID NO:3, and SEQ ID NO:23 |
| CLDN × 2IL10-MMP-CD3 (Fig.13A) | SEQ ID NO:22 | from the N-terminus to the C-terminus: SEQ ID NO:184, SEQ ID 32, SEQ ID NO:149, and SEQ ID NO:23 |

### Sequence Listing

**G4S₂**: GGGGSGGGGS (SEQ ID NO:1)
**G4S₄**: GGGGSGGGGSGGGGSGGGGS (SEQ ID NO:2)
**G4S₃**: GGGGSGGGGSGGGGS (SEQ ID NO:3)
**G4S₅**: GGGGSGGGGSGGGGSGGGGSGGGGS (SEQ ID NO:24)
**(GGS)₂**: GGSGGS (SEQ ID NO:28)
**(GGS)₃**: GGSGGSGGS (SEQ ID NO:30)
**HA-MMP**: NQHTIDLADSSGFIANPVTA (SEQ ID NO:4)
**MMP-HA**: SGFIANPVTANQHTIDLADS (SEQ ID NO:5)
**MMP high**: SGRSPAIFTA (SEQ ID NO:6)
**MMP medium**: SGFIANPVTA (SEQ ID NO:7)
**MMP low**: SGRSENIRTA (SEQ ID NO:8)
**MMP(Double)**: SGRSENIRTASGRSPAIFTA (SEQ ID NO:9)
**HA**: NQHTIDLADS (SEQ ID NO:10)
**HA₂**: NQHTIDLADSNQHTIDLADS (SEQ ID NO:11)
**HAₙ**: (NQHTIDLADS)ₙ (SEQ ID NO:12) n=3
**H(MMP)A**: NQHIANPVDS (SEQ ID NO:13)
**H(MMP)A₂**: NQHIANPVDSNQHIANPVDS (SEQ ID NO:14)
**anti-CD3(single-chain, clone: UCHT1, VL-VH)** 
DIQMTQSPSSLSASVGDRVTITCRASQDIRNYLNWYQQKPGKAPKLLIYYTSRLESGVPSRFSGSGSGT
DYTLTISSLQPEDFATYYCQQGNTLPWTFGQGTKVEIKGGGGSGGGGSGGGGSEVQLVESGGGLVQPG
GSLRLSCAASGYSFTGYTMNWVRQAPGKGLEWVALINPYKGVSTYNQKFKDRFTISVDKSKNTAYLQ
MNSLRAEDTAVYYCARSGYYGDSDWYFDVWGQGTLVTVSS (SEQ ID NO:15)
**anti-CD3(single-chain, clone: OKT3, VL-VH)** 
QIVLTQSPAIMSASPGEKVTMTCSASSSVSYMNWYQQKSGTSPKRWIYDTSKLASGVPAHFRGSGSGT
SYSLTISGMEAEDAATYYCQQWSSNPFTFGSGTKLEINGGGGSGGGGSGGGGSQVQLQQSGAELARP
GASVKMSCKASGYTFTRYTMHWVKQRPGQGLEWIGYINPSRGYTNYNQKFKDKATLTTDKSSSTAY
MQLSSLTSEDSAVYYCARYYDDHYCLDYWGQGTTLTVSS (SEQ ID NO:16)
**anti-PDL1(single-chain, Atezolizumab, VL-VH)**

DIQMTQSPSSLSASVGDRVTITCRASQDVSTAVAWYQQKPGKAPKLLIYSASFLYSGVPSRFSGSGSGT
DFTLTISSLQPEDFATYYCQQYLYHPATFGQGTKVEIKRGGGGSGGGGSGGGGSEVQLVESGGGLVQP
GGSLRLSCAASGFTFSDSWIHWVRQAPGKGLEWVAWISPYGGSTYYADSVKGRFTISADTSKNTAYLQ
MNSLRAEDTAVYYCARRHWPGGFDYWGQGTLVTVSA (SEQ ID NO:17)

The CDR1 sequence of PDL1 VL is QDVSTA (SEQ ID NO:73).

The CDR2 sequence of PDL1 VL is SAS.

The CDR3 sequence of PDL1 VL is QQYLYHPATF (SEQ ID NO: 74).

The CDR1 sequence of PDL1 VH is GFTFSDSW (SEQ ID NO: 75).

The CDR2 sequence of PDL1 VH is ISPYGGST (SEQ ID NO: 76).

The CDR3 sequence of PDL1 VH is ARRHWPGGFDYW (SEQ ID NO: 77).
**anti-CLDN18.2(single-chain, VL-VH)**

DIVMTQSPSSLTVTAGEKVTMSCKSSQSLLNSGNQKNYLTWYQQKPGQPPKLLIYWASTRESGVPDRF
TGSGSGTDFTLTISSVQAEDLAVYYCQNDYFYPFTFGSGTKLEIKGGGGSGGGGSGGGGSQVQLQQPG
AELVRPGASVKLSCKASGYTFTSYWINWVKQRPGQGLEWIGNIYPSDSYTNYNQKFKDKATLTVDKS
SSTAYMQLSSPTSEDSAVYYCARVYYGNSFAYWGQGTTLTVSS (SEQ ID NO:18)

The CDR1 sequence of CLDN VL is QSLLNSGNQKNY (SEQ ID NO:78).

The CDR2 sequence of CLDN VL is WAS.

The CDR3 sequence of CLDN VL is QNDYFYPFTF (SEQ ID NO:79).

The CDR1 sequence of CLDN VH is GYTFTSYW (SEQ ID NO:80).

The CDR2 sequence of CLDN VH is IYPSDSYT (SEQ ID NO:81).

The CDR3 sequence of CLDN VH is ARVYYGNSFAYW (SEQ ID NO:82).
**Inactivation Fc region (Knob)** 
DKTHTCPPCPAPEAAGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALGAPIEKTISKAKGQPREPQVCTLPPS
RDELTKNQVSLSCAVKGFYPSDIAVEWESNGQPENNYKTTPPVLDSDGSFKLVSKLTVDKSRWQQGN
VFSCSVMHEALHNHYTQKSLSLSPGK (SEQ ID NO:19)
**Inactivation Fc region (Hole)** 
DKTHTCPPCPAPEAAGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALGAPIEKTISKAKGQPREPQVYTLPPC
RDELTKNQVSLWCLVKGFYPSDIAVEWESNGQPENNYKTTPPVLDSDGSFFLYSALTVDKSRWQQGN
VFSCSVMHEALHNHYTQKSLSLSPGK (SEQ ID NO:20)
**IgGκ**: METDTLLLWVLLLWVPGSTG (SEQ ID NO:21)
**CLDN×CD3(UCHT1) [anti-CLDN single-chain (VL-VH) and CD3 single-chain (VL-VH)]**

| | |
|---|---|
| CLDN×CD3 | CLDN-Fc(Knob) |
| | DIVMTQSPSSLTVTAGEKVTMSCKSSQSLLNSGNQKNYLTWYQQK PGQPPKLLIYWASTRESGVPDRFTGSGSGTDFTLTISSVQAEDLAVY YCQNDYFYPFTFGSGTKLEIKGGGGSGGGGSGGGGSQVQLQQPGA ELVRPGASVKLSCKASGYTFTSYWINWVKQRPGQGLEWIGNIYPS DSYTNYNQKFKDKATLTVDKSSSTAYMQLSSPTSEDSAVYYCARVY YGNSFAYWGQGTTLTVSSDKTHTCPPCPAPEAAGGPSVFLFPPKPK DTLMISRTPEVTCVVVDVSHEDPEVKFNWYVDGVEVHNAKTKPRE EQYNSTYRVVSVLTVLHQDWLNGKEYKCKVSNKALGAPIEKTISK AKGQPREPQVCTLPPSRDELTKNQVSLSCAVKGFYPSDIAVEWESN GQPENNYKTTPPVLDSDGSFKLVSKLTVDKSRWQQGNVFSCSVMH EALHNHYTQKSLSLSPGK (SEQ ID NO:22) |
| | CD3-Fc(Hole) |
| | DIQMTQSPSSLSASVGDRVTITCRASQDIRNYLNWYQQKPGKAPKL LIYYTSRLESGVPSRFSGSGSGTDYTLTISSLQPEDFATYYCQQGNTL PWTFGQGTKVEIKGGGGSGGGGSGGGGSEVQLVESGGGLVQPGGS LRLSCAASGYSFTGYTMNWVRQAPGKGLEWVALINPYKGVSTYN QKFKDRFTISVDKSKNTAYLQMNSLRAEDTAVYYCARSGYYGDSD WYFDVWGQGTLVTVSSDKTHTCPPCPAPEAAGGPSVFLFPPKPKDT LMISRTPEVTCVVVDVSHEDPEVKFNWYVDGVEVHNAKTKPREEQ YNSTYRVVSVLTVLHQDWLNGKEYKCKVSNKALGAPIEKTISKAK GQPREPQVYTLPPCRDELTKNQVSLWCLVKGFYPSDIAVEWESNGQ PENNYKTTPPVLDSDGSFFLYSALTVDKSRWQQGNVFSCSVMHEA LHNHYTQKSLSLSPGK (SEQ ID NO:23) |

**CLDN×CLDN-MMP(double)-CD3(UCHT1) [anti-CLDN single-chain (VL-VH) and CD3 single-chain (VL-VH)]**

| | |
|---|---|
| CLDN× CLDN-MMP(double)-CD3 | CLDN-Fc(Knob) |
| | DIVMTQSPSSLTVTAGEKVTMSCKSSQSLLNSGNQKNYLTWYQQK PGQPPKLLIYWASTRESGVPDRFTGSGSGTDFTLTISSVQAEDLAVY YCQNDYFYPFTFGSGTKLEIKGGGGSGGGGSGGGGSQVQLQQPGA ELVRPGASVKLSCKASGYTFTSYWINWVKQRPGQGLEWIGNIYPS DSYTNYNQKFKDKATLTVDKSSSTAYMQLSSPTSEDSAVYYCARVY YGNSFAYWGQGTTLTVSSDKTHTCPPCPAPEAAGGPSVFLFPPKPK DTLMISRTPEVTCVVVDVSHEDPEVKFNWYVDGVEVHNAKTKPRE EQYNSTYRVVSVLTVLHQDWLNGKEYKCKVSNKALGAPIEKTISK AKGQPREPQVCTLPPSRDELTKNQVSLSCAVKGFYPSDIAVEWESN GQPENNYKTTPPVLDSDGSFKLVSKLTVDKSRWQQGNVFSCSVMH EALHNHYTQKSLSLSPGK (SEQ ID NO:22) |
| | CLDN-MMP(double)-CD3-Fc(Hole) |
| | DIVMTQSPSSLTVTAGEKVTMSCKSSQSLLNSGNQKNYLTWYQQK PGQPPKLLIYWASTRESGVPDRFTGSGSGTDFTLTISSVQAEDLAVY YCQNDYFYPFTFGSGTKLEIKGGGGSGGGGSGGGGSQVQLQQPGA ELVRPGASVKLSCKASGYTFTSYWINWVKQRPGQGLEWIGNIYPS DSYTNYNQKFKDKATLTVDKSSSTAYMQLSSPTSEDSAVYYCARVY YGNSFAYWGQGTTLTVSSSGRSENIRTASGRSPAIFTAMDIQMTQSP SSLSASVGDRVTITCRASQDIRNYLNWYQQKPGKAPKLLIYYTSRL ESGVPSRFSGSGSGTDYTLTISSLQPEDFATYYCQQGNTLPWTFGQG TKVEIKGGGGSGGGGSGGGGSEVQLVESGGGLVQPGGSLRLSCAA SGYSFTGYTMNWVRQAPGKGLEWVALINPYKGVSTYNQKFKDRF TISVDKSKNTAYLQMNSLRAEDTAVYYCARSGYYGDSDWYFDVW GQGTLVTVSSDKTHTCPPCPAPEAAGGPSVFLFPPKPKDTLMISRTP EVTCVVVDVSHEDPEVKFNWYVDGVEVHNAKTKPREEQYNSTYR VVSVLTVLHQDWLNGKEYKCKVSNKALGAPIEKTISKAKGQPREP QVYTLPPCRDELTKNQVSLWCLVKGFYPSDIAVEWESNGQPENNY KTTPPVLDSDGSFFLYSALTVDKSRWQQGNVFSCSVMHEALHNHY TQKSLSLSPGK (SEQ ID NO:25, wherein the underlined sequence represents MMP(double)) |

**PDL1×CD3(OKT3) [anti-PDL1 single-chain (VL-VH) and CD3 single-chain (VL-VH)]**

| | |
|---|---|
| PDL1×CD3 | PDL1-Fc(Knob) |
| | DIQMTQSPSSLSASVGDRVTITCRASQDVSTAVAWYQQKPGKAPKL LIYSASFLYSGVPSRFSGSGSGTDFTLTISSLQPEDFATYYCQQYLYH PATFGQGTKVEIKRGGGGSGGGGSGGGGSEVQLVESGGGLVQPGG SLRLSCAASGFTFSDSWIHWVRQAPGKGLEWVAWISPYGGSTYYA DSVKGRFTISADTSKNTAYLQMNSLRAEDTAVYYCARRHWPGGFD YWGQGTLVTVSADKTHTCPPCPAPEAAGGPSVFLFPPKPKDTLMIS RTPEVTCVVVDVSHEDPEVKFNWYVDGVEVHNAKTKPREEQYNS TYRVVSVLTVLHQDWLNGKEYKCKVSNKALGAPIEKTISKAKGQP REPQVCTLPPSRDELTKNQVSLSCAVKGFYPSDIAVEWESNGQPEN NYKTTPPVLDSDGSFKLVSKLTVDKSRWQQGNVFSCSVMHEALHN HYTQKSLSLSPGK (SEQ ID NO:26) |
| | CD3-Fc(Hole) |
| | QIVLTQSPAIMSASPGEKVTMTCSASSSVSYMNWYQQKSGTSPKR WIYDTSKLASGVPAHFRGSGSGTSYSLTISGMEAEDAATYYCQQWS SNPFTFGSGTKLEINGGGGSGGGGSGGGGSQVQLQQSGAELARPG ASVKMSCKASGYTFTRYTMHWVKQRPGQGLEWIGYINPSRGYTN YNQKFKDKATLTTDKSSSTAYMQLSSLTSEDSAVYYCARYYDDHY CLDYWGQGTTLTVSSDKTHTCPPCPAPEAAGGPSVFLFPPKPKDTL MISRTPEVTCVVVDVSHEDPEVKFNWYVDGVEVHNAKTKPREEQ YNSTYRVVSVLTVLHQDWLNGKEYKCKVSNKALGAPIEKTISKAK GQPREPQVYTLPPCRDELTKNQVSLWCLVKGFYPSDIAVEWESNGQ PENNYKTTPPVLDSDGSFFLYSALTVDKSRWQQGNVFSCSVMHEA LHNHYTQKSLSLSPGK (SEQ ID NO:27) |

**PDL1×CLDN-MMP(double)-CD3(OKT3) [anti-PDL1 single-chain (VL-VH) and CD3 single-chain (VL-VH)]**

| | |
|---|---|
| PDL1× CLDN-MMP(double)-CD3 | PDL1-Fc(Knob) |
| | DIQMTQSPSSLSASVGDRVTITCRASQDVSTAVAWYQQKPGKAPKL LIYSASFLYSGVPSRFSGSGSGTDFTLTISSLQPEDFATYYCQQYLYH PATFGQGTKVEIKRGGGGSGGGGSGGGGSEVQLVESGGGLVQPGG SLRLSCAASGFTFSDSWIHWVRQAPGKGLEWVAWISPYGGSTYYA DSVKGRFTISADTSKNTAYLQMNSLRAEDTAVYYCARRHWPGGFD YWGQGTLVTVSADKTHTCPPCPAPEAAGGPSVFLFPPKPKDTLMIS RTPEVTCVVVDVSHEDPEVKFNWYVDGVEVHNAKTKPREEQYNS TYRVVSVLTVLHQDWLNGKEYKCKVSNKALGAPIEKTISKAKGQP REPQVCTLPPSRDELTKNQVSLSCAVKGFYPSDIAVEWESNGQPEN NYKTTPPVLDSDGSFKLVSKLTVDKSRWQQGNVFSCSVMHEALHN HYTQKSLSLSPGK (SEQ ID NO:26) |
| | CLDN-MMP(double)-CD3-Fc(Hole) |
| | DIVMTQSPSSLTVTAGEKVTMSCKSSQSLLNSGNQKNYLTWYQQK PGQPPKLLIYWASTRESGVPDRFTGSGSGTDFTLTISSVQAEDLAVY YCQNDYFYPFTFGSGTKLEIKGGGGSGGGGSGGGGSQVQLQQPGA ELVRPGASVKLSCKASGYTFTSYWINWVKQRPGQGLEWIGNIYPS DSYTNYNQKFKDKATLTVDKSSSTAYMQLSSPTSEDSAVYYCARVY YGNSFAYWGQGTTLTVSSSGRSENIRTASGRSPAIFTAQIVLTQSPAI MSASPGEKVTMTCSASSSVSYMNWYQQKSGTSPKRWIYDTSKLAS GVPAHFRGSGSGTSYSLTISGMEAEDAATYYCQQWSSNPFTFGSGT KLEINGGGGSGGGGSGGGGSQVQLQQSGAELARPGASVKMSCKA SGYTFTRYTMHWVKQRPGQGLEWIGYINPSRGYTNYNQKFKDKA TLTTDKSSSTAYMQLSSLTSEDSAVYYCARYYDDHYCLDYWGQGT TLTVSSDKTHTCPPCPAPEAAGGPSVFLFPPKPKDTLMISRTPEVTCV VVDVSHEDPEVKFNWYVDGVEVHNAKTKPREEQYNSTYRVVSVL TVLHQDWLNGKEYKCKVSNKALGAPIEKTISKAKGQPREPQVYTL PPCRDELTKNQVSLWCLVKGFYPSDIAVEWESNGQPENNYKTTPPV LDSDGSFFLYSALTVDKSRWQQGNVFSCSVMHEALHNHYTQKSLS LSPGK (SEQ ID NO:29, wherein the underlined sequence represents MMP(double)) |

**PDL1×NGR-HA-MMP(double)-CD3(OKT3) [anti-PDL1 single-chain (VL-VH) and CD3 single-chain (VL-VH)]**

| | |
|---|---|
| PDL1× NGR-HA-MMP(double)-CD3 (OKT3) | PDL1-Fc(Knob) |
| | DIQMTQSPSSLSASVGDRVTITCRASQDVSTAVAWYQQKPGKAPKL LIYSASFLYSGVPSRFSGSGSGTDFTLTISSLQPEDFATYYCQQYLYH PATFGQGTKVEIKRGGGGSGGGGSGGGGSEVQLVESGGGLVQPGG SLRLSCAASGFTFSDSWIHWVRQAPGKGLEWVAWISPYGGSTYYA DSVKGRFTISADTSKNTAYLQMNSLRAEDTAVYYCARRHWPGGFD YWGQGTLVTVSADKTHTCPPCPAPEAAGGPSVFLFPPKPKDTLMIS RTPEVTCVVVDVSHEDPEVKFNWYVDGVEVHNAKTKPREEQYNS TYRVVSVLTVLHQDWLNGKEYKCKVSNKALGAPIEKTISKAKGQP REPQVCTLPPSRDELTKNQVSLSCAVKGFYPSDIAVEWESNGQPEN NYKTTPPVLDSDGSFKLVSKLTVDKSRWQQGNVFSCSVMHEALHN HYTQKSLSLSPGK (SEQ ID NO:26) |
| | NGR-HA-MMP(double)-CD3-Fc(Hole) |
| | GGGGSCNGRCGGGGSGGGGSCNGRCNQHTIDLADSSGRSENIRTA SGRSPAIFTAQIVLTQSPAIMSASPGEKVTMTCSASSSVSYMNWYQQ KSGTSPKRWIYDTSKLASGVPAHFRGSGSGTSYSLTISGMEAEDAAT YYCQQWSSNPFTFGSGTKLEINGGGGSGGGGSGGGGSQVQLQQSG AELARPGASVKMSCKASGYTFTRYTMHWVKQRPGQGLEWIGYIN PSRGYTNYNQKFKDKATLTTDKSSSTAYMQLSSLTSEDSAVYYCAR YYDDHYCLDYWGQGTTLTVSSDKTHTCPPCPAPEAAGGPSVFLFPP KPKDTLMISRTPEVTCVVVDVSHEDPEVKFNWYVDGVEVHNAKT KPREEQYNSTYRVVSVLTVLHQDWLNGKEYKCKVSNKALGAPIEK TISKAKGQPREPQVYTLPPCRDELTKNQVSLWCLVKGFYPSDIAVE WESNGQPENNYKTTPPVLDSDGSFFLYSALTVDKSRWQQGNVFSC SVMHEALHNHYTQKSLSLSPGK (SEQ ID NO:31) |

**G4S**: GGGGS (SEQ ID NO:32)
**NGR**: GGGGSCNGRC (SEQ ID NO:33)
**NGR₂**: GGGGSCNGRCGGGGSGGGGSCNGRC (SEQ ID NO:34)
**Human CD3 Clone: OKT3**
**VL**


 (SEQ ID NO:35)

The CDR1 sequence of OKT3 VL is SSVSY (SEQ ID NO:48).

The CDR2 sequence of OKT3 VL is DTS.

The CDR3 sequence of OKT3 VL is QQWSSNPFTF (SEQ ID NO:49).
**VH:**


 ((SEQ ID NO:36)

The CDR1 sequence of OKT3 VH is GYTFTRYT (SEQ ID NO:50).

The CDR2 sequence of OKT3 VH is INPSRGYT (SEQ ID NO:51).

The CDR3 sequence of OKT3 VH is ARYYDDHYCLDYW (SEQ ID NO:52).
**Human CD3 Clone: L2K**
**VL:** DIQLTQSPAIMSASPGEKVTMTCRASSSVSYMNWYQQKSGTSPKRWIYDTSKVASGVPYRFSGSGSGT SYSLTISSMEAEDAATYYCQQWSS NPLTFGAGTKLELKVE (SEQ ID NO:37)
**VH:** 
DIKLQQSGAELARPGASVKMSCKTSGYTFTRYTMHWVKQRPGQGLEWIGYINPSRGYTNYNQKFKD
KATLTTDKSSSTAYMQLSSLTSEDSAVYYCARYYDDHYCLDYWGQGTTLTVSS (SEQ ID NO:38)
**Human CD3 Clone: UCHT1**
**VL:**

MDIQMTQSPSSLSASVGDRVTITCRASQDIRNYLNWYQQKPGKAPKLLIYYTSRLESGVPSRFSGSGSG
TDYTLTISSLQPEDFATYYCQQGNTLPWTFGQGTKVEIK (SEQ ID NO:39)

The CDR1 sequence of UCHT1 VL is QDIRNY (SEQ ID NO:53).

The CDR2 sequence of UCHT1 VL is YTS.

The CDR3 sequence of UCHT1 VL is QQGNTLPWTF (SEQ ID NO:54).
**VH:**


 (SEQ ID NO:40)

The CDR1 sequence of UCHT1 VH is GYSFTGYT (SEQ ID NO:55).

The CDR2 sequence of UCHT1 VH is INPYKGVS (SEQ ID NO:56).

The CDR3 sequence of UCHT1 VH is ARSGYYGDSDWYFDV (SEQ ID NO:57).
**Murine CD3 Clone: SP34**
**VL:**

QAVVTQESADTTSPGETVTITCRSSTGAVTTSNYANWVQEKPDHLETGLTGGTNKRAPGVPARESGSLI
GDKAAITITGAQTEDEAIYFCALWYSNLWVFGGGTKLTVL (SEQ ID NO:41)

The CDR1 sequence of SP34 VL is TGAVTTSNY (SEQ ID NO:58).

The CDR2 sequence of SP34 VL is GTN.

The CDR3 sequence of SP34 VL is ALWYSNLWVF (SEQ ID NO:59).
**VH:**

EVQLVESGGGLVQPKGSLKLSCAASGFTFVTYAMNTWVRQAPGKGLEWVARIRSKYNNYATYYADS
VKDRFTTSRDDSQSILYLQMNNLKTEDTAMYYCVRHGNFGNSYVSWFAYWGQGTLVTVSS (SEQ ID
NO:42)

The CDR1 sequence of SP34 VH is GETFVTYA (SEQ ID NO:60).

The CDR2 sequence of SP34 VH is IRSKYNNYAT (SEQ ID NO:61).

The CDR3 sequence of SP34 VH is VRHGNFGNSYVSWFAYW (SEQ ID NO:62).
**Murine CD3 Clone: 17A2**
**VL:**

DIVMTQSPKSMSISIGDRVTMNCKASQNVINYIAWYQQKPGQSPKLLIYYASNRYTGVPDRFTGGGSG
TDFTLTINSVQAEDAAFYYCQRIYNSPWTFGGGTKLELKR (SEQ ID NO:43)

The CDR1 sequence of 17A2 VL is QNVINY (SEQ ID NO:63).

The CDR2 sequence of 17A2 VL is YAS.

The CDR3 sequence of 17A2 VL is QRIYNSPWTF (SEQ ID NO:64).
**VH:**

EVKLVESGGGLVQPGGSLRLSCAASGFTFSNFPMAWVRQAPKKGLEWVASISSGGGGTFYPDSVKDR
FTISRNNAKNTLYLQIDSLRSEDTASYYCVRPQGGFASWGQGTLVTVSS (SEQ ID NO:44)

The CDR1 sequence of 17A2 VH is GFTFSNFP (SEQ ID NO:65).

The CDR2 sequence of 17A2 VH is ISSGGGGT (SEQ ID NO:66).

The CDR3 sequence of 17A2 VH is VRPQGGFASW (SEQ ID NO:67).
**Murine CD3 Clone: 2C11**
**VL:**

DIQMTQSPSSLPASLGDRVTINCQASQDISNYLNWYQQKPGKAPKLLIYYTNKLADGVPSRFSGSGSG
RDSSFTISSLESEDIGSYYCQQYYNYPWTFGPGTKLEIK (SEQ ID NO:45)

The CDR1 sequence of 2C11 VL is QDISNY (SEQ ID NO:68).

The CDR2 sequence of 2C11 VL is YTN.

The CDR3 sequence of 2C11 VL is QQYYNYPWTF (SEQ ID NO:69).
**VH:**

EVQLVESGGGLVQPGKSLKLSCEASGFTFSGYGMHWVRQAPGRGLESVAYITSSSINIKYADAVKGRFT
VSRDNAKNLLFLQMNILKSEDTAMYYCARFDWDKNYWGQGTMVTVSS (SEQ ID NO:46)

The CDR1 sequence of 2C11 VH is GFTFSGYG (SEQ ID NO:70).

The CDR2 sequence of 2C11 VH is ITSSSINI (SEQ ID NO:71).

The CDR3 sequence of 2C11 VH is ARFDWDKNYW (SEQ ID NO:72).
**Signal peptide**
MYRMQLLSCIALSLALVTNS,SEQ ID NO:47
**Cleavable linkers**
MPYDLYHP, (SEQ ID NO:83)
DSGGFMLT, (SEQ ID NO:84)
HEQLTV, (SEQ ID NO:85)
RAAAVKSP, (SEQ ID NO:86)
VPLSLY, (SEQ ID NO:87)
ISSGLLSGRS, (SEQ ID NO:88)
DLLAVVAAS, (SEQ ID NO:89)
LSGSDN, (SEQ ID NO:90)
SGSDN, (SEQ ID NO:91)
HPVGLLAR, (SEQ ID NO:92)
LSGRSDNH, (SEQ ID NO:93)
VPLSLYSG, (SEQ ID NO:94)

**HA-MMP(double)-Anti-CD3(OKT3)-Fc(Hole)** 
NQHTIDLADSSGRSENIRTASGRSPAIFTAQIVLTQSPAIMSASPGEKVTMTCSASSSVSYMNWYQQKSG
TSPKRWIYDTSKLASGVPAHFRGSGSGTSYSLTISGMEAEDAATYYCQQWSSNPFTFGSGTKLEINGGG
GSGGGGSGGGGSQVQLQQSGAELARPGASVKMSCKASGYTFTRYTMHWVKQRPGQGLEWIGYINPS
 
RGYTNYNQKFKDKATLTTDKSSSTAYMQLSSLTSEDSAVYYCARYYDDHYCLDYWGQGTTLTVSSDK
THTCPPCPAPEAAGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALGAPIEKTISKAKGQPREPQVYTLPPCRD
ELTKNQVSLWCLVKGFYPSDIAVEWESNGQPENNYKTTPPVLDSDGSFFLYSALTVDKSRWQQGNVFS
CSVMHEALHNHYTQKSLSLSPGK (SEQ ID NO:95)
**HA-MMP-Anti-CD3(OKT3)-Fc(Hole)** 
NQHTIDLADSSGRSPAIFTAQIVLTQSPAIMSASPGEKVTMTCSASSSVSYMNWYQQKSGTSPKRWIYD
TSKLASGVPAHFRGSGSGTSYSLTISGMEAEDAATYYCQQWSSNPFTFGSGTKLEINGGGGSGGGGSG
GGGSQVQLQQSGAELARPGASVKMSCKASGYTFTRYTMHWVKQRPGQGLEWIGYINPSRGYTNYN
QKFKDKATLTTDKSSSTAYMQLSSLTSEDSAVYYCARYYDDHYCLDYWGQGTTLTVSSDKTHTCPPCP
APEAAGGPSVFLFPPKPKDTLMISRTPEVTCVVVDVSHEDPEVKFNWYVDGVEVHNAKTKPREEQYN
STYRVVSVLTVLHQDWLNGKEYKCKVSNKALGAPIEKTISKAKGQPREPQVYTLPPCRDELTKNQVS
LWCLVKGFYPSDIAVEWESNGQPENNYKTTPPVLDSDGSFFLYSALTVDKSRWQQGNVFSCSVMHEA
LHNHYTQKSLSLSPGK (SEQ ID NO:96)
**NGR-HA-MMP(Double)-Anti-CD3(OKT3)-Fc(Hole)** 
GGGGSCNGRCGGGGSGGGGSCNGRCNQHTIDLADSSGRSENIRTASGRSPAIFTAQIVLTQSPAIMSASP
GEKVTMTCSASSSVSYMNWYQQKSGTSPKRWIYDTSKLASGVPAHFRGSGSGTSYSLTISGMEAEDA
ATYYCQQWSSNPFTFGSGTKLEINGGGGSGGGGSGGGGSQVQLQQSGAELARPGASVKMSCKASGY
TFTRYTMHWVKQRPGQGLEWIGYINPSRGYTNYNQKFKDKATLTTDKSSSTAYMQLSSLTSEDSAVY
YCARYYDDHYCLDYWGQGTTLTVSSDKTHTCPPCPAPEAAGGPSVFLFPPKPKDTLMISRTPEVTCVV
VDVSHEDPEVKFNWYVDGVEVHNAKTKPREEQYNSTYRVVSVLTVLHQDWLNGKEYKCKVSNKAL
GAPIEKTISKAKGQPREPQVYTLPPCRDELTKNQVSLWCLVKGFYPSDIAVEWESNGQPENNYKTTPPV
LDSDGSFFLYSALTVDKSRWQQGNVFSCSVMHEALHNHYTQKSLSLSPGK (SEQ ID NO:97)
**CLDN-MMP-Anti-CD3(UCHT1 VH VL)-Fc(Hole)** 
DIVMTQSPSSLTVTAGEKVTMSCKSSQSLLNSGNQKNYLTWYQQKPGQPPKLLIYWASTRESGVPDRF
TGSGSGTDFTLTISSVQAEDLAVYYCQNDYFYPFTFGSGTKLEIKGGGGSGGGGSGGGGSQVQLQQPG
AELVRPGASVKLSCKASGYTFTSYWINWVKQRPGQGLEWIGNIYPSDSYTNYNQKFKDKATLTVDKS
SSTAYMQLSSPTSEDSAVYYCARVYYGNSFAYWGQGTTLTVSSSGFIANPVTAEVQLVESGGGLVQPG
GSLRLSCAASGYSFTGYTMNWVRQAPGKGLEWVALINPYKGVSTYNQKFKDRFTISVDKSKNTAYLQ
MNSLRAEDTAVYYCARSGYYGDSDWYFDVWGQGTLVTVSSGGGGSGGGGSGGGGSMDIQMTQSPS
SLSASVGDRVTITCRASQDIRNYLNWYQQKPGKAPKLLIYYTSRLESGVPSRFSGSGSGTDYTLTISSLQ
PEDFATYYCQQGNTLPWTFGQGTKVEIKDKTHTCPPCPAPEAAGGPSVFLFPPKPKDTLMISRTPEVTC
VVVDVSHEDPEVKFNWYVDGVEVHNAKTKPREEQYNSTYRVVSVLTVLHQDWLNGKEYKCKVSN
KALGAPIEKTISKAKGQPREPQVYTLPPCRDELTKNQVSLWCLVKGFYPSDIAVEWESNGQPENNYKT
TPPVLDSDGSFFLYSALTVDKSRWQQGNVFSCSVMHEALHNHYTQKSLSLSPGK (SEQ ID NO:98)
**CLDN-MMP(Double)-Anti-CD3(UCHT1 VH VL)-Fc(Hole)** 
DIVMTQSPSSLTVTAGEKVTMSCKSSQSLLNSGNQKNYLTWYQQKPGQPPKLLIYWASTRESGVPDRF
TGSGSGTDFTLTISSVQAEDLAVYYCQNDYFYPFTFGSGTKLEIKGGGGSGGGGSGGGGSQVQLQQPG
 
AELVRPGASVKLSCKASGYTFTSYWINWVKQRPGQGLEWIGNIYPSDSYTNYNQKFKDKATLTVDKS
SSTAYMQLSSPTSEDSAVYYCARVYYGNSFAYWGQGTTLTVSSSGRSENIRTASGRSPAIFTAEVQLVES
GGGLVQPGGSLRLSCAASGYSFTGYTMNWVRQAPGKGLEWVALINPYKGVSTYNQKFKDRFTISVD
KSKNTAYLQMNSLRAEDTAVYYCARSGYYGDSDWYFDVWGQGTLVTVSSGGGGSGGGGSGGGGSM
DIQMTQSPSSLSASVGDRVTITCRASQDIRNYLNWYQQKPGKAPKLLIYYTSRLESGVPSRFSGSGSGT
DYTLTISSLQPEDFATYYCQQGNTLPWTFGQGTKVEIKDKTHTCPPCPAPEAAGGPSVFLFPPKPKDTL
MISRTPEVTCVVVDVSHEDPEVKFNWYVDGVEVHNAKTKPREEQYNSTYRVVSVLTVLHQDWLNGK
EYKCKVSNKALGAPIEKTISKAKGQPREPQVYTLPPCRDELTKNQVSLWCLVKGFYPSDIAVEWESNG
QPENNYKTTPPVLDSDGSFFLYSALTVDKSRWQQGNVFSCSVMHEALHNHYTQKSLSLSPGK (SEQ ID
NO:99)
**CLDN-HA-MMP-Anti-CD3(UCHT1)-Fc(Hole)** 
DIVMTQSPSSLTVTAGEKVTMSCKSSQSLLNSGNQKNYLTWYQQKPGQPPKLLIYWASTRESGVPDRF
TGSGSGTDFTLTISSVQAEDLAVYYCQNDYFYPFTFGSGTKLEIKGGGGSGGGGSGGGGSQVQLQQPG
AELVRPGASVKLSCKASGYTFTSYWINWVKQRPGQGLEWIGNIYPSDSYTNYNQKFKDKATLTVDKS
SSTAYMQLSSPTSEDSAVYYCARVYYGNSFAYWGQGTTLTVSSNQHTIDLADSSGFIANPVTAMDIQM
TQSPSSLSASVGDRVTITCRASQDIRNYLNWYQQKPGKAPKLLIYYTSRLESGVPSRFSGSGSGTDYTL
TISSLQPEDFATYYCQQGNTLPWTFGQGTKVEIKGGGGSGGGGSGGGGSEVQLVESGGGLVQPGGSLR
LSCAASGYSFTGYTMNWVRQAPGKGLEWVALINPYKGVSTYNQKFKDRFTISVDKSKNTAYLQMNS
LRAEDTAVYYCARSGYYGDSDWYFDVWGQGTLVTVSSDKTHTCPPCPAPEAAGGPSVFLFPPKPKDT
LMISRTPEVTCVVVDVSHEDPEVKFNWYVDGVEVHNAKTKPREEQYNSTYRVVSVLTVLHQDWLNG
KEYKCKVSNKALGAPIEKTISKAKGQPREPQVYTLPPCRDELTKNQVSLWCLVKGFYPSDIAVEWESN
GQPENNYKTTPPVLDSDGSFFLYSALTVDKSRWQQGNVFSCSVMHEALHNHYTQKSLSLSPGK (SEQ
ID NO:100)
**CLDN-[H(MMP)A]*2-Anti-CD3(UCHT1)-Fc(Hole)** 
DIVMTQSPSSLTVTAGEKVTMSCKSSQSLLNSGNQKNYLTWYQQKPGQPPKLLIYWASTRESGVPDRF
TGSGSGTDFTLTISSVQAEDLAVYYCQNDYFYPFTFGSGTKLEIKGGGGSGGGGSGGGGSQVQLQQPG
AELVRPGASVKLSCKASGYTFTSYWINWVKQRPGQGLEWIGNIYPSDSYTNYNQKFKDKATLTVDKS
SSTAYMQLSSPTSEDSAVYYCARVYYGNSFAYWGQGTTLTVSSNQHIANPVDSNQHIANPVDSMDIQM
TQSPSSLSASVGDRVTITCRASQDIRNYLNWYQQKPGKAPKLLIYYTSRLESGVPSRFSGSGSGTDYTL
TISSLQPEDFATYYCQQGNTLPWTFGQGTKVEIKGGGGSGGGGSGGGGSEVQLVESGGGLVQPGGSLR
LSCAASGYSFTGYTMNWVRQAPGKGLEWVALINPYKGVSTYNQKFKDRFTISVDKSKNTAYLQMNS
LRAEDTAVYYCARSGYYGDSDWYFDVWGQGTLVTVSSDKTHTCPPCPAPEAAGGPSVFLFPPKPKDT
LMISRTPEVTCVVVDVSHEDPEVKFNWYVDGVEVHNAKTKPREEQYNSTYRVVSVLTVLHQDWLNG
KEYKCKVSNKALGAPIEKTISKAKGQPREPQVYTLPPCRDELTKNQVSLWCLVKGFYPSDIAVEWESN
GQPENNYKTTPPVLDSDGSFFLYSALTVDKSRWQQGNVFSCSVMHEALHNHYTQKSLSLSPGK (SEQ
ID NO:101)
**CLDN-MMP-Anti-CD3(UCHT1)-Fc(Hole)** DIVMTQSPSSLTVTAGEKVTMSCKSSQSLLNSGNQKNYLTWYQQKPGQPPKLLIYWASTRESGVPDRF 
TGSGSGTDFTLTISSVQAEDLAVYYCQNDYFYPFTFGSGTKLEIKGGGGSGGGGSGGGGSQVQLQQPG
AELVRPGASVKLSCKASGYTFTSYWINWVKQRPGQGLEWIGNIYPSDSYTNYNQKFKDKATLTVDKS
SSTAYMQLSSPTSEDSAVYYCARVYYGNSFAYWGQGTTLTVSSSGFIANPVTAMDIQMTQSPSSLSASV
GDRVTITCRASQDIRNYLNWYQQKPGKAPKLLIYYTSRLESGVPSRFSGSGSGTDYTLTISSLQPEDFAT
YYCQQGNTLPWTFGQGTKVEIKGGGGSGGGGSGGGGSEVQLVESGGGLVQPGGSLRLSCAASGYSFT
GYTMNWVRQAPGKGLEWVALINPYKGVSTYNQKFKDRFTISVDKSKNTAYLQMNSLRAEDTAVYYC
ARSGYYGDSDWYFDVWGQGTLVTVSSDKTHTCPPCPAPEAAGGPSVFLFPPKPKDTLMISRTPEVTCV
VVDVSHEDPEVKFNWYVDGVEVHNAKTKPREEQYNSTYRVVSVLTVLHQDWLNGKEYKCKVSNK
ALGAPIEKTISKAKGQPREPQVYTLPPCRDELTKNQVSLWCLVKGFYPSDIAVEWESNGQPENNYKTT
PPVLDSDGSFFLYSALTVDKSRWQQGNVFSCSVMHEALHNHYTQKSLSLSPGK (SEQ ID NO:102)
**CLDN-(G4S)₄-Anti-CD3(UCHT1)-Fc(Hole)** 
DIVMTQSPSSLTVTAGEKVTMSCKSSQSLLNSGNQKNYLTWYQQKPGQPPKLLIYWASTRESGVPDRF
TGSGSGTDFTLTISSVQAEDLAVYYCQNDYFYPFTFGSGTKLEIKGGGGSGGGGSGGGGSQVQLQQPG
AELVRPGASVKLSCKASGYTFTSYWINWVKQRPGQGLEWIGNIYPSDSYTNYNQKFKDKATLTVDKS
SSTAYMQLSSPTSEDSAVYYCARVYYGNSFAYWGQGTTLTVSSGGGGSGGGGSGGGGSGGGGSMDIQ
MTQSPSSLSASVGDRVTITCRASQDIRNYLNWYQQKPGKAPKLLIYYTSRLESGVPSRFSGSGSGTDYT
LTISSLQPEDFATYYCQQGNTLPWTFGQGTKVEIKGGGGSGGGGSGGGGSEVQLVESGGGLVQPGGSL
RLSCAASGYSFTGYTMNWVRQAPGKGLEWVALINPYKGVSTYNQKFKDRFTISVDKSKNTAYLQMN
SLRAEDTAVYYCARSGYYGDSDWYFDVWGQGTLVTVSSDKTHTCPPCPAPEAAGGPSVFLFPPKPKD
TLMISRTPEVTCVVVDVSHEDPEVKFNWYVDGVEVHNAKTKPREEQYNSTYRVVSVLTVLHQDWLN
GKEYKCKVSNKALGAPIEKTISKAKGQPREPQVYTLPPCRDELTKNQVSLWCLVKGFYPSDIAVEWES
NGQPENNYKTTPPVLDSDGSFFLYSALTVDKSRWQQGNVFSCSVMHEALHNHYTQKSLSLSPGK
(SEQ ID NO:103)
**CLDN-(G4S)₂-Anti-CD3(UCHT1)-Fc(Hole)** 
DIVMTQSPSSLTVTAGEKVTMSCKSSQSLLNSGNQKNYLTWYQQKPGQPPKLLIYWASTRESGVPDRF
TGSGSGTDFTLTISSVQAEDLAVYYCQNDYFYPFTFGSGTKLEIKGGGGSGGGGSGGGGSQVQLQQPG
AELVRPGASVKLSCKASGYTFTSYWINWVKQRPGQGLEWIGNIYPSDSYTNYNQKFKDKATLTVDKS
SSTAYMQLSSPTSEDSAVYYCARVYYGNSFAYWGQGTTLTVSSGGGGSGGGGSMDIQMTQSPSSLSAS
VGDRVTITCRASQDIRNYLNWYQQKPGKAPKLLIYYTSRLESGVPSRFSGSGSGTDYTLTISSLQPEDF
ATYYCQQGNTLPWTFGQGTKVEIKGGGGSGGGGSGGGGSEVQLVESGGGLVQPGGSLRLSCAASGY
SFTGYTMNWVRQAPGKGLEWVALINPYKGVSTYNQKFKDRFTISVDKSKNTAYLQMNSLRAEDTAV
YYCARSGYYGDSDWYFDVWGQGTLVTVSSDKTHTCPPCPAPEAAGGPSVFLFPPKPKDTLMISRTPE
VTCVVVDVSHEDPEVKFNWYVDGVEVHNAKTKPREEQYNSTYRVVSVLTVLHQDWLNGKEYKCK
VSNKALGAPIEKTISKAKGQPREPQVYTLPPCRDELTKNQVSLWCLVKGFYPSDIAVEWESNGQPENN
YKTTPPVLDSDGSFFLYSALTVDKSRWQQGNVFSCSVMHEALHNHYTQKSLSLSPGK (SEQ ID NO:104)
**CLDN-(MMP)₂-Anti-CD3(UCHT1)-Fc(Hole)** 
DIVMTQSPSSLTVTAGEKVTMSCKSSQSLLNSGNQKNYLTWYQQKPGQPPKLLIYWASTRESGVPDRF
TGSGSGTDFTLTISSVQAEDLAVYYCQNDYFYPFTFGSGTKLEIKGGGGSGGGGSGGGGSQVQLQQPG
 
AELVRPGASVKLSCKASGYTFTSYWINWVKQRPGQGLEWIGNIYPSDSYTNYNQKFKDKATLTVDKS
SSTAYMQLSSPTSEDSAVYYCARVYYGNSFAYWGQGTTLTVSSSGRSENIRTASGRSPAIFTAMDIQMT
QSPSSLSASVGDRVTITCRASQDIRNYLNWYQQKPGKAPKLLIYYTSRLESGVPSRFSGSGSGTDYTLTI
SSLQPEDFATYYCQQGNTLPWTFGQGTKVEIKGGGGSGGGGSGSEVQLVESGGGLVQPGGSLRLSCA
ASGYSFTGYTMNWVRQAPGKGLEWVALINPYKGVSTYNQKFKDRFTISVDKSKNTAYLQMNSLRAE
DTAVYYCARSGYYGDSDWYFDVWGQGTLVTVSSDKTHTCPPCPAPEAAGGPSVFLFPPKPKDTLMIS
RTPEVTCVVVDVSHEDPEVKFNWYVDGVEVHNAKTKPREEQYNSTYRVVSVLTVLHQDWLNGKEY
KCKVSNKALGAPIEKTISKAKGQPREPQVYTLPPCRDELTKNQVSLWCLVKGFYPSDIAVEWESNGQP
ENNYKTTPPVLDSDGSFFLYSALTVDKSRWQQGNVFSCSVMHEALHNHYTQKSLSLSPGK (SEQ ID
NO:105)
**CLDN-[H(MMP)A]-Anti-CD3(UCHT1)-Fc(Hole)** 
DIVMTQSPSSLTVTAGEKVTMSCKSSQSLLNSGNQKNYLTWYQQKPGQPPKLLIYWASTRESGVPDRF
TGSGSGTDFTLTISSVQAEDLAVYYCQNDYFYPFTFGSGTKLEIKGGGGSGGGGSGGGGSQVQLQQPG
AELVRPGASVKLSCKASGYTFTSYWINWVKQRPGQGLEWIGNIYPSDSYTNYNQKFKDKATLTVDKS
SSTAYMQLSSPTSEDSAVYYCARVYYGNSFAYWGQGTTLTVSSNQHIANPVDSMDIQMTQSPSSLSAS
VGDRVTITCRASQDIRNYLNWYQQKPGKAPKLLIYYTSRLESGVPSRFSGSGSGTDYTLTISSLQPEDF
ATYYCQQGNTLPWTFGQGTKVEIKGGGGSGGGGSGGGGSEVQLVESGGGLVQPGGSLRLSCAASGY
SFTGYTMNWVRQAPGKGLEWVALINPYKGVSTYNQKFKDRFTISVDKSKNTAYLQMNSLRAEDTAV
YYCARSGYYGDSDWYFDVWGQGTLVTVSSDKTHTCPPCPAPEAAGGPSVFLFPPKPKDTLMISRTPE
VTCVVVDVSHEDPEVKFNWYVDGVEVHNAKTKPREEQYNSTYRVVSVLTVLHQDWLNGKEYKCK
VSNKALGAPIEKTISKAKGQPREPQVYTLPPCRDELTKNQVSLWCLVKGFYPSDIAVEWESNGQPENN
YKTTPPVLDSDGSFFLYSALTVDKSRWQQGNVFSCSVMHEALHNHYTQKSLSLSPGK (SEQ ID NO:106)
**Anti-CLDN(VL-CL)** 
DIVMTQSPSSLTVTAGEKVTMSCKSSQSLLNSGNQKNYLTWYQQKPGQPPKLLIYWASTRESGVPDRF
TGSGSGTDFTLTISSVQAEDLAVYYCQNDYFYPFTFGSGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTH
QGLSSPVTKSFNRGEC (SEQ ID NO:107)
**Anti-CLDN(VH-CH)-MMP(Double)-Anti-CD3-Fc(Hole)**
QVQLQQPGAELVRPGASVKLSCKASGYTFTSYWINWVKQRPGQGLEWIGNIYPSDSYTNYNQKFKD KATLTVDKSSSTAYMQLSSPTSEDSAVYYCARVYYGNSFAYWGQGTTLTVSSASTKGPSVFPLAPSSKS TSGGTAALGCLVKDYFPEPVTVSWNSGALTSGVHTFPAVLQSSGLYSLSSVVTVPSSSLGTQTYICNVN HKPSNTKVDKRVSGRSENIRTASGRSPAIFTAMDIQMTQSPSSLSASVGDRVTITCRASQDIRNYLNWY QQKPGKAPKLLIYYTSRLESGVPSRFSGSGSGTDYTLTISSLQPEDFATYYCQQGNTLPWTFGQGTKVE IKGGGGSGGGGSGGGGSEVQLVESGGGLVQPGGSLRLSCAASGYSFTGYTMNWVRQAPGKGLEWVA LINPYKGVSTYNQKFKDRFTISVDKSKNTAYLQMNSLRAEDTAVYYCARSGYYGDSDWYFDVWGQG TLVTVSSDKTHTCPPCPAPEAAGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALGAPIEKTISKAKGQPREPQ VYTLPPCRDELTKNQVSLWCLVKGFYPSDIAVEWESNGQPENNYKTTPPVLDSDGSFFLYSALTVDKS RWQQGNVFSCSVMHEALHNHYTQKSLSLSPGK (SEQ ID NO:108, wherein the underlined sequence represents Anti-CLDN(VH-CH))
**Anti-CLDN(VH-CH)-MMP-Anti-CD3-Fc(Hole)**
QVQLQQPGAELVRPGASVKLSCKASGYTFTSYWINWVKQRPGQGLEWIGNIYPSDSYTNYNQKFKD KATLTVDKSSSTAYMQLSSPTSEDSAVYYCARVYYGNSFAYWGQGTTLTVSSASTKGPSVFPLAPSSKS TSGGTAALGCLVKDYFPEPVTVSWNSGALTSGVHTFPAVLQSSGLYSLSSVVTVPSSSLGTQTYICNVN HKPSNTKVDKRVSGRSENIRTAMDIQMTQSPSSLSASVGDRVTITCRASQDIRNYLNWYQQKPGKAPK LLIYYTSRLESGVPSRFSGSGSGTDYTLTISSLQPEDFATYYCQQGNTLPWTFGQGTKVEIKGGGGSGG GGSGGGGSEVQLVESGGGLVQPGGSLRLSCAASGYSFTGYTMNWVRQAPGKGLEWVALINPYKGVS TYNQKFKDRFTISVDKSKNTAYLQMNSLRAEDTAVYYCARSGYYGDSDWYFDVWGQGTLVTVSSDK THTCPPCPAPEAAGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALGAPIEKTISKAKGQPREPQVYTLPPCRD ELTKNQVSLWCLVKGFYPSDIAVEWESNGQPENNYKTTPPVLDSDGSFFLYSALTVDKSRWQQGNVFS CSVMHEALHNHYTQKSLSLSPGK (SEQ ID NO:109, wherein the underlined sequence representsAnti-CLDN(VH-CH))
**Mutated IL2 (F42A, R38L):** 
APTSSSTKKTQLQLEHLLLDLQMILNGINNYKNPKLTLMLTAKFYMPKKATELKHLQCLEEELKPLEE
VLNLAQSKNFHLRPRDLISNINVIVLELKGSETTFMCEYADETATIVEFLNRWITFCQSIISTLT (SEQ ID
NO:110)
**CLDN-MMP(double)-CD3-mutIL2 [anti-CLDN single-chain (VL-VH) and CD3 single-chain (VL-VH) and mut IL2 (F42A, R38L)]**

| | |
|---|---|
| CLDN-MMP(double)-CD3-Fc(Hole)-**mutIL2** | DIVMTQSPSSLTVTAGEKVTMSCKSSQSLLNSGNQKNYLTWYQQ KPGQPPKLLIYWASTRESGVPDRFTGSGSGTDFTLTISSVQAEDLA VYYCQNDYFYPFTFGSGTKLEIKGGGGSGGGGSGGGGSQVQLQQ PGAELVRPGASVKLSCKASGYTFTSYWINWVKQRPGQGLEWIGN IYPSDSYTNYNQKFKDKATLTVDKSSSTAYMQLSSPTSEDSAVYYC ARVYYGNSFAYWGQGTTLTVSSSGRSENIRTASGRSPAIFTAMDIQ MTQSPSSLSASVGDRVTITCRASQDIRNYLNWYQQKPGKAPKLLI YYTSRLESGVPSRFSGSGSGTDYTLTISSLQPEDFATYYCQQGNTL PWTFGQGTKVEIKGGGGSGGGGSGGGGSEVQLVESGGGLVQPGG SLRLSCAASGYSFTGYTMNWVRQAPGKGLEWVALINPYKGVSTY NQKFKDRFTISVDKSKNTAYLQMNSLRAEDTAVYYCARSGYYGD SDWYFDVWGQGTLVTVSSDKTHTCPPCPAPEAAGGPSVFLFPPKP KDTLMISRTPEVTCVVVDVSHEDPEVKFNWYVDGVEVHNAKTK PREEQYNSTYRVVSVLTVLHQDWLNGKEYKCKVSNKALGAPIEK TISKAKGQPREPQVYTLPPCRDELTKNQVSLWCLVKGFYPSDIAVE WESNGQPENNYKTTPPVLDSDGSFFLYSALTVDKSRWQQGNVFS CSVMHEALHNHYTQKSLSLSPGKGGGG**SAPTSSSTKKTQLQLEHLLLDLQMILNGINNYKNPKLTLMLTAKFYMPKKATELKHLQ CLEEELKPLEEVLNLAQSKNFHLRPRDLISNINVIVLELKGSET TFMCEYADETATIVEFLNRWITFCQSIISTLT** (SEQ ID NO:111, wherein the underlined sequence represents MMP enzymatic cleavage site, and the sequence in bold is mutIL2.) |

**Anti-CLDN-Fc(Knob)-IL10**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSDKTHTCPPCPAPEAAGGPSVFLFPPK PKDTLMISRTPEVTCVVVDVSHEDPEVKFNWYVDGVEVHNAKTKPREEQYNSTYRVVSVLTVLHQD WLNGKEYKCKVSNKALGAPIEKTISKAKGQPREPQVCTLPPSRDELTKNQVSLSCAVKGFYPSDIAVE WESNGQPENNYKTTPPVLDSDGSFKLVSKLTVDKSRWQQGNVFSCSVMHEALHNHYTQKSLSLSPGK TGGGGGSSPGQGTQSENSCTHFPGNLPNMLRDLRDAFSRVKTFFQMKDQLDNLLLKESLLEDFKGYL GCQALSEMIQFYLEEVMPQAENQDPDIKAHVNSLGENLKTLRLRLRRCHRFLPCENKSKAVEQVKNA FNKLQEKGIYKAMSEFDIFINYIEAYMTMKIRN (SEQ ID NO:112, wherein the underlined sequence represents the sequence of IL10.)
**Anti-CLDN-MMP(Double)-Anti-CD3-Fc(Hole)-IL10**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSSGRSENIRTASGRSPAIFTAMDIQMT QSPSSLSASVGDRVTITCRASQDIRNYLNWYQQKPGKAPKLLIYYTSRLESGVPSRFSGSGSGTDYTLTI SSLQPEDFATYYCQQGNTLPWTFGQGTKVEIKGGGGSGGGGSGGGGSEVQLVESGGGLVQPGGSLRL SCAASGYSFTGYTMNWVRQAPGKGLEWVALINPYKGVSTYNQKFKDRFTISVDKSKNTAYLQMNSL RAEDTAVYYCARSGYYGDSDWYFDVWGQGTLVTVSSDKTHTCPPCPAPEAAGGPSVFLFPPKPKDTL MISRTPEVTCVVVDVSHEDPEVKFNWYVDGVEVHNAKTKPREEQYNSTYRVVSVLTVLHQDWLNGK EYKCKVSNKALGAPIEKTISKAKGQPREPQVYTLPPCRDELTKNQVSLWCLVKGFYPSDIAVEWESNG QPENNYKTTPPVLDSDGSFFLYSALTVDKSRWQQGNVFSCSVMHEALHNHYTQKSLSLSPGKTGGGG GSSPGQGTQSENSCTHFPGNLPNMLRDLRDAFSRVKTFFQMKDQLDNLLLKESLLEDFKGYLGCQAL SEMIQFYLEEVMPQAENQDPDIKAHVNSLGENLKTLRLRLRRCHRFLPCENKSKAVEQVKNAFNKLQ EKGIYKAMSEFDIFINYIEAYMTMKIRN (SEQ ID NO:113, wherein the underlined sequence represents the sequence of IL10.)
**Anti-CLDN-MMP-Anti-CD3-Fc(Hole)-IL10**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSSGRSENIRTAMDIQMTQSPSSLSASV GDRVTITCRASQDIRNYLNWYQQKPGKAPKLLIYYTSRLESGVPSRFSGSGSGTDYTLTISSLQPEDFAT YYCQQGNTLPWTFGQGTKVEIKGGGGSGGGGSGGGGSEVQLVESGGGLVQPGGSLRLSCAASGYSFT GYTMNWVRQAPGKGLEWVALINPYKGVSTYNQKFKDRFTISVDKSKNTAYLQMNSLRAEDTAVYYC ARSGYYGDSDWYFDVWGQGTLVTVSSDKTHTCPPCPAPEAAGGPSVFLFPPKPKDTLMISRTPEVTCV VVDVSHEDPEVKFNWYVDGVEVHNAKTKPREEQYNSTYRVVSVLTVLHQDWLNGKEYKCKVSNK ALGAPIEKTISKAKGQPREPQVYTLPPCRDELTKNQVSLWCLVKGFYPSDIAVEWESNGQPENNYKTT PPVLDSDGSFFLYSALTVDKSRWQQGNVFSCSVMHEALHNHYTQKSLSLSPGKTGGGGGSSPGQGTQ SENSCTHFPGNLPNMLRDLRDAFSRVKTFFQMKDQLDNLLLKESLLEDFKGYLGCQALSEMIQFYLEE VMPQAENQDPDIKAHVNSLGENLKTLRLRLRRCHRFLPCENKSKAVEQVKNAFNKLQEKGIYKAMSE FDIFINYIEAYMTMKIRN (SEQ ID NO:114, wherein the underlined sequence represents the sequence of IL10.)
**Anti-CLDN-G4S-Anti-CD3-Fc(Hole)-IL10**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSGGGGSGGGGSMDIQMTQSPSSLSAS VGDRVTITCRASQDIRNYLNWYQQKPGKAPKLLIYYTSRLESGVPSRFSGSGSGTDYTLTISSLQPEDF ATYYCQQGNTLPWTFGQGTKVEIKGGGGSGGGGSGGGGSEVQLVESGGGLVQPGGSLRLSCAASGY SFTGYTMNWVRQAPGKGLEWVALINPYKGVSTYNQKFKDRFTISVDKSKNTAYLQMNSLRAEDTAV YYCARSGYYGDSDWYFDVWGQGTLVTVSSDKTHTCPPCPAPEAAGGPSVFLFPPKPKDTLMISRTPE VTCVVVDVSHEDPEVKFNWYVDGVEVHNAKTKPREEQYNSTYRVVSVLTVLHQDWLNGKEYKCK VSNKALGAPIEKTISKAKGQPREPQVYTLPPCRDELTKNQVSLWCLVKGFYPSDIAVEWESNGQPENN YKTTPPVLDSDGSFFLYSALTVDKSRWQQGNVFSCSVMHEALHNHYTQKSLSLSPGKTGGGGGSSPG QGTQSENSCTHFPGNLPNMLRDLRDAFSRVKTFFQMKDQLDNLLLKESLLEDFKGYLGCQALSEMIQ FYLEEVMPQAENQDPDIKAHVNSLGENLKTLRLRLRRCHRFLPCENKSKAVEQVKNAFNKLQEKGIY KAMSEFDIFINYIEAYMTMKIRN (SEQ ID NO:115, wherein the underlined sequence represents the sequence of IL10.)
**IL10-Anti-CD3-Fc(Hole)**
SPGQGTQSENSCTHFPGNLPNMLRDLRDAFSRVKTFFQMKDQLDNLLLKESLLEDFKGYLGCQALSE MIQFYLEEVMPQAENQDPDIKAHVNSLGENLKTLRLRLRRCHRFLPCENKSKAVEQVKNAFNKLQEK GIYKAMSEFDIFINYIEAYMTMKIRNGGGSGGGSPGQGTQSENSCTHFPGNLPNMLRDLRDAFSRVKT FFQMKDQLDNLLLKESLLEDFKGYLGCQALSEMIQFYLEEVMPQAENQDPDIKAHVNSLGENLKTLR LRLRRCHRFLPCENKSKAVEQVKNAFNKLQEKGIYKAMSEFDIFINYIEAYMTMKIRNGGGGSMDIQ MTQSPSSLSASVGDRVTITCRASQDIRNYLNWYQQKPGKAPKLLIYYTSRLESGVPSRFSGSGSGTDYT LTISSLQPEDFATYYCQQGNTLPWTFGQGTKVEIKGGGGSGGGGSGGGGSEVQLVESGGGLVQPGGSL RLSCAASGYSFTGYTMNWVRQAPGKGLEWVALINPYKGVSTYNQKFKDRFTISVDKSKNTAYLQMN SLRAEDTAVYYCARSGYYGDSDWYFDVWGQGTLVTVSSDKTHTCPPCPAPEAAGGPSVFLFPPKPKD TLMISRTPEVTCVVVDVSHEDPEVKFNWYVDGVEVHNAKTKPREEQYNSTYRVVSVLTVLHQDWLN GKEYKCKVSNKALGAPIEKTISKAKGQPREPQVYTLPPCRDELTKNQVSLWCLVKGFYPSDIAVEWES NGQPENNYKTTPPVLDSDGSFFLYSALTVDKSRWQQGNVFSCSVMHEALHNHYTQKSLSLSPGK (SEQ ID NO:116, wherein the underlined sequence represents the sequence of IL10.)
**Anti-CLDN(VL-CL)-IL10**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGECGGGGSSPGQGTQSENSCTHFPGNLPNMLRDLRDAFSRVKTFFQMKDQLDNL LLKESLLEDFKGYLGCQALSEMIQFYLEEVMPQAENQDPDIKAHVNSLGENLKTLRLRLRRCHRFLPC ENKSKAVEQVKNAFNKLQEKGIYKAMSEFDIFINYIEAYMTMKIRNGGGGSSPGQGTQSENSCTHFPG NLPNMLRDLRDAFSRVKTFFQMKDQLDNLLLKESLLEDFKGYLGCQALSEMIQFYLEEVMPQAENQ DPDIKAHVNSLGENLKTLRLRLRRCHRFLPCENKSKAVEQVKNAFNKLQEKGIYKAMSEFDIFINYIEA YMTMKIRN (SEQ ID NO:117, wherein the underlined sequence represents the sequence of IL10.)
**Anti-CLDN(VH-CH)- MMP(Double)-Anti-CD3-Fc(Hole)-IL10**
QVQLQQPGAELVRPGASVKLSCKASGYTFTSYWINWVKQRPGQGLEWIGNIYPSDSYTNYNQKFKD KATLTVDKSSSTAYMQLSSPTSEDSAVYYCARVYYGNSFAYWGQGTTLTVSSASTKGPSVFPLAPSSKS TSGGTAALGCLVKDYFPEPVTVSWNSGALTSGVHTFPAVLQSSGLYSLSSVVTVPSSSLGTQTYICNVN HKPSNTKVDKRVSGRSENIRTASGRSPAIFTAMDIQMTQSPSSLSASVGDRVTITCRASQDIRNYLNWY QQKPGKAPKLLIYYTSRLESGVPSRFSGSGSGTDYTLTISSLQPEDFATYYCQQGNTLPWTFGQGTKVE IKGGGGSGGGGSGGGGSEVQLVESGGGLVQPGGSLRLSCAASGYSFTGYTMNWVRQAPGKGLEWVA LINPYKGVSTYNQKFKDRFTISVDKSKNTAYLQMNSLRAEDTAVYYCARSGYYGDSDWYFDVWGQG TLVTVSSDKTHTCPPCPAPEAAGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALGAPIEKTISKAKGQPREPQ VYTLPPCRDELTKNQVSLWCLVKGFYPSDIAVEWESNGQPENNYKTTPPVLDSDGSFFLYSALTVDKS RWQQGNVFSCSVMHEALHNHYTQKSLSLSPGKTGGGGGS*SPGQGTQSENSCTHFPGNLPNMLRDLRD AFSRVKTFFQMKDQLDNLLLKESLLEDFKGYLGCQALSEMIQFYLEEVMPQAENQDPDIKAHVNSLGENLK TLRLRLRRCHRFLPCENKSKAVEQVKNAFNKLQEKGIYKAMSEFDIFINYIEAYMTMKIRN* (SEQ ID NO:118, wherein the underlined sequence represents the sequence of anti-CLDN(VH-CH), and the sequence in italics represents the sequence of IL10.)
**Anti-CLDN(VH-CH)-MMP-Anti-CD3-Fc(Hole)-IL10**
*QVQLQQPGAELVRPGASVKLSCKASGYTFTSYWINWVKQRPGQGLEWIGNIYPSDSYTNYNQKFKDKATLTV DKSSSTAYMQLSSPTSEDSAVYYCARVYYGNSFAYWGQGTTLTVSSASTKGPSVFPLAPSSKSTSGGTAALGCLV KDYFPEPVTVSWNSGALTSGVHTFPAVLQSSGLYSLSSVVTVPSSSLGTQTYICNVNHKPSNTKVDKRV*SGRSE NIRTAMDIQMTQSPSSLSASVGDRVTITCRASQDIRNYLNWYQQKPGKAPKLLIYYTSRLESGVPSRFS GSGSGTDYTLTISSLQPEDFATYYCQQGNTLPWTFGQGTKVEIKGGGGSGGGGSGGGGSEVQLVESGG GLVQPGGSLRLSCAASGYSFTGYTMNWVRQAPGKGLEWVALINPYKGVSTYNQKFKDRFTISVDKSK NTAYLQMNSLRAEDTAVYYCARSGYYGDSDWYFDVWGQGTLVTVSSDKTHTCPPCPAPEAAGGPSV FLFPPKPKDTLMISRTPEVTCVVVDVSHEDPEVKFNWYVDGVEVHNAKTKPREEQYNSTYRVVSVLT VLHQDWLNGKEYKCKVSNKALGAPIEKTISKAKGQPREPQVYTLPPCRDELTKNQVSLWCLVKGFYP SDIAVEWESNGQPENNYKTTPPVLDSDGSFFLYSALTVDKSRWQQGNVFSCSVMHEALHNHYTQKSL SLSPGKTGGGGGSSPGQGTQSENSCTHFPGNLPNMLRDLRDAFSRVKTFFQMKDQLDNLLLKESLLE DFKGYLGCQALSEMIQFYLEEVMPQAENQDPDIKAHVNSLGENLKTLRLRLRRCHRFLPCENKSKAV EQVKNAFNKLQEKGIYKAMSEFDIFINYIEAYMTMKIRN (SEQ ID NO:119, wherein the underlined sequence represents the sequence of IL10, and the sequence in italics represents the sequence of anti-CLDN(VH-CH).)
**Anti-CLDN-Fc(Knob)-Pro-IL2**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSDKTHTCPPCPAPEAAGGPSVFLFPPK PKDTLMISRTPEVTCVVVDVSHEDPEVKFNWYVDGVEVHNAKTKPREEQYNSTYRVVSVLTVLHQD WLNGKEYKCKVSNKALGAPIEKTISKAKGQPREPQVCTLPPSRDELTKNQVSLSCAVKGFYPSDIAVE WESNGQPENNYKTTPPVLDSDGSFKLVSKLTVDKSRWQQGNVFSCSVMHEALHNHYTQKSLSLSPGK TGGGGGSELCDDDPPEIPHATFKAMAYKEGTMLNCECKRGFRRIKSGSLYMLCTGNSSHSSWDNQCQ CTSSATRNTTKQVTPQPEEQKERKTTEMQSPMQPVDQASLPGHCREPPPWENEATERIYHFVVGQMV YYQCVQGYRALHRGPAESVCKMTHGKTRWTQPQLICTGEMETSQFPGEEKPQASPEGRPESETSCLV TTTDFQIQTEMAATMETSGGGGSGGGGSSGARYRWLTAGGGGSGGGGSAPTSSSTKKTQLQLEHLLL DLQMILNGINNYKNPKLTRMLTFKFYMPKKATELKHLQCLEEELKPLEEVLNLAQSKNFHLRPRDLIS NINVIVLELKGSETTFMCEYADETATIVEFLNRWITFCQSIISTLT (SEQ ID NO:120, wherein the underlined sequence represents the sequence of Pro-IL-2.)
**Anti-CLDN-Fc(Knob)-mutIL2**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSDKTHTCPPCPAPEAAGGPSVFLFPPK PKDTLMISRTPEVTCVVVDVSHEDPEVKFNWYVDGVEVHNAKTKPREEQYNSTYRVVSVLTVLHQD WLNGKEYKCKVSNKALGAPIEKTISKAKGQPREPQVCTLPPSRDELTKNQVSLSCAVKGFYPSDIAVE WESNGQPENNYKTTPPVLDSDGSFKLVSKLTVDKSRWQQGNVFSCSVMHEALHNHYTQKSLSLSPGK TGGGGGSAPTSSSTKKTQLQLEHLLLDLQMILNGINNYKNPKLTLMLTAKFYMPKKATELKHLQCLEE ELKPLEEVLNLAQSKNFHLRPRDLISNINVIVLELKGSETTFMCEYADETATIVEFLNRWITFCQSIISTLT (SEQ ID NO:121, wherein the underlined sequence represents the sequence of mutIL2 with R38L/F42A.)
**Anti-CLDN-Fc(Knob)-mutIL2**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSDKTHTCPPCPAPEAAGGPSVFLFPPK PKDTLMISRTPEVTCVVVDVSHEDPEVKFNWYVDGVEVHNAKTKPREEQYNSTYRVVSVLTVLHQD WLNGKEYKCKVSNKALGAPIEKTISKAKGQPREPQVCTLPPSRDELTKNQVSLSCAVKGFYPSDIAVE WESNGQPENNYKTTPPVLDSDGSFKLVSKLTVDKSRWQQGNVFSCSVMHEALHNHYTQKSLSLSPGK TGGGGGSAPTSSSTKKTQLQLEHLLLDLQMILNGINNYKNPKLTEMLTFKFYMPKKATELKHLQCLEE ELKPLEEVLNLAQSKNFHLRPRDLISNINVIVLELKGSETTFMCEYADETATIVEFLNRWITFCQSIISTLT (SEQ ID NO:122, wherein the underlined sequence represents the sequence of mutIL2 with R38E.)
**Anti-CLDN-Fc(Knob)-mutIL2**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSDKTHTCPPCPAPEAAGGPSVFLFPPK PKDTLMISRTPEVTCVVVDVSHEDPEVKFNWYVDGVEVHNAKTKPREEQYNSTYRVVSVLTVLHQD WLNGKEYKCKVSNKALGAPIEKTISKAKGQPREPQVCTLPPSRDELTKNQVSLSCAVKGFYPSDIAVE WESNGQPENNYKTTPPVLDSDGSFKLVSKLTVDKSRWQQGNVFSCSVMHEALHNHYTQKSLSLSPGK TGGGGGSAPTSSSTKKTQLQLEHLLLDLQMILNGINNYKNPKLTEMLTFKFYMPKKATELKHLQCLEE ELKPLEEVLNLAQSKNFHLRPRDLISNINVIVLELKGSETTFMCEYADETATIVEFLNRWITFCQSIISTLT (SEQ ID NO:123, wherein the underlined sequence represents the sequence of mutIL2 with K35E.)
**Anti-CLDN-Fc(Knob)-mutIL2**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSDKTHTCPPCPAPEAAGGPSVFLFPPK PKDTLMISRTPEVTCVVVDVSHEDPEVKFNWYVDGVEVHNAKTKPREEQYNSTYRVVSVLTVLHQD WLNGKEYKCKVSNKALGAPIEKTISKAKGQPREPQVCTLPPSRDELTKNQVSLSCAVKGFYPSDIAVE WESNGQPENNYKTTPPVLDSDGSFKLVSKLTVDKSRWQQGNVFSCSVMHEALHNHYTQKSLSLSPGK TGGGGGSAPTSSSTKKTQLQLEHLLLDLQMILNGINNYKNPELTEMLTFKFYMPKKATELKHLQCLEE ELKPLEEVLNLAQSKNFHLRPRDLISNINVIVLELKGSETTFMCEYADETATIVEFLNRWITFCQSIISTLT (SEQ ID NO:124, wherein the underlined sequence represents the sequence of mutIL2 with K35E/R38E.)
**Anti-CLDN-Fc(Knob)-mutIL2**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSDKTHTCPPCPAPEAAGGPSVFLFPPK PKDTLMISRTPEVTCVVVDVSHEDPEVKFNWYVDGVEVHNAKTKPREEQYNSTYRVVSVLTVLHQD WLNGKEYKCKVSNKALGAPIEKTISKAKGQPREPQVCTLPPSRDELTKNQVSLSCAVKGFYPSDIAVE WESNGQPENNYKTTPPVLDSDGSFKLVSKLTVDKSRWQQGNVFSCSVMHEALHNHYTQKSLSLSPGK TGGGGGSAPTSSSTKKTQLQLEHLLLDLQMILNGINNYKNPALTEMLTFKFYMPKKATELKHLQCLEE ELKPLEEVLNLAQSKNFHLRPRDLISNINVIVLELKGSETTFMCEYADETATIVEFLNRWITFCQSIISTLT (SEQ ID NO:125, wherein the underlined sequence represents the sequence of mutIL2 with K35A/R38E.)
**Anti-CLDN-MMP(Double)-Anti-CD3-Fc(Hole)-mutIL2**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSSGRSENIRTASGRSPAIFTAMDIQMT QSPSSLSASVGDRVTITCRASQDIRNYLNWYQQKPGKAPKLLIYYTSRLESGVPSRFSGSGSGTDYTLTI SSLQPEDFATYYCQQGNTLPWTFGQGTKVEIKGGGGSGGGGSGGGGSEVQLVESGGGLVQPGGSLRL SCAASGYSFTGYTMNWVRQAPGKGLEWVALINPYKGVSTYNQKFKDRFTISVDKSKNTAYLQMNSL RAEDTAVYYCARSGYYGDSDWYFDVWGQGTLVTVSSDKTHTCPPCPAPEAAGGPSVFLFPPKPKDTL MISRTPEVTCVVVDVSHEDPEVKFNWYVDGVEVHNAKTKPREEQYNSTYRVVSVLTVLHQDWLNGK EYKCKVSNKALGAPIEKTISKAKGQPREPQVYTLPPCRDELTKNQVSLWCLVKGFYPSDIAVEWESNG QPENNYKTTPPVLDSDGSFFLYSALTVDKSRWQQGNVFSCSVMHEALHNHYTQKSLSLSPGKTGGGG GSAPTSSSTKKTQLQLEHLLLDLQMILNGINNYKNPELTEMLTFKFYMPKKATELKHLQCLEEELKPLE EVLNLAQSKNFHLRPRDLISNINVIVLELKGSETTFMCEYADETATIVEFLNRWITFCQSIISTLT (SEQ ID NO:126, wherein the underlined sequence represents the sequence of mutIL2 with K35A/R38E.)
**Anti-CLDN-MMP-Anti-CD3-Fc(Hole)-mutIL2**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSSGRSENIRTAMDIQMTQSPSSLSASV GDRVTITCRASQDIRNYLNWYQQKPGKAPKLLIYYTSRLESGVPSRFSGSGSGTDYTLTISSLQPEDFAT YYCQQGNTLPWTFGQGTKVEIKGGGGSGGGGSGGGGSEVQLVESGGGLVQPGGSLRLSCAASGYSFT GYTMNWVRQAPGKGLEWVALINPYKGVSTYNQKFKDRFTISVDKSKNTAYLQMNSLRAEDTAVYYC ARSGYYGDSDWYFDVWGQGTLVTVSSDKTHTCPPCPAPEAAGGPSVFLFPPKPKDTLMISRTPEVTCV VVDVSHEDPEVKFNWYVDGVEVHNAKTKPREEQYNSTYRVVSVLTVLHQDWLNGKEYKCKVSNK ALGAPIEKTISKAKGQPREPQVYTLPPCRDELTKNQVSLWCLVKGFYPSDIAVEWESNGQPENNYKTT PPVLDSDGSFFLYSALTVDKSRWQQGNVFSCSVMHEALHNHYTQKSLSLSPGKTGGGGGSAPTSSSTK KTQLQLEHLLLDLQMILNGINNYKNPELTEMLTFKFYMPKKATELKHLQCLEEELKPLEEVLNLAQSK NFHLRPRDLISNINVIVLELKGSETTFMCEYADETATIVEFLNRWITFCQSIISTLT (SEQ ID NO:127, wherein the underlined sequence represents the sequence of mutIL2 with K35E/R38E.)
**Anti-CLDN-G4S-Anti-CD3-Fc(Hole)-mutIL2**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSGGGGSGGGGSMDIQMTQSPSSLSAS VGDRVTITCRASQDIRNYLNWYQQKPGKAPKLLIYYTSRLESGVPSRFSGSGSGTDYTLTISSLQPEDF ATYYCQQGNTLPWTFGQGTKVEIKGGGGSGGGGSGGGGSEVQLVESGGGLVQPGGSLRLSCAASGY SFTGYTMNWVRQAPGKGLEWVALINPYKGVSTYNQKFKDRFTISVDKSKNTAYLQMNSLRAEDTAV YYCARSGYYGDSDWYFDVWGQGTLVTVSSDKTHTCPPCPAPEAAGGPSVFLFPPKPKDTLMISRTPE VTCVVVDVSHEDPEVKFNWYVDGVEVHNAKTKPREEQYNSTYRVVSVLTVLHQDWLNGKEYKCK VSNKALGAPIEKTISKAKGQPREPQVYTLPPCRDELTKNQVSLWCLVKGFYPSDIAVEWESNGQPENN YKTTPPVLDSDGSFFLYSALTVDKSRWQQGNVFSCSVMHEALHNHYTQKSLSLSPGKTGGGGGSAPT SSSTKKTQLQLEHLLLDLQMILNGINNYKNPELTEMLTFKFYMPKKATELKHLQCLEEELKPLEEVLNL AQSKNFHLRPRDLISNINVIVLELKGSETTFMCEYADETATIVEFLNRWITFCQSIISTLT (SEQ ID NO:128, wherein the underlined sequence represents the sequence of mutIL2 with K35E/R38E.)
**Anti-CLDN-Fc(Knob)-IL21**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSDKTHTCPPCPAPEAAGGPSVFLFPPK PKDTLMISRTPEVTCVVVDVSHEDPEVKFNWYVDGVEVHNAKTKPREEQYNSTYRVVSVLTVLHQD WLNGKEYKCKVSNKALGAPIEKTISKAKGQPREPQVCTLPPSRDELTKNQVSLSCAVKGFYPSDIAVE WESNGQPENNYKTTPPVLDSDGSFKLVSKLTVDKSRWQQGNVFSCSVMHEALHNHYTQKSLSLSPGK TGGGGGSKSSSQGQDRHMIRMRQLIDIVDQLKNYVNDLVPEFLPAPEDVETNCEWSAFSCFQKAQLK SANTGNNERIINVSIKKLKRKPPSTNAGRRQKHRLTCPSCDSYEKKPPKEFLERFKSLLQKMIHQHLSS RTHGSEDS (SEQ ID NO:129, wherein the underlined sequence represents the sequence of IL21.)
**Anti-CLDN(VL-CL)-IL21**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGECGGGGGSGGGGSKSSSQGQDRHMIRMRQLIDIVDQLKNYVNDLVPEFLPAPED VETNCEWSAFSCFQKAQLKSANTGNNERIINVSIKKLKRKPPSTNAGRRQKHRLTCPSCDSYEKKPPK EFLERFKSLLQKMIHQHLSSRTHGSEDS (SEQ ID NO:130, wherein the underlined sequence represents the sequence of IL21.)
**Anti-CLDN-MMP(Double)-Anti-CD3-Fc(Hole)-IL21**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSSGRSENIRTASGRSPAIFTAMDIQMT QSPSSLSASVGDRVTITCRASQDIRNYLNWYQQKPGKAPKLLIYYTSRLESGVPSRFSGSGSGTDYTLTI SSLQPEDFATYYCQQGNTLPWTFGQGTKVEIKGGGGSGGGGSGGGGSEVQLVESGGGLVQPGGSLRL SCAASGYSFTGYTMNWVRQAPGKGLEWVALINPYKGVSTYNQKFKDRFTISVDKSKNTAYLQMNSL RAEDTAVYYCARSGYYGDSDWYFDVWGQGTLVTVSSDKTHTCPPCPAPEAAGGPSVFLFPPKPKDTL MISRTPEVTCVVVDVSHEDPEVKFNWYVDGVEVHNAKTKPREEQYNSTYRVVSVLTVLHQDWLNGK EYKCKVSNKALGAPIEKTISKAKGQPREPQVYTLPPCRDELTKNQVSLWCLVKGFYPSDIAVEWESNG QPENNYKTTPPVLDSDGSFFLYSALTVDKSRWQQGNVFSCSVMHEALHNHYTQKSLSLSPGKTGGGG GSKSSSQGQDRHMIRMRQLIDIVDQLKNYVNDLVPEFLPAPEDVETNCEWSAFSCFQKAQLKSANTG NNERIINVSIKKLKRKPPSTNAGRRQKHRLTCPSCDSYEKKPPKEFLERFKSLLQKMIHQHLSSRTHGSE DS (SEQ ID NO:131, wherein the underlined sequence represents the sequence of IL21.)
**Anti-CLDN-MMP-Anti-CD3-Fc(Hole)-IL21**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSSGRSENIRTAMDIQMTQSPSSLSASV GDRVTITCRASQDIRNYLNWYQQKPGKAPKLLIYYTSRLESGVPSRFSGSGSGTDYTLTISSLQPEDFAT YYCQQGNTLPWTFGQGTKVEIKGGGGSGGGGSGGGGSEVQLVESGGGLVQPGGSLRLSCAASGYSFT GYTMNWVRQAPGKGLEWVALINPYKGVSTYNQKFKDRFTISVDKSKNTAYLQMNSLRAEDTAVYYC ARSGYYGDSDWYFDVWGQGTLVTVSSDKTHTCPPCPAPEAAGGPSVFLFPPKPKDTLMISRTPEVTCV VVDVSHEDPEVKFNWYVDGVEVHNAKTKPREEQYNSTYRVVSVLTVLHQDWLNGKEYKCKVSNK ALGAPIEKTISKAKGQPREPQVYTLPPCRDELTKNQVSLWCLVKGFYPSDIAVEWESNGQPENNYKTT PPVLDSDGSFFLYSALTVDKSRWQQGNVFSCSVMHEALHNHYTQKSLSLSPGKTGGGGGSKSSSQGQ DRHMIRMRQLIDIVDQLKNYVNDLVPEFLPAPEDVETNCEWSAFSCFQKAQLKSANTGNNERIINVSIK KLKRKPPSTNAGRRQKHRLTCPSCDSYEKKPPKEFLERFKSLLQKMIHQHLSSRTHGSEDS (SEQ ID NO:132, wherein the underlined sequence represents the sequence of IL21.)
**Mouse IL15 Ra complex:**
**NWIDVRYDLEKIESLIQSIHIDTTLYTDSDFHPSCKVTAMNCFLLELQVILHEYSNMTLNETVRNV LYLANSTLSSNKNVAESGCKECEELEEKTFTEFLQSFIRIVQMFINTS**GGGGSGGGGSGGGGS*GTTC PPPVSIEHADIRVKNYSVNSRERYVCNSGFKRKAGTSTLIECVINKNTNVAHWTTPSLKCIRDPSLAHYSPVPT* (SEQ ID NO:133**,** wherein the sequence in bold is the sequence of IL15, and the sequence in italics is the sequence of the IL15 receptor.)
**CLDN-MMP(double)-CD3-Fc(Hole)-mouse IL15 Ra**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSS**SGRSENIRTASGRSPAIFTA**MDIQM TQSPSSLSASVGDRVTITCRASQDIRNYLNWYQQKPGKAPKLLIYYTSRLESGVPSRFSGSGSGTDYTL TISSLQPEDFATYYCQQGNTLPWTFGQGTKVEIKGGGGSGGGGSGGGGSEVQLVESGGGLVQPGGSLR LSCAASGYSFTGYTMNWVRQAPGKGLEWVALINPYKGVSTYNQKFKDRFTISVDKSKNTAYLQMNS LRAEDTAVYYCARSGYYGDSDWYFDVWGQGTLVTVSSDKTHTCPPCPAPEAAGGPSVFLFPPKPKDT LMISRTPEVTCVVVDVSHEDPEVKFNWYVDGVEVHNAKTKPREEQYNSTYRVVSVLTVLHQDWLNG KEYKCKVSNKALGAPIEKTISKAKGQPREPQVYTLPPCRDELTKNQVSLWCLVKGFYPSDIAVEWESN GQPENNYKTTPPVLDSDGSFFLYSALTVDKSRWQQGNVFSCSVMHEALHNHYTQKSLSLSPGKGGGG S**NWIDVRYDLEKIESLIQSIHIDTTLYTDSDFHPSCKVTAMNCFLLELQVILHEYSNMTLNETVRN VLYLANSTLSSNKNVAESGCKECEELEEKTFTEFLQSFIRIVQMFINTS**GGGGSGGGGSGGGGS**GT TCPPPVSIEHADIRVKNYSVNSRERYVCNSGFKRKAGTSTLIECVINKNTNVAHWTTPSLKCIRDP SLAHYSPVPT (**SEQ ID NO:134, wherein the sequence in bold and underlined represents the MMP site, the sequence in bold is the sequence of IL15, and the sequence in italics is the sequence of the IL15 receptor.)
**Mouse IL15:** 
NWIDVRYDLEKIESLIQSIHIDTTLYTDSDFHPSCKVTAMNCFLLELQVILHEYSNMTLNETVRNVLYL
ANSTLSSNKNVAESGCKECEELEEKTFTEFLQSFIRIVQMFINT (SEQ ID NO:135)
**Mouse IL15 receptor:** 
GTTCPPPVSIEHADIRVKNYSVNSRERYVCNSGFKRKAGTSTLIECVINKNTNVAHWTTPSLKCIRDPSL
AHYSPVPT (SEQ ID NO:136)
**IL-15 human:** 
NWVNVISDLKKIEDLIQSMHIDATLYTESDVHPSCKVTAMKCFLLELQVISLESGDASIHDTVENLIILA
NNSLSSNGNVTESGCKECEELEEKNIKEFLQSFVHIVQMFINTS (SEQ ID NO:137)
**human IL15 receptor:** 
LQITCPPPMSVEHADIWVKSYSLYSRERYICNSGFKRKAGTSSLTECVLNKATNVAHWTTPSLKCIRDP
ALVHQRPAPP (SEQ ID NO:138)
**CLDN-MMP(double)-CD3-Fc(Hole)-human IL15 Ra**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSSGRSENIRTASGRSPAIFTAMDIQMT QSPSSLSASVGDRVTITCRASQDIRNYLNWYQQKPGKAPKLLIYYTSRLESGVPSRFSGSGSGTDYTLTI SSLQPEDFATYYCQQGNTLPWTFGQGTKVEIKGGGGSGGGGSGGGGSEVQLVESGGGLVQPGGSLRL SCAASGYSFTGYTMNWVRQAPGKGLEWVALINPYKGVSTYNQKFKDRFTISVDKSKNTAYLQMNSL RAEDTAVYYCARSGYYGDSDWYFDVWGQGTLVTVSSDKTHTCPPCPAPEAAGGPSVFLFPPKPKDTL MISRTPEVTCVVVDVSHEDPEVKFNWYVDGVEVHNAKTKPREEQYNSTYRVVSVLTVLHQDWLNGK EYKCKVSNKALGAPIEKTISKAKGQPREPQVYTLPPCRDELTKNQVSLWCLVKGFYPSDIAVEWESNG QPENNYKTTPPVLDSDGSFFLYSALTVDKSRWQQGNVFSCSVMHEALHNHYTQKSLSLSPGKGGGGS **NWVNVISDLKKIEDLIQSMHIDATLYTESDVHPSCKVTAMKCFLLELQVISLESGDASIHDTVENL IILANNSLSSNGNVTESGCKECEELEEKNIKEFLQSFVHIVQMFINTS**GGGGSGGGGSGGGGS*LQIT CPPPMSVEHADIWVKSYSLYSRERYICNSGFKRKAGTSSLTECVLNKATNVAHWTTPSLKCIRDPALVHQRPAP P* (SEQ ID NO:139, wherein the sequence in bold is the sequence of IL15, and the sequence in italics is the sequence of the IL15 receptor.)
**CLDN-MMP(double)-CD3-Fc(Hole)-human IL15**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSSGRSENIRTASGRSPAIFTAMDIQMT QSPSSLSASVGDRVTITCRASQDIRNYLNWYQQKPGKAPKLLIYYTSRLESGVPSRFSGSGSGTDYTLTI SSLQPEDFATYYCQQGNTLPWTFGQGTKVEIKGGGGSGGGGSGGGGSEVQLVESGGGLVQPGGSLRL SCAASGYSFTGYTMNWVRQAPGKGLEWVALINPYKGVSTYNQKFKDRFTISVDKSKNTAYLQMNSL RAEDTAVYYCARSGYYGDSDWYFDVWGQGTLVTVSSDKTHTCPPCPAPEAAGGPSVFLFPPKPKDTL MISRTPEVTCVVVDVSHEDPEVKFNWYVDGVEVHNAKTKPREEQYNSTYRVVSVLTVLHQDWLNGK EYKCKVSNKALGAPIEKTISKAKGQPREPQVYTLPPCRDELTKNQVSLWCLVKGFYPSDIAVEWESNG QPENNYKTTPPVLDSDGSFFLYSALTVDKSRWQQGNVFSCSVMHEALHNHYTQKSLSLSPGKGGGGS **NWVNVISDLKKIEDLIQSMHIDATLYTESDVHPSCKVTAMKCFLLELQVISLESGDASIHDTVENL IILANNSLSSNGNVTESGCKECEELEEKNIKEFLQSFVHIVQMFINTS** (SEQ ID NO: 140, wherein the sequence in bold is the sequence of IL 15.)
**Anti-CLDN-Fc(Knob)-IL15 Ra**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSDKTHTCPPCPAPEAAGGPSVFLFPPK PKDTLMISRTPEVTCVVVDVSHEDPEVKFNWYVDGVEVHNAKTKPREEQYNSTYRVVSVLTVLHQD WLNGKEYKCKVSNKALGAPIEKTISKAKGQPREPQVCTLPPSRDELTKNQVSLSCAVKGFYPSDIAVE WESNGQPENNYKTTPPVLDSDGSFKLVSKLTVDKSRWQQGNVFSCSVMHEALHNHYTQKSLSLSPGK **TGGGGGSNWVNVISDLKKIEDLIQSMHIDATLYTESDVHPSCKVTAMKCFLLELQVISLESGDASI HDTVENLIILANNSLSSNGNVTESGCKECEELEEKNIKEFLQSFVHIVQMFINTSGGGGSGGGGSG** *GGGSLQITCPPPMSVEHADIWVKSYSLYSRERYICNSGFKRKAGTSSLTECVLNKATNVAHWTTPSLKCIRDPA LVHQRPAPP* (SEQ ID NO:141, wherein the sequence in bold is the sequence of IL15, and the sequence in italics is the sequence of the IL15 receptor.)
**Anti-CLDN-Fc(Knob)-IL15**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKGGGGSGGGGSGGGGSQVQLQQPG AELVRPGASVKLSCKASGYTFTSYWINWVKQRPGQGLEWIGNIYPSDSYTNYNQKFKDKATLTVDKS SSTAYMQLSSPTSEDSAVYYCARVYYGNSFAYWGQGTTLTVSSDKTHTCPPCPAPEAAGGPSVFLFPPK PKDTLMISRTPEVTCVVVDVSHEDPEVKFNWYVDGVEVHNAKTKPREEQYNSTYRVVSVLTVLHQD WLNGKEYKCKVSNKALGAPIEKTISKAKGQPREPQVCTLPPSRDELTKNQVSLSCAVKGFYPSDIAVE WESNGQPENNYKTTPPVLDSDGSFKLVSKLTVDKSRWQQGNVFSCSVMHEALHNHYTQKSLSLSPGK **TGGGGGSNWVNVISDLKKIEDLIQSMHIDATLYTESDVHPSCKVTAMKCFLLELQVISLESGDASI HDTVENLIILANNSLSSNGNVTESGCKECEELEEKNIKEFLQSFVHIVQMFINTS** (SEQ ID NO: 142, wherein the sequence in bold is the sequence of IL15.)
**IL15-Anti-CD3-Fc(Hole)**
**NWVNVISDLKKIEDLIQSMHIDATLYTESDVHPSCKVTAMKCFLLELQVISLESGDASIHDTVENL IILANNSLSSNGNVTESGCKECEELEEKNIKEFLQSFVHIVQMFINTS**GGGGSMDIQMTQSPSSLSA SVGDRVTITCRASQDIRNYLNWYQQKPGKAPKLLIYYTSRLESGVPSRFSGSGSGTDYTLTISSLQPEDF ATYYCQQGNTLPWTFGQGTKVEIKGGGGSGGGGSGGGGSEVQLVESGGGLVQPGGSLRLSCAASGY SFTGYTMNWVRQAPGKGLEWVALINPYKGVSTYNQKFKDRFTISVDKSKNTAYLQMNSLRAEDTAV YYCARSGYYGDSDWYFDVWGQGTLVTVSSDKTHTCPPCPAPEAAGGPSVFLFPPKPKDTLMISRTPE VTCVVVDVSHEDPEVKFNWYVDGVEVHNAKTKPREEQYNSTYRVVSVLTVLHQDWLNGKEYKCK VSNKALGAPIEKTISKAKGQPREPQVYTLPPCRDELTKNQVSLWCLVKGFYPSDIAVEWESNGQPENN YKTTPPVLDSDGSFFLYSALTVDKSRWQQGNVFSCSVMHEALHNHYTQKSLSLSPGK (SEQ ID NO:143, wherein the sequence in bold is the sequence of IL15.)
**Anti-CLDN(VL-CL)-IL15**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGECGGGGG**SNWVNVISDLKKIEDLIQSMHIDATLYTESDVHPSCKVTAMKCFL LELQVISLESGDASIHDTVENLIILANNSLSSNGNVTESGCKECEELEEKNIKEFLQSFVHIVQMFI NTS** (SEQ ID NO:144, wherein the sequence in bold is the sequence of IL15.)
**Anti-CLDN(VL-CL)-IL15 Ra**
DIVMTQSPSSLTVTAGEKVTMSCKSSQSLLNSGNQKNYLTWYQQKPGQPPKLLIYWASTRESGVPDRF TGSGSGTDFTLTISSVQAEDLAVYYCQNDYFYPFTFGSGTKLEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGECGGGGG**SNWVNVISDLKKIEDLIQSMHIDATLYTESDVHPSCKVTAMKCFL LELQVISLESGDASIHDTVENLIILANNSLSSNGNVTESGCKECEELEEKNIKEFLQSFVHIVQMFI NTS**GGGGSGGGGSGGGG*SLQITCPPPMSVEHADIWVKSYSLYSRERYICNSGFKRKAGTSSLTECVLNKATN VAHWTTPSLKCIRDPALVHQRPAPP* (SEQ ID NO:145, wherein the sequence in bold is the sequence of IL15, and the sequence in italics is the sequence of the IL15 receptor.)
**The sequence of the human IL-15Rα complex** 
NWVNVISDLKKIEDLIQSMHIDATLYTESDVHPSCKVTAMKCFLLELQVISLESGDASIHDTVENLIILA
NNSLSSNGNVTESGCKECEELEEKNIKEFLQSFVHIVQMFINTSGGGGSGGGGSGGGGSLQITCPPPMS
VEHADIWVKSYSLYSRERYICNSGFKRKAGTSSLTECVLNKATNVAHWTTPSLKCIRDPALVHQRPAPP
(SEQ ID NO:146)
**IL-15 human mutant E87C:** 
NWVNVISDLKKIEDLIQSMHIDATLYTESDVHPSCKVTAMKCFLLELQVISLESGDASIHDTVENLIILA
NNSLSSNGNVTESGCKCCEELEEKNIKEFLQSFVHIVQMFINTS (SEQ ID NO:147)
**IL-15 human mutant L45C:** 
NWVNVISDLKKIEDLIQSMHIDATLYTESDVHPSCKVTAMKCFLCELQVISLESGDASIHDTVENLIILA
NNSLSSNGNVTESGCKCCEELEEKNIKEFLQSFVHIVQMFINTS (SEQ ID NO:148)
**Cleavable linkers:**
MMP 10AA:GSWLPSSITA (SEQ ID NO:149)
MMP 8AA:WLPSSITA (SEQ ID NO:150)
MMP 7AA: LPSSITA (SEQ ID NO:151)
MMP 6AA:WLPSSI (SEQ ID NO:152)
MMP 6AA(TA): PSSITA (SEQ ID NO:153)
MMP 5AA: PSSIT (SEQ ID NO:154)
LSGKD (SEQ ID NO:155)

**MLSN-Fc (Knob)** 
DIVMTQSPSSLTVTAGEKVTMSCKSSQSLLNSGNQKNYLTWYQQKPGQPPKLLIYWASTRESGVPDRF
TGSGSGTDFTLTISSVQAEDLAVYYCQNDYFYPFTFGSGTKLEIKGGGSGGGSGGGSQVQLQQPGAEL
VRPGASVKLSCKASGYTFTSYWINWVKQRPGQGLEWIGNIYPSDSYTNYNQKFKDKATLTVDKSSST
AYMQLSSPTSEDSAVYYCARVYYGNSFAYWGQGTTLTVSSEPKSCDKTHTCPPCPAPEAAGGPSVFLF
PPKPKDTLMISRTPEVTCVVVDVSHEDPEVKFNWYVDGVEVHNAKTKPREEQYNSTYRVVSVLTVLH
QDWLNGKEYKCKVSNKALGAPIEKTISKAKGQPREPQVCTLPPSRDELTKNQVSLSCAVKGFYPSDIA
VEWESNGQPENNYKTTPPVLDSDGSFKLVSKLTVDKSRWQQGNVFSCSVMHEALHNHYTQKSLSLSP
GK (SEQ ID NO:156)
**Anti-CD3(SP34-scFv)-Fc (Hole)** 
ELVVTQEPSLTVSPGGTVTLTCRSSNGAVTSSNYANWVQQKPGQAPRGLIGGTNKRAPGTPARFSGSLL
GGKAALTLSGVQPEDEAVYYCALWYPNLWVFGGGTKLTVLGGGSGGGSGGGSEVQLLESGGGIVQP
GGSLKLSCAASGFTFNTYAMNWVRQAPGKGLEWVARIRSKYNNYATYYADSVKDRFTISRDDSKNTV
YLQMNNLKTEDTAVYYCVRHENFGNSYVSWFAHWGQGTLVTVSSEPKSCDKTHTCPPCPAPEAAGG
PSVFLFPPKPKDTLMISRTPEVTCVVVDVSHEDPEVKFNWYVDGVEVHNAKTKPREEQYNSTYRVVS
VLTVLHQDWLNGKEYKCKVSNKALGAPIEKTISKAKGQPREPQVYTLPPCRDELTKNQVSLWCLVKG
FYPSDIAVEWESNGQPENNYKTTPPVLDSDGSFFLYSALTVDKSRWQQGNVFSCSVMHEALHNHYTQ
KSLSLSPGK (SEQ ID NO:157)
**Anti-CD3(SP34-VH-CH)-Fc (Hole)** 
EVQLLESGGGIVQPGGSLKLSCAASGFTFNTYAMNWVRQAPGKGLEWVARIRSKYNNYATYYADSVK
DRFTISRDDSKNTVYLQMNNLKTEDTAVYYCVRHENFGNSYVSWFAHWGQGTLVTVSSRSASTKGPS
VFPLAPSSKSTSGGTAALGCLVKDYFPEPVTVSWNSGALTSGVHTFPAVLQSSGLYSLSSVVTVPSSSLG
TQTYICNVNHKPSNTKVDKRVEPKSCDKTHTCPPCPAPEAAGGPSVFLFPPKPKDTLMISRTPEVTCVV
VDVSHEDPEVKFNWYVDGVEVHNAKTKPREEQYNSTYRVVSVLTVLHQDWLNGKEYKCKVSNKAL
GAPIEKTISKAKGQPREPQVYTLPPCRDELTKNQVSLWCLVKGFYPSDIAVEWESNGQPENNYKTTPPV
LDSDGSFFLYSALTVDKSRWQQGNVFSCSVMHEALHNHYTQKSLSLSPGK (SEQ ID NO:158)
**Anti-CD3(SP34-VL-CL)** 
ELVVTQEPSLTVSPGGTVTLTCRSSNGAVTSSNYANWVQQKPGQAPRGLIGGTNKRAPGTPARFSGSLL
GGKAALTLSGVQPEDEAVYYCALWYPNLWVFGGGTKLTVLRSRTVAAPSVFIFPPSDEQLKSGTASVV
CLLNNFYPREAKVQWKVDNALQSGNSQESVTEQDSKDSTYSLSSTLTLSKADYEKHKVYACEVTHQ
GLSSPVTKSFNRGEC (SEQ ID NO:159)
**Anti-CD3(UCHT1-VH-CH)-Fc (Hole)** 
EVQLVESGGGLVQPGGSLRLSCAASGYSFTGYTMNWVRQAPGKGLEWVALINPYKGVSTYNQKFKD
RFTISVDKSKNTAYLQMNSLRAEDTAVYYCARSGYYGDSDWYFDVWGQGTLVTVSSRSASTKGPSVF
 
PLAPSSKSTSGGTAALGCLVKDYFPEPVTVSWNSGALTSGVHTFPAVLQSSGLYSLSSVVTVPSSSLGTQ
TYICNVNHKPSNTKVDKRVEPKSCDKTHTCPPCPAPEAAGGPSVFLFPPKPKDTLMISRTPEVTCVVVD
VSHEDPEVKFNWYVDGVEVHNAKTKPREEQYNSTYRVVSVLTVLHQDWLNGKEYKCKVSNKALGA
PIEKTISKAKGQPREPQVYTLPPCRDELTKNQVSLWCLVKGFYPSDIAVEWESNGQPENNYKTTPPVLD
SDGSFFLYSALTVDKSRWQQGNVFSCSVMHEALHNHYTQKSLSLSPGK (SEQ ID NO:160)
**Anti-CD3(UCHT1-VL-CL)** 
DIQMTQSPSSLSASVGDRVTITCRASQDIRNYLNWYQQKPGKAPKLLIYYTSRLESGVPSRFSGSGSGT
DYTLTISSLQPEDFATYYCQQGNTLPWTFGQGTKVEIKRSRTVAAPSVFIFPPSDEQLKSGTASVVCLLN
NFYPREAKVQWKVDNALQSGNSQESVTEQDSKDSTYSLSSTLTLSKADYEKHKVYACEVTHQGLSSP
VTKSFNRGEC (SEQ ID NO:161)
**Anti-CLDN (VL)** 
DIVMTQSPSSLTVTAGEKVTMSCKSSQSLLNSGNQKNYLTWYQQKPGQPPKLLIYWASTRESGVPDRF
TGSGSGTDFTLTISSVQAEDLAVYYCQNDYFYPFTFGSGTKLEIK (SEQ ID NO:162)
**Anti-CLDN (VH)** 
QVQLQQPGAELVRPGASVKLSCKASGYTFTSYWINWVKQRPGQGLEWIGNIYPSDSYTNYNQKFKD
KATLTVDKSSSTAYMQLSSPTSEDSAVYYCARVYYGNSFAYWGQGTTLTVSS (SEQ ID NO:163)
**Anti-MSLN (VL)** 
DIELTQSPAIMSASPGEKVTMTCSASSSVSYMHWYQQKSGTSPKRWIYDTSKLASGVPGRFSGSGSGN
SYSLTISSVEAEDDATYYCQQWSKHPLTFGSGTKVEIK (SEQ ID NO:164)
**Anti-MSLN (VH)** 
QVQLQQSGPELEKPGASVKISCKASGYSFTGYTMNWVKQSHGKSLEWIGLITPYNGASSYNQKFRGK
ATLTVDKSSSTAYMDLLSLTSEDSAVYFCARGGYDGRGFDYWGSGTPVTVSS (SEQ ID NO:165)
**IL2** 

 (SEQ ID
NO:166)
**IL2Ra** 
ELCDDDPPEIPHATFKAMAYKEGTMLNCECKRGFRRIKSGSLYMLCTGNSSHSSWDNQCQCTSSATRN
TTKQVTPQPEEQKERKTTEMQSPMQPVDQASLPGHCREPPPWENEATERIYHFVVGQMVYYQCVQG
YRALHRGPAESVCKMTHGKTRWTQPQLICTGEMETSQFPGEEKPQASPEGRPESETSCLVTTTDFQIQT
EMAATMETS (SEQ ID NO:167)
**IL2Rb** 
AVNGTSQFTCFYNSRANISCVWSQDGALQDTSCQVHAWPDRRRWNQTCELLPVSQASWACNLILGAP
DSQKLTTVDIVTLRVLCREGVRWRVMAIQDFKPFENLRLMAPISLQVVHVETHRCNISWEISQASHYF
ERHLEFEARTLSPGHTWEEAPLLTLKQKQEWICLETLTPDTQYEFQVRVKPLQGEFTTWSPWSQPLAF
RTKPAALGKDT (SEQ ID NO:168)
**Pro-IL2(MMP)** ELCDDDPPEIPHATFKAMAYKEGTMLNCECKRGFRRIKSGSLYMLCTGNSSHSSWDNQCQCTSSATRN 
TTKQVTPQPEEQKERKTTEMQSPMQPVDQASLPGHCREPPPWENEATERIYHFVVGQMVYYQCVQG
YRALHRGPAESVCKMTHGKTRWTQPQLICTGEMETSQFPGEEKPQASPEGRPESETSCLVTTTDFQIQT
EMAATMETSGGGGSGGGGSSGARYRWLTAGGGGSGGGGSAPTSSSTKKTQLQLEHLLLDLQMILNGI
NNYKNPKLTRMLTFKFYMPKKATELKHLQCLEEELKPLEEVLNLAQSKNFHLRPRDLISNINVIVLELK
GSETTFMCEYADETATIVEFLNRWITFCQSIISTLT (SEQ ID NO:169)
**Pro-IL2(G4S)** 
ELCDDDPPEIPHATFKAMAYKEGTMLNCECKRGFRRIKSGSLYMLCTGNSSHSSWDNQCQCTSSATRN
TTKQVTPQPEEQKERKTTEMQSPMQPVDQASLPGHCREPPPWENEATERIYHFVVGQMVYYQCVQG
YRALHRGPAESVCKMTHGKTRWTQPQLICTGEMETSQFPGEEKPQASPEGRPESETSCLVTTTDFQIQT
EMAATMETSGGGGSGGGGSGGGGSGGGGSGGGGSAPTSSSTKKTQLQLEHLLLDLQMILNGINNYK
NPKLTRMLTFKFYMPKKATELKHLQCLEEELKPLEEVLNLAQSKNFHLRPRDLISNINVIVLELKGSET
TFMCEYADETATIVEFLNRWITFCQSIISTLT (SEQ ID NO:170)
**mutIL2 R38E** 
APTSSSTKKTQLQLEHLLLDLQMILNGINNYKNPKLTEMLTFKFYMPKKATELKHLQCLEEELKPLEE
VLNLAQSKNFHLRPRDLISNINVIVLELKGSETTFMCEYADETATIVEFLNRWITFCQSIISTLT (SEQ ID
NO:171)
**mutIL2 K35E** 
APTSSSTKKTQLQLEHLLLDLQMILNGINNYKNPKLTEMLTFKFYMPKKATELKHLQCLEEELKPLEE
VLNLAQSKNFHLRPRDLISNINVIVLELKGSETTFMCEYADETATIVEFLNRWITFCQSIISTLT (SEQ ID
NO:172)
**mutIL2 K35E/R38E** 
APTSSSTKKTQLQLEHLLLDLQMILNGINNYKNPELTEMLTFKFYMPKKATELKHLQCLEEELKPLEEV
LNLAQSKNFHLRPRDLISNINVIVLELKGSETTFMCEYADETATIVEFLNRWITFCQSIISTLT (SEQ ID
NO:173)
**mutIL2 K35A/R38E** 
APTSSSTKKTQLQLEHLLLDLQMILNGINNYKNPALTEMLTFKFYMPKKATELKHLQCLEEELKPLEEV
LNLAQSKNFHLRPRDLISNINVIVLELKGSETTFMCEYADETATIVEFLNRWITFCQSIISTLT (SEQ ID
NO:174)
**mutIL2 K35E/R38E** 
APTSSSTKKTQLQLEHLLLDLQMILNGINNYKNPELTEMLTFKFYMPKKATELKHLQCLEEELKPLEEV
LNLAQSKNFHLRPRDLISNINVIVLELKGSETTFMCEYADETATIVEFLNRWITFCQSIISTLT (SEQ ID
NO:175)
**mutIL2 K35E/R38E/K43E** 
APTSSSTKKTQLQLEHLLLDLQMILNGINNYKNPELTEMLTFEFYMPKKATELKHLQCLEEELKPLEEV
LNLAQSKNFHLRPRDLISNINVIVLELKGSETTFMCEYADETATIVEFLNRWITFCQSIISTLT (SEQ ID
NO:176)
**mutIL2 K35E/R38E/K43E/N88E** APTSSSTKKTQLQLEHLLLDLQMILNGINNYKNPELTEMLTFEFYMPKKATELKHLQCLEEELKPLEEV 
LNLAQSKNFHLRPRDLISEINVIVLELKGSETTFMCEYADETATIVEFLNRWITFCQSIISTLT (SEQ ID
NO:177)
**hIL15 E46K** 
NWVNVISDLKKIEDLIQSMHIDATLYTESDVHPSCKVTAMKCFLLKLQVISLESGDASIHDTVENLIILA
NNSLSSNGNVTESGCKECEELEEKNIKEFLQSFVHIVQMFINTS (SEQ ID NO:178)
**hIL21** 
KSSSQGQDRHMIRMRQLIDIVDQLKNYVNDLVPEFLPAPEDVETNCEWSAFSCFQKAQLKSANTGNN
ERIINVSIKKLKRKPPSTNAGRRQKHRLTCPSCDSYEKKPPKEFLERFKSLLQKMIHQHLSSRTHGSEDS
(SEQ ID NO:179)
**mIL21** 
HKSSPQGPDRLLIRLRHLIDIVEQLKIYENDLDPELLSAPQDVKGHCEHAAFACFQKAKLKPSNPGNNK
TFIIDLVAQLRRRLPARRGGKKQKHIAKCPSCDSYEKRTPKEFLERLKWLLQKMIHQHLS (SEQ ID
NO:180)
**mIL4** 
HIHGCDKNHLREIIGILNEVTGEGTPCTEMDVPNVLTATKNTTESELVCRASKVLRIFYLKHGKTPCLK
KNSSVLMELQRLFRAFRCLDSSISCTMNESKSTSLKDFLESLKSIMQMDYS (SEQ ID NO:181)
**hIL4** 

 (SEQ ID
NO:182)
**IL10** 
SPGQGTQSENSCTHFPGNLPNMLRDLRDAFSRVKTFFQMKDQLDNLLLKESLLEDFKGYLGCQALSE
MIQFYLEEVMPQAENQDPDIKAHVNSLGENLKTLRLRLRRCHRFLPCENKSKAVEQVKNAFNKLQEK
GIYKAMSEFDIFINYIEAYMTMKIRN (SEQ ID NO:183)
**2×IL10** 
SPGQGTQSENSCTHFPGNLPNMLRDLRDAFSRVKTFFQMKDQLDNLLLKESLLEDFKGYLGCQALSE
MIQFYLEEVMPQAENQDPDIKAHVNSLGENLKTLRLRLRRCHRFLPCENKSKAVEQVKNAFNKLQEK
GIYKAMSEFDIFINYIEAYMTMKIRNGGGSGGGSSPGQGTQSENSCTHFPGNLPNMLRDLRDAFSRVK
TFFQMKDQLDNLLLKESLLEDFKGYLGCQALSEMIQFYLEEVMPQAENQDPDIKAHVNSLGENLKTL
RLRLRRCHRFLPCENKSKAVEQVKNAFNKLQEKGIYKAMSEFDIFINYIEAYMTMKIRN (SEQ ID
NO:184)
**CLDN-hIL15Ra** 
METDTLLLWVLLLWVPGSTGDIVMTQSPSSLTVTAGEKVTMSCKSSQSLLNSGNQKNYLTWYQQKPG
QPPKLLIYWASTRESGVPDRFTGSGSGTDFTLTISSVQAEDLAVYYCQNDYFYPFTFGSGTKLEIKGGG
GSGGGGSGGGGSQVQLQQPGAELVRPGASVKLSCKASGYTFTSYWINWVKQRPGQGLEWIGNIYPS
DSYTNYNQKFKDKATLTVDKSSSTAYMQLSSPTSEDSAVYYCARVYYGNSFAYWGQGTTLTVSSITCP
PPMSVEHADIWVKSYSLYSRERYICNSGFKRKAGTSSLTECVLNKATNVAHWTTPSLKCIRDPALVHQ
RPAPPDKTHTCPPCPAPEAAGGPSVFLFPPKPKDTLMISRTPEVTCVVVDVSHEDPEVKFNWYVDGVE
 
VHNAKTKPREEQYNSTYRVVSVLTVLHQDWLNGKEYKCKVSNKALGAPIEKTISKAKGQPREPQVCT
LPPSRDELTKNQVSLSCAVKGFYPSDIAVEWESNGQPENNYKTTPPVLDSDGSFKLVSKLTVDKSRWQ
QGNVFSCSVMHEALHNHYTQKSLSLSPGK (SEQ ID NO:185)
**CLDN-MMP-CD3-hIL15**

QVQLQQPGAELVRPGASVKLSCKASGYTFTSYWINWVKQRPGQGLEWIGNIYPSDSYTNYNQKFKD
KATLTVDKSSSTAYMQLSSPTSEDSAVYYCARVYYGNSFAYWGQGTTLTVSSGSWLPSSITAEVQLVES
GGGLVQPGGSLRLSCAASGYSFTGYTMNWVRQAPGKGLEWVALINPYKGVSTYNQKFKDRFTISVD
KSKNTAYLQMNSLRAEDTAVYYCARSGYYGDSDWYFDVWGQGTLVTVSSRSASTKGPSVFPLAPSSK
STSGGTAALGCLVKDYFPEPVTVSWNSGALTSGVHTFPAVLQSSGLYSLSSVVTVPSSSLGTQTYICNV
NHKPSNTKVDKRVNWVNVISDLKKIEDLIQSMHIDATLYTESDVHPSCKVTAMKCFLLELQVISLESG
DASIHDTVENLIILANNSLSSNGNVTESGCKECEELEEKNIKEFLQSFVHIVQMFINTSEPKSCDKTHTC
PPCPAPEAAGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALGAPIEKTISKAKGQPREPQVYTLPPCRDELTKN
QVSLWCLVKGFYPSDIAVEWESNGQPENNYKTTPPVLDSDGSFFLYSALTVDKSRWQQGNVFSCSVM
HEALHNHYTQKSLSLSPGK (SEQ ID NO:186)

## Claims

1. A fusion protein comprising structural unit A and structural unit B, wherein structural unit A is an IgG-type bispecific antibody comprising structural unit 1 and structural unit 2, wherein structural unit 1 is an anti-CD3 antibody, and structural unit 2 is an anti-tumor cell surface antigen antibody, and wherein the antibody represented by structural unit 1 and the antibody represented by structural unit 2 are in the form of Fab or scFv, respectively; and structural unit B is one or more (preferably two) cytokines or pro-cytokines, wherein structural unit B is linked to the N-terminus and/or C-terminus of the bispecific antibody via a cleavable or non-cleavable linker, and wherein the pro-cytokine is the cytokine linked to an antibody or a receptor of the cytokine via a cleavable linker; preferably, the fusion protein is in the form of a protein drug, mRNA, or CART.

2. The fusion protein according to claim 1, wherein
when structural unit 1 and structural unit 2 are both in the form of scFv, structural unit B is located at the N-terminus or C-terminus of structural unit 1;
when structural unit 1 and/or structural unit 2 is in the form of scFv and/or Fab, the bispecific antibody further comprises structural unit 4 which is an Fc fragment of an antibody or a human serum albumin,
I) when structural unit 4 is an Fc fragment of an antibody:
structural unit 1 and structural unit 2 are both located at the N-terminus of the bispecific antibody; and structural unit 4 is linked to the C-terminus of structural unit 1 and the C-terminus of structural unit 2, respectively; and
structural unit B is located at the N-terminus of structural unit 1 and/or the N-terminus of structural unit 2, or is located at the C-terminus of structural unit 4, and structural unit B is one or two cytokines or pro-cytokines;
II) when structural unit 4 is a human serum albumin:
the bispecific antibody comprises, in sequence from the N-terminus to the C-terminus:
a) structural unit 2 -structural unit 4 -structural unit 1,
b) structural unit 4 -structural unit 1 -structural unit 2,
c) structural unit 2 -structural unit 1 -structural unit 4, or
d) structural unit 1 -structural unit 4 -structural unit 2.

3. The fusion protein according to claim 1, wherein structural unit A further comprises structural unit 4 which is an Fc fragment of an antibody; structural unit 1 and structural unit 2 are in the form of scFv, and structural unit 2 is located at the N-terminus of structural unit 1; the C-terminus of structural unit 1 is linked to one chain of the Fc fragment, and structural unit B is linked to the other chain of the Fc fragment; preferably, structural unit B is one or two cytokines or pro-cytokines.

4. The fusion protein according to claim 1 or 2, wherein
structural unit A further comprises structural unit 3 and/or structural unit 4,
structural unit 3 is a tumor cell targeting peptide, linked to the N-terminus of structural unit 1 via a linker selected from the group consisting of:
(2) linker 1, which is a pH-sensitive linker;
(2) linker 2, which is a cleavable linker; and
(3) linker 3, which is a combination of a pH-sensitive linker and a cleavable linker, wherein the cleavable linker and the pH-sensitive linker are sequentially linked (wherein the cleavable linker is located at the N-terminus or C-terminus), or the sequence of linker 3 is set forth in SEQ ID NO: 13 or 14;
structural unit 4 is an Fc fragment of an antibody or a human serum albumin,
I) when structural unit 4 is an Fc fragment of an antibody:
structural unit 1, structural unit 2 and structural unit 3 are all located at the N-terminus or C-terminus of the bispecific antibody;
structural unit 4 is linked to the C-terminus of structural unit 1 and the C-terminus of structural unit 2, respectively, while the N-terminus of structural unit 1 is linked to structural unit 3; or
structural unit 4 is linked to the N-terminus of structural unit 1 and the N-terminus of structural unit 2, respectively, while the C-terminus of structural unit 1 is linked to structural unit 3;
II) when structural unit 4 is a human serum albumin:
the bispecific antibody comprises, in sequence from N-terminus to C-terminus:
a) structural unit 2 -structural unit 4 -structural unit 1-structural unit 3,
b) structural unit 3 -structural unit 4 -structural unit 1 - structural unit 2,
c) structural unit 2 -structural unit 1 -structural unit 4-structural unit 3, or
d) structural unit 3 -structural unit 1 -structural unit 4 -structural unit 2.

5. The fusion protein according to claim 4, wherein
(1) the bispecific antibody consists of structural units 1 to 4; structural units 1 to 3 are located at the N-terminus of the bispecific antibody, structural unit 4 is located at the C-terminus of the bispecific antibody, and structural unit 3 is linked to the N-terminus of structural unit 1 via a linker; structural units 1 and 3 are in the form of Fab, structural unit 2 is in the form of scFv, and structural unit 4 is an Fc fragment of an antibody;
wherein the cytokine is linked to the C-terminus of the light chain variable region of structural unit 1 or to the C-terminus of either chain of structural unit 4 via a cleavable or non-cleavable linker;
(2) the bispecific antibody consists of structural unit 1, structural unit 2 and structural unit 4; structural units 1 and 2 are located at the N-terminus of the bispecific antibody, structural unit 4 is located at the C-terminus of the bispecific antibody; structural unit 1 is in the form of Fab, structural unit 2 is in the form of scFv, and structural unit 4 is an Fc fragment of an antibody;
wherein the cytokine is linked to the N-terminus of one chain of structural unit 1 via a cleavable or non-cleavable linker, and the receptor of the cytokine is linked to the N-terminus of the other chain of sstructural unit 1 via a cleavable or non-cleavable linker.

6. The fusion protein according to claim 5, wherein
when the cytokine is linked to the C-terminus of the light chain variable region of structural unit 1, another terminus of the cytokine is further linked to a receptor of the cytokine via a cleavable or non-cleavable linker; or
when the cytokine is linked to the C-terminus of either chain of structural unit 4, structural unit B further comprises a receptor of the cytokine, wherein the receptor of the cytokine is linked to the C-terminus of another chain of structural unit 4 via a cleavable or non-cleavable linker, or the receptor of the cytokine is linked toanother terminus of the cytokine via a cleavable or non-cleavable linker.

7. The fusion protein according to claim 4, wherein structural unit 3 is selected from an anti-tumor cell surface antigen antibody, NGR, CNGRC, RGD, MT1-AF7p, YSA or TNYL; and when structural unit 3 is an anti-tumor cell surface antigen antibody which is Fab or scFv format, the tumor cell surface antigen targeted by structural unti 3 is same as or different from the tumor cell surface antigen targeted by structural unit 2.

8. The fusion protein according to claim 4 or 7, wherein structural unit B is located at the C-terminus of structural unit A, and structural unit B is one or two cytokines or pro-cytokines.

9. The fusion protein according to claim 1 or 2, wherein the cytokine is selected from a wild- of IL2 (such as ACCESSION P60568), IL15 (such as ACCESSION P40933), IL-21 (such as ACCESSION Q9HBE4-1 or Q9HBE4-2), IL-10 (such as ACCESSION P22301), or IL-4 (such as ACCESSION P05112), or a mutant thereof.

10. The fusion protein according to claim 5 or 6, wherein the cytokine is a wild-type of IL2 or IL15, or a mutant thereof;
preferably, the sequence of IL2 is set forth in ACCESSION P60568, the sequence of IL15 is set forth in SEQ ID NO: 135 or 137, the sequence of the IL2 mutant is set forth in any one of SEQ ID NOs: 110, and 171-177, and the sequence of the IL15 mutant is set forth in SEQ ID NOs: 147, 148, or 178;
preferably, when structural unit B comprises a receptor of the cytokine, the sequence of the IL15 receptor is set forth in SEQ ID NO: 136 or 138, the IL15-IL15 receptor complex is selected from the sequence set forth in SEQ ID NO: 133 or 146, and the sequence of the IL2 receptor is set forth in SEQ ID NO: 167 or 168.

11. The fusion protein according to any one of claims 1 to 10, wherein the anti-CD3 antibody is selected from the group consisting of:
(1) OKT3, wherein the CDR1 sequence of the VL thereof is set forth in SEQ ID NO: 48, the CDR2 sequence of the VL thereof is DTS, the CDR3 sequence of the VL thereof is set forth in SEQ ID NO: 49, the CDR1 sequence of the VH thereof is set forth in SEQ ID NO: 50, the CDR2 sequence of the VH thereof is set forth in SEQ ID NO: 51, and the CDR3 sequence of the VH thereof is set forth in SEQ ID NO: 52; preferably, the amino acid sequence of the VL thereof is set forth in SEQ ID NO: 35, and the amino acid sequence of the VH thereof is set forth in SEQ ID NO: 36;
(2) L2K, wherein the amino acid sequence of the VL thereof is set forth in SEQ ID NO: 37 and the amino acid sequence of the VH thereof is set forth in SEQ ID NO: 38;
(3) UCHT1, wherein the CDR1 sequence of the VL thereof is set forth in SEQ ID NO: 53, the CDR2 sequence of the VL thereof is YTS, the CDR3 sequence of the VL thereof is set forth in SEQ ID NO: 54, the CDR1 sequence of the VH thereof is set forth in SEQ ID NO: 55, the CDR2 sequence of the VH thereof is set forth in SEQ ID NO: 56, and the CDR3 sequence of the VH thereof is set forth in SEQ ID NO: 57;
preferably, the amino acid sequence of the VL thereof is set forth in SEQ ID NO: 39, and the amino acid sequence of the VH thereof is set forth in SEQ ID NO: 40;
(4) SP34, wherein the CDR1 sequence of the VL thereof is set forth in SEQ ID NO: 58, the CDR2 sequence of the VL thereof is GTN, the CDR3 sequence of the VL thereof is set forth in SEQ ID NO: 59, the CDR1 sequence of the VH thereof is set forth in SEQ ID NO: 60, the CDR2 sequence of the VH thereof is set forth in SEQ ID NO: 61, and the CDR3 sequence of the VH thereof is set forth in SEQ ID NO: 62;
preferably, the amino acid sequence of the VL thereof is set forth in SEQ ID NO: 41, and the amino acid sequence of the VH thereof is set forth in SEQ ID NO: 42;
(5) 17A2, wherein the CDR1 sequence of the VL thereof is set forth in SEQ ID NO: 63, the CDR2 sequence of the VL thereof is YAS, the CDR3 sequence of the VL thereof is set forth in SEQ ID NO: 64, the CDR1 sequence of the VH thereof is set forth in SEQ ID NO: 65, the CDR2 sequence of the VH thereof is set forth in SEQ ID NO: 66, and the CDR3 sequence of the VH thereof is set forth in SEQ ID NO: 67;
preferably, the amino acid sequence of the VL thereof is set forth in SEQ ID NO: 43, and the amino acid sequence of the VH thereof is set forth in SEQ ID NO: 44; and
(6) 145-2C11, wherein the CDR1 sequence of the VL thereof is set forth in SEQ ID NO: 68, the CDR2 sequence of the VL thereof is YTN, the CDR3 sequence of the VL thereof is set forth in SEQ ID NO: 69, the CDR1 sequence of the VH thereof is set forth in SEQ ID NO: 70, the CDR2 sequence of the VH thereof is set forth in SEQ ID NO: 71, and the CDR3 sequence of the VH thereof is set forth in SEQ ID NO: 72; preferably, the amino acid sequence of the VL thereof is set forth in SEQ ID NO: 45, and the amino acid sequence of the VH thereof is set forth in SEQ ID NO: 46.

12. The fusion protein according to any one of claims 4 to 10, wherein
the pH-sensitive linker is selected from HA, or the cleavable linker is selected from the cleavage site of MMP, uPA, or CD13;
preferably, the MMP is selected from the group consisting of:
(1) any one of SEQ ID NOs: 6-8 and 83-94; or
(2) each pairwise combination of any one sequence selected from SEQ ID NOs: 6-8 and any one sequence selected from SEQ ID NOs: 83-94; or
(3) each pairwise combination of any one sequence selected from SEQ ID NOs: 1-3, 24, 28, 30, and 32 and any one sequence selected from SEQ ID NOs: 6-8 and 83-94.

13. The fusion protein according to any one of claims 1 to 12, wherein the tumor cell surface antigen is selected from the group consisting of Claudin18.2 (such as ACCESSION: P56856), PDL1 (such as ACCESSION: Q9NZQ7), CD139 (such as ACCESSION: P15144), MSLN (such as ACCESSION: Q12321), GPC3 (such as ACCESSION: Q8CFZ4), Her29r (such as ACCESSION: P04626), EGFR (such as ACCESSION: P00533), GD2 (such as ACCESSION: Q9UI17), Trop2 (such as ACCESSION: P09758), CD19 (such as ACCESSION: P15391), CD20 (such as ACCESSION: P11836), Her3 ACCESSION: P21860, FolR1 (such as ACCESSION: P15328), Nectin4 (such as ACCESSION: Q96NY8), CD276 (such as ACCESSION: Q5ZPR3), CD30 (such as ACCESSION: P28908), CD70 (such as ACCESSION: P32970), BCMA (such as ACCESSION: Q02223), CD22 (such as ACCESSION: P20273), CD33 (such as ACCESSION: P20138), GPRC5D (such as ACCESSION: Q9NZD1), HLADR (such as ACCESSION: Q29744), VTCN1 (such as ACCESSION: Q7Z7D3), CD79B (such as ACCESSION: P40295), CDH3 ACCESSION: P22223, DR5 ACCESSION: P21918, MUC1 (such as ACCESSION: P15941), NT5E (such as ACCESSION: P21589), FAP (such as ACCESSION: Q12884), and PSMA (such as ACCESSION: Q04609).

14. The fusion protein according to any one of claims 4 to 13, wherein the tumor cell surface antigen targeted by structural unit 2 is Claudin18.2, and the tumor cell surface antigen targeted by structural unit 3 is Claudin18.2;
wherein the tumor cell surface antigen targeted by structural unit 2 is Claudin18.2, and the tumor cell surface antigen targeted by structural unit 3 is PDL1;
wherein the tumor cell surface antigen targeted by structural unit 2 is PDL1, and the tumor cell surface antigen targeted by structural unit 3 is Claudin18.2;
wherein the tumor cell surface antigen targeted by structural unit 2 is PDL1, and the tumor cell surface antigen targeted by structural unit 3 is PDL1;
wherein the tumor cell surface antigen targeted by structural unit 2 is MSLN, and the tumor cell surface antigen targeted by structural unit 3 is MSLN; or
wherein the tumor cell surface antigen targeted by structural unit 2 is FAP, and the tumor cell surface antigen targeted by structural unit 3 is FAP;
preferably, for structural unit 2 or 3 targeting the tumor cell surface antigen PDL1, the LCDR1 sequence thereof is set forth in SEQ ID NO: 73, the LCDR2 sequence thereof is SAS, the LCDR3 sequence thereof is set forth in SEQ ID NO: 74, the HCDR1 sequence thereof is set forth in SEQ ID NO: 75, the HCDR2 sequence thereof is set forth in SEQ ID NO: 76, and the HCDR3 sequence thereof is set forth in SEQ ID NO: 77;
for structural unit 2 or 3 targeting the tumor cell surface antigen Claudin18.2, the LCDR1 sequence thereof is set forth in SEQ ID NO: 78, the LCDR2 sequence thereof is WAS, the LCDR3 sequence thereof is set forth in SEQ ID NO: 79, the HCDR1 sequence thereof is set forth in SEQ ID NO: 80, the VCDR2 sequence is set forth in SEQ ID NO: 81, and the HCDR3 sequence thereof is set forth in SEQ ID NO: 82; or
for structural unit 2, the LCDR1 sequence thereof is set forth in SEQ ID NO: 78, the LCDR2 sequence thereof is WAS, the LCDR3 sequence thereof is set forth in SEQ ID NO: 79, the HCDR1 sequence thereof is set forth in SEQ ID NO: 80, the HCDR2 sequence thereof is set forth in SEQ ID NO: 81, and the HCDR3 sequence thereof is set forth in SEQ ID NO: 82; and for structural unit 3, the LCDR1 sequence there thereof of is set forth in SEQ ID NO: 73, the LCDR2 sequence thereof is SAS, the LCDR3 sequence is set forth in SEQ ID NO: 74, the HCDR1 sequence thereof is set forth in SEQ ID NO: 75, the HCDR2 sequence thereof is set forth in SEQ ID NO: 76, and the HCDR3 sequence thereof is set forth in SEQ ID NO: 77.

15. The fusion protein according to any one of claims 4 to13, wherein
the tumor cell surface antigen targeted by structural unit 2 is Claudin18.2 as set forth in ACCESSION: P56856, and structural unit 3 is CNGRC;
the tumor cell surface antigen targeted by structural unit 2 is Claudin18.2, and structural unit 3 is RGD;
the tumor cell surface antigen targeted by structural unit 2 is Claudin18.2 as set forth in ACCESSION: P56856, and structural unit 3 is TNYL; or
the tumor cell surface antigen targeted by structural unit 2 is MSLN, and structural unit 3 is CNGRC.

16. The fusion protein according to any one of claims 4 to 15, wherein the Fc fragment is an Fc fragment of IgG1, IgG2, IgG3, or IgG4; preferably, the Fc fragment is a humanized Fc fragment.

17. The fusion protein according to any one of claims 4 to 16, wherein the heavy chain constant region of the Fc fragment is an Ig gamma-1 chain C region of ACCESSION: P01857; and the light chain constant region of the Fc fragment is an Ig kappa chain C region of ACCESSION: P01834;
preferably, according to the EU numbering system, based on the Ig gamma-1 chain C region of ACCESSION: P01857, the heavy chain constant region of the Fc fragment has the following mutations at positions 234, 235, and/or 237:
L234A and L235A,
L234A and G237A,
L235A and G237A, or
L234A, L235A, and G237A;
preferably, according to the EU numbering system, the heavy chain constant region of the Fc fragment further has one or more mutations selected from the group consisting of: N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, H268A, E318A, and K320A.

18. The fusion protein according to any one of claims 4 to 17, wherein the sequence of the Fc fragment is selected from SEQ ID NO: 19 and SEQ ID NO: 20.

19. The fusion protein according to any one of claims 4 to 18, wherein when structural unit 4 is a human serum albumin, it is the human serum albumin of ACCESSION: P02768.

20. The fusion protein according to any one of claims 4 to 19, wherein the sequence of structural unit 2 is selected from SEQ ID NO: 17, SEQ ID NO: 18, or SEQ ID NO: 156; and/or the sequence of structural unit 3 is selected from SEQ ID NO: 17, SEQ ID NO: 18, SEQ ID NO: 156, SEQ ID NO: 33, or SEQ ID NO: 34;
preferably, the bispecific antibody is selected from the group consisting of:
(1) wherein the sequence of structural unit 2 is selected from the sequence as set forth in SEQ ID NO: 17, and the sequence of structural unit 3 is selected from the sequence as set forth in SEQ ID NO: 18;
(2) wherein the sequence of structural unit 2 is selected from the sequence as set forth in SEQ ID NO: 18, and the sequence of structural unit 3 is selected from the sequence as set forth in SEQ ID NO: 17;
(3) wherein the sequence of structural unit 2 is selected from the sequence as set forth in SEQ ID NO: 17, and the sequence of structural unit 3 is selected from the sequence as set forth in SEQ ID NO: 33;
(4) wherein the sequence of structural unit 2 is selected from the sequence as set forth in SEQ ID NO: 18, and the sequence of structural unit 3 is selected from the sequence as set forthin SEQ ID NO: 33;
(5) wherein the sequence of structural unit 2 is selected from the sequence as set forth inSEQ ID NO: 18, and the sequence of structural unit 3 is selected from the sequence as set forth in SEQ ID NO: 34; and
(6) wherein the sequence of structural unit 2 is selected from the sequence as set forth in SEQ ID NO: 17, and the sequence of structural unit 3 is selected from the sequence as set forth in SEQ ID NO: 34.

21. The fusion protein according to any one of claims 4 to 20, wherein the bispecific antibody is selected from the group consisting of:
(1) a bispecific antibody comprising or consisting of the sequences as set forth in SEQ ID NO: 22 and SEQ ID NO: 23;
(2) a bispecific antibody comprising or consisting of the sequences as set forth in SEQ ID NO: 22 and SEQ ID NO: 25;
(3) a bispecific antibody comprising or consisting of the sequences as set forth in SEQ ID NO: 26 and SEQ ID NO: 27;
(4) a bispecific antibody comprising or consisting of the sequences as set forth in SEQ ID NO: 26 and SEQ ID NO: 29;
(5) a bispecific antibody comprising or consisting of the sequences as set forth in SEQ ID NO: 26 and SEQ ID NO: 31;
(6) a bispecific antibody comprising or consisting of the sequence as set forth in SEQ ID NO: 22 and a sequence selected from any one of SEQ ID NOs: 95-109;
(7) a bispecific antibody comprising or consisting of the sequences as set forth in SEQ ID NO: 22, SEQ ID NO: 107, and SEQ ID NO: 108;
(8) a bispecific antibody comprising or consisting of the sequences as set forth in SEQ ID NO: 22, SEQ ID NO: 107, and SEQ ID NO: 109;
(9) a bispecific antibody prodrug comprising or consisting of the sequences represented by SEQ ID NO: 22, sequence A, SEQ ID NO: 158, SEQ ID NO: 159, SEQ ID NO: 162, and SEQ ID NO: 163, wherein sequence A is selected from any one of SEQ ID NOs: 4-14, 83-94, or 149-155;
(10) a bispecific antibody prodrug comprising or consisting of the sequences represented by SEQ ID NO: 22, sequence A, SEQ ID NO: 160, SEQ ID NO: 161, SEQ ID NO: 162, and SEQ ID NO: 163, wherein sequence A is selected from any one of SEQ ID NOs: 4-14, 83-94, or 149-155;
(11) a bispecific antibody prodrug comprising the sequences represented by SEQ ID NO: 156, sequence A, SEQ ID NO: 158, SEQ ID NO: 159, SEQ ID NO: 164, and SEQ ID NO: 165, or consisting of the sequences represented by SEQ ID NO: 22, sequence A, SEQ ID NO: 158, SEQ ID NO: 159, SEQ ID NO: 164, and SEQ ID NO: 165, wherein sequence A is selected from any one of SEQ ID NOs: 4-14, 83-94, or 149-155; and
(12) a bispecific antibody prodrug comprising or consisting of the sequences represented by SEQ ID NO: 156, sequence A, SEQ ID NO: 160, SEQ ID NO: 161, SEQ ID NO: 164, and SEQ ID NO: 165, wherein sequence A is selected from any one of SEQ ID NOs: 4-14, or 83-94, and 149-155;
preferably, the fusion protein is selected from the group consisting of:
(1) a fusion protein comprising or consisting of the sequence as set forth in SEQ ID NO: 111 and the sequence selected from any one of SEQ ID NOs: 22, 26, or 156;
(2) a fusion protein comprising or consisting of the sequences as set forth in SEQ ID NO: 112 and SEQ ID NO: 113;
(3) a fusion protein comprising or consisting the sequences as set forth in SEQ ID NO: 112 and SEQ ID NO: 114;
(4) a fusion protein comprising or consisting of the sequences as set forth in SEQ ID NO: 112 and SEQ ID NO: 115;
(5) a fusion protein comprising or consisting of the sequence as set forth in SEQ ID NO: 116 and the sequence selected from any one of SEQ ID NOs: 22, 26, or 156;
(6) a fusion protein comprising or consisting of the sequence selected from any one of SEQ ID NO: 22, 26, or 156, and the sequences as set forth in SEQ ID NO: 117 and SEQ ID NO: 108;
(7) a fusion protein comprising or consisting of the sequences as set forth in SEQ ID NO: 112, SEQ ID NO: 118, and SEQ ID NO: 107;
(8) a fusion protein comprising or consisting of the sequences as set forth in SEQ ID NO: 112, SEQ ID NO: 119, and SEQ ID NO: 107;
(9) a fusion protein comprising or consisting of the sequence selected from any one of SEQ ID NOs: 120-125 and the sequence selected from any one of SEQ ID NOs: 25, 29, or 95-109;
(10) a fusion protein comprising or consisting of the sequence selected from any one of SEQ ID NOs: 22, 26, or 156 and the sequence selected from any one of SEQ ID NOs: 126-128;
(11) a fusion protein comprising or consisting of the sequence as set forth in SEQ ID NO: 129 and the sequence selected from any one of SEQ ID NOs: 23, 25, 27, 29, 95-109, or 157;
(12) a fusion protein comprising or consisting of the sequences as set forth in SEQ ID NO: 130, and SEQ ID NO: 109, and the sequence selected from any one of SEQ ID NOs: 22, 26, or 156;
(13) a fusion protein comprising or consisting of the sequence as set forth in SEQ ID NO: 131 and the sequence selected from any one of SEQ ID NOs: 22, 26, or 156;
(14) a fusion protein comprising or consisting of the sequence as set forth in SEQ ID NO: 132 and the sequence selected from any one of SEQ ID NOs: 22, 26, or 156;
(15) a fusion protein comprising or consisting of the sequence selected from any one of SEQ ID NO: 22, 26, or 156 and a sequence selected from any one of SEQ ID NOs: 134, 139, 140, or 143;
(16) a fusion protein comprising or consisting of the sequence as set forth in SEQ ID NO: 141 and a sequence selected from any one of SEQ ID NOs: 25, 29, 95-109, or 157;
(17) a fusion protein comprising or consisting of the sequence as set forth in SEQ ID NO: 142 and a sequence selected from any one of SEQ ID NOs: 25, 29, 95-109, or 157;
(18) a fusion protein comprising or consisting of the sequences as set forth in SEQ ID NO: 139 and SEQ ID NO: 141;
(19) a fusion protein comprising or consisting of the sequences as set forth in SEQ ID NO: 140 and SEQ ID NO: 142;
(20) a fusion protein comprising or consisting of the sequence selected from any one of SEQ ID NOs: 22, 26, or 156, and the sequences as set forth in SEQ ID NO: 144 and SEQ ID NO: 108;
(21) a fusion protein comprising or consisting of the sequence selected from any one of SEQ ID NOs: 22, 26, or 156, and the sequences as set forth in SEQ ID NO: 144 and SEQ ID NO: 109;
(22) a fusion protein comprising or consisting of the sequence selected from any one of SEQ ID NOs: 22, 26, or 156, and the sequences as set forth in SEQ ID NO: 145 and SEQ ID NO: 108;
(23) a fusion protein comprising or consisting of the sequence selected from any one of SEQ ID NO: 22, 26, or 156, and the sequences as set forth in SEQ ID NO: 145 and SEQ ID NO: 109;
(24) a fusion protein comprising or consisting of the sequences as set forth in SEQ ID NO: 185, SEQ ID NO: 186, SEQ ID NO: 162, SEQ ID NO: 149, and SEQ ID NO: 161;
(25) a fusion protein comprising or consisting of an anti-CD3 antibody, an anti-tumor cell surface antigen antibody, an Fc fragment, a cytokine, and a linker, wherein the sequence of the anti-CD3 antibody is selected from the sequence as set forth in any one of SEQ ID NOs: 23, 25, 27, 29, 95-109, or 157, the sequence of the anti-tumor cell surface antigen antibody is selected from the sequence as set forth in any one of SEQ ID NO: 22, 26, or 156, the sequence of the cytokine is selected from the sequence as set forth in any one of SEQ ID NOs: 110, 133, 135, 137, 146-148, 166, or 169-184, and the sequence of the linker is selected from the sequence as set forth in any one of SEQ ID NOs: 1-14, 24, 28, 30, 32, 83-94, or 149-155; preferably, the anti-CD3 antibody is in the form of scFv, and the cytokine is located at the C-terminus of the Fc fragment;
(26) a fusion protein comprising or consisting of an anti-CD3 antibody, an anti-tumor cell surface antigen antibody, an Fc fragment, a cytokine, a receptor of the cytokine, and a linker, wherein the anti-CD3 antibody comprises the sequences as set forth in SEQ ID NO: 158 and SEQ ID NO: 159, the sequence of the anti-tumor cell surface antigen antibody is selected from the sequence as set forth in any one of SEQ ID NOs: 22, 26, or 156, the sequence of the cytokine is selected from the sequence as set forth in any one of SEQ ID NOs: 110, 133, 135, 137, 147-148, 166, or 171-178, the sequence of the receptor of the cytokine is selected from the sequence as set forth in SEQ ID NOs: 136, 138, 167, or 168, and the sequence of the linker is selected from the sequence as set forth in any one of SEQ ID NOs: 1-14, 24, 28, 30, 32, 83-94, or 149-155; wherein the anti-CD3 antibody is in the form of Fab, and the cytokine and the receptor of the cytokine are located at the N-terminus of the anti-CD3 antibody;
(27) a fusion protein comprising or consisting of an anti-CD3 antibody, an anti-tumor cell surface antigen antibody, an Fc fragment, a cytokine, a receptor of the cytokine, and a linker, wherein the anti-CD3 antibody comprises the sequences as set forth in SEQ ID NO: 160 and SEQ ID NO: 161, the sequence of the anti-tumor cell surface antigen antibody is selected from the the sequence as set forth in any one of SEQ ID NOs: 22, 26, or 156, the sequence of the cytokine is selected from the sequence as set forth in any one of SEQ ID NOs: 110, 133, 135, 137, 147-148, 166, or 171-178, the sequence of the receptor of the cytokine is selected from the sequence as set forth in SEQ ID NOs: 136, 138, 167, or 168, and the sequence of the linker is selected from the sequence as set forth in any one of SEQ ID NOs: 1-14, 24, 28, 30, 32, 83-94, or 149-155; wherein the anti-CD3 antibody is in the form of Fab, and the cytokine and the receptor of the cytokine are located at the N-terminus of the anti-CD3 antibody;
(28) a fusion protein comprising or consisting of an anti-CD3 antibody, two anti-tumor cell surface antigen antibodies, an Fc fragment, a cytokine, and a linker, wherein the anti-CD3 antibody comprises the sequences as set forth in SEQ ID NO: 158, SEQ ID NO: 159, SEQ ID NO: 162, and SEQ ID NO: 163, the anti-tumor cell surface antigen antibody comprises the sequence as set forth in SEQ ID NO: 22, the sequence of the cytokine is selected from the sequence as set forth in any one of SEQ ID NOs: 110, 133, 135, 137, 146-148, 166, or 169-184, and the sequence of the linker is selected from the sequence as set forth in any one of SEQ ID NOs: 1-14, 24, 28, 30, 32, 83-94, or 149-155; wherein the anti-CD3 antibody is in the form of Fab, one of the anti-tumor cell surface antigen antibodies is in the form of Fab and is located at the N-terminus of the anti-CD3 antibody, and the cytokine is located at the C-terminus of the anti-CD3 antibody or at the C-terminus of the Fc fragment;
(29) a fusion protein comprising or consisting of an anti-CD3 antibody, two anti-tumor cell surface antigen antibodies, an Fc fragment, a cytokine, and a linker, wherein the anti-CD3 antibody comprises the sequences as set forth in SEQ ID NO: 160, SEQ ID NO: 161, SEQ ID NO: 162, and SEQ ID NO: 163, the anti-tumor cell surface antigen antibody comprises the sequence as set forth in SEQ ID NO: 22, the sequence of the cytokine is selected from the sequence as set forth in any one of SEQ ID NOs: 110, 133, 135, 137, 146-148, 166, or 169-184, and the sequence of the linker is selected from the sequence as set forth in any one of SEQ ID NOs: 1-14, 24, 28, 30, 32, 83-94, or 149-155; wherein the anti-CD3 antibody is in the form of Fab, one of the anti-tumor cell surface antigen antibodies is in the form of Fab and is located at the N-terminus of the anti-CD3 antibody, and the cytokine is located at the C-terminus of the anti-CD3 antibody;
(30) a fusion protein comprising or consisting of an anti-CD3 antibody, two anti-tumor cell surface antigen antibodies, an Fc fragment, a cytokine, and a linker, wherein the anti-CD3 antibody comprises the sequences as set forth in SEQ ID NO: 160, SEQ ID NO: 161, SEQ ID NO: 164, and SEQ ID NO: 165, the anti-tumor cell surface antigen antibody comprises the sequence as set forth in SEQ ID NO: 156, the sequence of the cytokine is selected from the sequence as set forth in any one of SEQ ID NOs: 110, 133, 135, 137, 147-148, 166, or 169-184, and the sequence of the linker is selected from the sequence as set forth in any one of SEQ ID NOs: 1-14, 24, 28, 30, 32, 83-94, or 149-155; wherein the anti-CD3 antibody is in the form of Fab, one of the anti-tumor cell surface antigen antibodies is in the form of Fab and is located at the N-terminus of the anti-CD3 antibody, and the cytokine is located at the C-terminus of the anti-CD3 antibody;
(31) a fusion protein comprising or consisting of an anti-CD3 antibody, two anti-tumor cell surface antigen antibodies, an Fc fragment, a cytokine, and a linker, wherein the anti-CD3 antibody comprises the sequences as set forth in SEQ ID NO: 158, SEQ ID NO: 159, SEQ ID NO: 164, and SEQ ID NO: 165, the anti-tumor cell surface antigen antibody comprises the sequence as set forth in SEQ ID NO: 156, the sequence of the cytokine is selected from the sequence as set forth in any one of SEQ ID NOs: 110, 133, 135, 137, 147-148, 166, or 169-184, and the sequence of the linker is selected from the sequence as set forth in any one of SEQ ID NOs: 1-14, 24, 28, 30, 32, 83-94, or 149-155; wherein the anti-CD3 antibody is in the form of Fab, one of the anti-tumor cell surface antigen antibodies is in the form of Fab and is located at the N-terminus of the anti-CD3 antibody, and the cytokine is located at the C-terminus of the anti-CD3 antibody;
(32) a fusion protein comprising or consisting of an anti-CD3 antibody, two anti-tumor cell surface antigen antibodies, an Fc fragment, a cytokine, a receptor of the cytokine, and a linker, wherein the anti-CD3 antibody comprises the sequences as set forth in SEQ ID NO: 158, SEQ ID NO: 159, SEQ ID NO: 162, and SEQ ID NO: 163, the anti-tumor cell surface antigen antibody comprises the sequence as set forth in SEQ ID NO: 22, the sequence of the cytokine is selected from the sequence as set forth in any one of SEQ ID NOs: 110, 133, 135, 137, 147-148, 166, or 171-178, the sequence of the receptor of the cytokine is selected from the sequence as set forth in SEQ ID NOs: 136, 138, 167, or 168, and the sequence of the linker is selected from the sequence as set forth in any one of SEQ ID NOs: 1-14, 24, 28, 30, 32, 83-94, or 149-155; wherein the anti-CD3 antibody is in the form of Fab, one of the anti-tumor cell surface antigen antibodies is in the form of Fab and is located at the N-terminus of the anti-CD3 antibody;
wherein the cytokine and the receptor of the cytokine are located at the C-terminus of the Fc fragment, respectively, or located at the C-terminus of the anti-CD3 antibody and the C-terminus of the anti-tumor cell surface antigen antibody in the form of scFv, respectively; or
wherein the cytokine and the receptor of the cytokine are linked together and the receptor of the cytokine is linked to the C-terminus of the Fc fragment or the C-terminus of the anti-CD3 antibody;
(33) a fusion protein comprising or consisting of an anti-CD3 antibody, two anti-tumor cell surface antigen antibodies, an Fc fragment, a cytokine, a receptor of the cytokine, and a linker, wherein the anti-CD3 antibody comprises the sequences as set forth in SEQ ID NO: 160, SEQ ID NO: 161, SEQ ID NO: 162, and SEQ ID NO: 163, the anti-tumor cell surface antigen antibody comprises the sequence as set forth in SEQ ID NO: 22, the sequence of the cytokine is selected from the sequence as set forth in any one of SEQ ID NOs: 110, 133, 135, 137, 147-148, 166, or 171-178, the sequence of the receptor of the cytokine is selected from the sequence as set forth in SEQ ID NO: 136, 138, 167, or 168, and the sequence of the linker is selected from the sequence as set forth in any one of SEQ ID NOs: 1-14, 24, 28, 30, 32, 83-94, or 149-155; wherein the anti-CD3 antibody is in the form of Fab, one of the two anti-tumor cell surface antigen antibodies is in the form of Fab and is located at the N-terminus of the anti-CD3 antibody;
wherein the cytokine and the receptor of the cytokine are located at the C-terminus of the Fc fragment, respectively, or located at the C-terminus of the anti-CD3 antibody and the C-terminus of the anti-tumor cell surface antigen antibody in the form of scFv, respectively; or
wherein the cytokine and the receptor of the cytokine are linked together and the receptor of the cytokine is linked to the C-terminus of the Fc fragment or the C-terminus of the anti-CD3 antibody;
(34) a fusion protein comprising or consisting of an anti-CD3 antibody, two anti-tumor cell surface antigen antibodies, an Fc fragment, a cytokine, a receptor of the cytokine, and a linker, wherein the anti-CD3 antibody comprises the sequences as set forth in SEQ ID NO: 160, SEQ ID NO: 161, SEQ ID NO: 164, and SEQ ID NO: 165, the anti-tumor cell surface antigen antibody comprises the sequence as set forth in SEQ ID NO: 156, the sequence of the cytokine is selected from the sequence as set forth in any one of SEQ ID NOs: 110, 133, 135, 137, 147-148, 166, or 171-178, the sequence of the receptor of the cytokine is selected from the sequence as set forth in SEQ ID NO: 136, 138, 167, or 168, and the sequence of the linker is selected from the sequence as set forth in any one of SEQ ID NOs: 1-14, 24, 28, 30, 32, 83-94, or 149-155; wherein the anti-CD3 antibody is in the form of Fab, one of the anti-tumor cell surface antigen antibodies is in the form of Fab and is located at the N-terminus of the anti-CD3 antibody;
wherein the cytokine and the receptor of the cytokine are located at the C-terminus of the Fc fragment, respectively, or located at the C-terminus of the anti-CD3 antibody and the C-terminus of the anti-tumor cell surface antigen antibody in the form of scFv, respectively; or
wherein the cytokine and the receptor of the cytokine are linked together and the receptor of the cytokine is linked to the C-terminus of the Fc fragment or the C-terminus of the anti-CD3 antibody;
(35) a fusion protein comprising or consisting of an anti-CD3 antibody, two anti-tumor cell surface antigen antibodies, an Fc fragment, a cytokine, a receptor of the cytokine, and a linker, wherein the anti-CD3 antibody comprises the sequences as set forth in SEQ ID NO: 158, SEQ ID NO: 159, SEQ ID NO: 164, and SEQ ID NO: 165, the anti-tumor cell surface antigen antibody comprises the sequence as set forth in SEQ ID NO: 156, the sequence of the cytokine is selected from the sequence as set forth in any one of SEQ ID NOs: 110, 133, 135, 137, 147-148, 166, or 171-178, the sequence of the receptor of the cytokine is selected from the sequence as set forth in SEQ ID NO: 136, 138, 167, or 168, and the sequence of the linker is selected from the sequence as set forth inany one of SEQ ID NOs: 1-14, 24, 28, 30, 32, 83-94, or 149-155; wherein the anti-CD3 antibody is in the form of Fab, one of the anti-tumor cell surface antigen antibodies is in the form of Fab and is located at the N-terminus of the anti-CD3 antibody;
wherein the cytokine and the receptor of the cytokine are located at the C-terminus of the Fc fragment, respectively, or located at the C-terminus of the anti-CD3 antibody and the C-terminus of the anti-tumor cell surface antigen antibody in the form of scFv, respectively; or
wherein the cytokine and the receptor of the cytokine are linked together and the receptor of the cytokine is linked to the C-terminus of the Fc fragment or the C-terminus of the anti-CD3 antibody;
(36) a fusion protein comprising the sequences as set forth in SEQ ID NO: 22, SEQ ID NO: 160, and SEQ ID NO: 161;
(37) a fusion protein comprising the sequence as set forth in SEQ ID NO: 22, sequence a, and sequence b, wherein sequence a comprises, from the N-terminus to the C-terminus, the sequences as set forth in SEQ ID NO: 163, SEQ ID NO: 149, and SEQ ID NO: 160; and sequence b comprises, from the N-terminus to the C-terminus, the sequences as set forth in SEQ ID NO: 162, SEQ ID NO: 149, and SEQ ID NO: 161;
(38) a fusion protein comprising sequence a, sequence b, and sequence c, wherein sequence a comprises, from the N-terminus to the C-terminus, the sequences as set forth in SEQ ID NO: 22, SEQ ID NO: 3, and SEQ ID NO: 110; sequence b comprises, from the N-terminus to the C-terminus, the sequences as set forth in SEQ ID NO: 163, SEQ ID NO: 149, and SEQ ID NO: 160; and sequence c comprises, from the N-terminus to the C-terminus, the sequences as set forth in SEQ ID NO: 162, SEQ ID NO: 149, and SEQ ID NO: 161;
(39) a fusion protein comprising sequence a, sequence b, and sequence c, wherein sequence a comprises, from the N-terminus to the C-terminus, the sequences as set forth in SEQ ID NO: 22, SEQ ID NO: 3, and SEQ ID NO: 176; sequence b comprises, from the N-terminus to the C-terminus, the sequences as set forth in SEQ ID NO: 163, SEQ ID NO: 149, and SEQ ID NO: 160; and sequence c comprises, from the N-terminus to the C-terminus, the sequences as set forth in SEQ ID NO: 162, SEQ ID NO: 149, and SEQ ID NO: 161;
(40) a fusion protein comprising sequence a, and the sequences as set forth in SEQ ID NO: 160 and SEQ ID NO: 161, wherein sequence a comprises, from the N-terminus to the C-terminus, the sequences as set forth in SEQ ID NO: 22, SEQ ID NO: 3, and SEQ ID NO: 176;
(41) a fusion protein comprising the sequence as set forth in SEQ ID NO: 22, sequence a, and sequence b, wherein sequence a comprises, from the N-terminus to the C-terminus, the sequences as set forth in SEQ ID NO: 167, SEQ ID NO: 149, and SEQ ID NO: 160; and sequence b comprises, from the N-terminus to the C-terminus, the sequences as set forth in SEQ ID NO: 110, SEQ ID NO: 32, and SEQ ID NO: 161;
(42) a fusion protein comprising the sequence as set forth in SEQ ID NO: 22, sequence a, and sequence b, wherein sequence a comprises, from the N-terminus to the C-terminus, the sequences as set forth in SEQ ID NO: 163, SEQ ID NO: 149, and SEQ ID NO: 160; and sequence b comprises, from the N-terminus to the C-terminus, the sequences as set forth in SEQ ID NO: 162, SEQ ID NO: 149, and SEQ ID NO: 161;
(43) a fusion protein comprising the sequences as set forth in SEQ ID NO: 185 and SEQ ID NO: 186, and sequence a, wherein sequence a comprises, from the N-terminus to the C-terminus, the sequences as set forth in SEQ ID NO: 162, SEQ ID NO: 149, and SEQ ID NO: 161;
(44) a fusion protein comprising sequence a, sequence b, and sequence c, wherein sequence a comprises, from the N-terminus to the C-terminus, the sequences as set forth in SEQ ID NO: 22, SEQ ID NO: 3, and SEQ ID NO: 137; sequence b comprises, from the N-terminus to the C-terminus, the sequences as set forth in SEQ ID NO: 163, SEQ ID NO: 149, and SEQ ID NO: 160; and sequence c comprises, from the N-terminus to the C-terminus, the sequences as set forth in SEQ ID NO: 162, SEQ ID NO: 149, and SEQ ID NO: 161;
(45) a fusion protein comprising the sequence as set forth in SEQ ID NO: 22 and sequence a, wherein sequence a comprises, from the N-terminus to the C-terminus, the sequences as set forth in SEQ ID NO: 23, SEQ ID NO: 3, and SEQ ID NO: 137;
(46) a fusion protein comprising the sequences as set forth in SEQ ID NO: 22 and SEQ ID NO: 23;
(47) a fusion protein comprising the sequences as set forth in SEQ ID NO: 22 and SEQ ID NO: 157;
(48) a fusion protein comprising the sequences as set forth in SEQ ID NO: 129 and SEQ ID NO: 157;
(49) a fusion protein comprising the sequence as set forth in SEQ ID NO: 22 and sequence a, wherein sequence a comprises, from the N-terminus to the C-terminus, the sequences as set forth in SEQ ID NO: 181, SEQ ID NO: 32, SEQ ID NO: 149, and SEQ ID NO: 157;
(50) a fusion protein comprising the sequences as set forth in SEQ ID NO: 22 and SEQ ID NO: 116;
(51) a fusion protein comprising the sequence as set forth in SEQ ID NO: 22 and sequence a, wherein sequence a comprises, from the N-terminus to the C-terminus, the sequences as set forth in SEQ ID NO: 184, SEQ ID NO: 3, and SEQ ID NO: 23; or
(52) a fusion protein comprising the sequence as set forth in SEQ ID NO: 22 and sequence a, wherein sequence a comprises, from the N-terminus to the C-terminus, the sequences as set forth in SEQ ID NO: 184, SEQ ID NO: 32, SEQ ID NO: 149, and SEQ ID NO: 23.

22. A biological material selected from the group consisting of:
(1) a polynucleotide encoding the fusion protein according to any one of claims 1 to 21;
(2) a vector comprising the fusion protein according to any one of claims 1 to 21; and
(3) a host cell comprising the fusion protein according to any one of claims 1-21.

23. A conjugate comprising the fusion protein according to any one of claims 1 to 21 and a conjugated moiety, wherein the conjugated moiety is selected from a purification tag (such as a His tag, a streptavidin tag), a cytotoxic agent, a detectable label, a radioisotope, a luminescent substance, a colored substance, an enzyme, or polyethylene glycol.

24. A pharmaceutical composition comprising the fusion protein according to any one of claims 1 to 21 and a pharmaceutical carrier, preferably, the pharmaceutical composition is in a dosage form suitable for oral administration to the gastrointestinal (GI) tract; preferably, the dosage form is selected from a tablet, a capsule, a pill, a powder, a granule, an emulsion, a microemulsion, a solution, a suspension, a syrup, and an elixir; or the pharmaceutical composition is in a dosage form suitable for administration by subcutaneous injection, intradermal injection, intravenous injection, intramuscular injection, or intralesional injection.

25. A kit comprising the fusion protein according to any one of claims 1 to 21 and a therapeutic agent for treating a tumor, preferably, the therapeutic agent for treating a tumor is selected from a chemotherapeutic agent, a targeted therapeutic agent, T cells expressing a chimeric antigen receptor, or an angiogenesis inhibitor.

26. Use of the fusion protein according to any one of claims 1 to 21 in the treatment of a tumor, or in the manufacture of a medicament for the treatment of a tumor.

27. Use according to claim 26, wherein the tumor is selected from gastric cancer, pancreatic cancer, lung cancer, colon cancer, melanoma, microglioma.
